# EUROPEAN PATENT APPLICATION

(11) **EP 1 884 568 A1**
(43) Date of publication of application: **06.02.2008**
(21) Application number: 06745969.3
(22) Date of filing: 01.05.2006
(51) Int. Cl.: C12N 15/09, C07K 14/705, C12N 1/15, C12N 1/19, C12N 1/21, C12N 5/10, C12P 21/02, C12Q 1/02, G01N 33/15, G01N 33/50, A61K 45/00, A61P 25/24, C07K 16/28

(54) **METHOD FOR IDENTIFICATION OF COMPOUND HAVING ANTIDEPRESSANT EFFECT**

(30) Priority: 02.05.2005 JP 2005134367
(71) Applicant: Daiichi Sankyo Company, Limited, Chuo-ku Tokyo 103-8426 (JP); Kazusa DNA Research Institute Foundation, Kisarazu-shi, Chiba 292-0818 (JP)
(72) Inventor: OHARA, Osamu KAZUSA DNA RESEARCH INST. FOUNDATION, Kisarazu-shi, Chiba 2920818 (JP); NAGASE, T. KAZUSA DNA RESEARCH INST. FOUNDATION, Kisarazu-shi, Chiba 2920818 (JP); OHISHI, M. KAZUSA DNA RESEARCH INST. FOUNDATION, Kisarazu-shi, Chiba 2920818 (JP); OKAJIMA, Daisuke Daiichi Sankyo Co Ltd, Edogawa-ku, Tokyo 1348630 (JP); YOKOTA, Hiroshi Daiichi Sankyo Co Ltd, Edogawa-ku, Tokyo 1348630 (JP)
(74) Representative: De Clercq, Ann G. Y.
(86) International application number: PCT/JP2006/309118
(87) International publication number: WO 2006/118289

(57) **Abstract**

The present invention intended to find out a gene encoding a protein having functions equivalent to those of G-protein-coupled receptor (GPCR) and the protein, and to provide a method for identifying a compound that regulates functions and/or expression of the protein and a useful means for preventing and/or treating diseases relating to the gene and the protein, and has provided a DNA comprising nucleotide sequence as set forth in SEQ ID NO: 1 or complementary strand thereof, equivalents of the DNA, a protein encoded by the DNA, a vector containing the DNA, a transformant containing the vector, an antibody against the protein, a method for identifying a compound that regulates functions and/or expression of the protein using aforementioned members, an agent and a method for improving depression state, a pharmaceutical composition, and a reagent kit.

## Description

### TECHNICAL FIELD

The present invention relates to a method for identifying a compound having antidepressant action. Specifically, the present invention relates to a method for identifying a compound having antidepressant action, which is an antagonist of any one protein selected from the group consisting of a protein encoded by a DNA represented by a nucleotide sequence as set forth in SEQ ID NO: 1 in the sequence listing and a splicing variant of the protein. Further, the present invention relates to a DNA encoding the splicing variant or a complementary strand thereof, a recombinant vector containing the DNA or the complementary strand thereof, and a transformant in which the recombinant vector is introduced. Furthermore, the present invention relates to a protein translated from a DNA encoding the splicing variant and a method for producing the protein. Besides, the present invention relates to a reagent kit comprising at least one member selected from a DNA represented by the nucleotide sequence described by SEQ ID NO: 1 in the sequence listing and a splicing variant of the DNA, a recombinant vector containing any one DNA selected from the DNA and a splicing variant of the DNA, a transformant in which the recombinant vector is introduced, a protein encoded by the DNA, and an antibody recognizing the protein. Further, the present invention relates to an agent for improving depression state comprising a compound that inhibits the function and/or expression of any one protein selected from the group consisting of a protein encoded by a DNA represented by the nucleotide sequence as set forth in SEQ ID NO: 1 in the sequence listing and a splicing variant of the protein. Besides, the present invention relates to a method for improving depression state comprising inhibiting the function and/or expression of any one protein selected from the group consisting of a protein encoded by a DNA represented by the nucleotide sequence as set forth in SEQ ID NO: 1 in the sequence listing and a splicing variant of the protein. Further, the present invention relates to an agent for preventing and/or treating depression comprising an effective amount of the agent for improving the depression state. Further, the present invention relates to a method for preventing and/or treating depression comprising using the agent for improving the depression state and the method for improving the depression state. Besides, the present invention relates to a method for quantitatively or qualitatively assaying a splicing variant of a DNA represented by the nucleotide sequence as set forth in SEQ ID NO: 1, or the complementary strand thereof, or a protein encoded by the splicing variant. Further, the present invention relates to an assay method for use in diagnosing depression or a method for diagnosing depression, comprising assaying at least one DNA selected from the group consisting of a DNA represented by a nucleotide sequence as set forth in SEQ ID NO: 1 in the sequence listing and a splicing variant of the DNA, and/or, the protein encoded by the DNA.

### BACKGROUND ART

The membrane protein receptor is a protein that has a domain for penetrating the lipid bilayer of a biological membrane to be present in a cell membrane, and specifically recognizes various physiological active substances to transmit and express their actions. The physiological active substance specifically binding to the membrane protein receptor is generally referred to as a ligand. The ligand is exemplified by a peptide hormone, a neurotransmitter, a growth factor and the like. The binding of the ligand to the membrane protein receptor causes a cell response via formation of second messenger, change in intracellular ion concentration, phosphorylation of proteins and the like. A series of reactions involving in changes such as formation of second messenger in cells, change in intracellular ion concentration and phosphorylation of proteins by binding of a ligand to the membrane protein receptor are generally referred to as a signal transduction, and a process for the series of reactions is referred to as a signal transduction pathway.

G-protein coupled receptor (hereunder, may be abbreviated as GPCR) is a glycoprotein present in cell membrane that is one kind of seven-span transmembrane receptor with a structure characterized by seven cell membrane spanning domains, and it constitutes a super family with a many members. One thousand or more GPCR genes have already been identified, and studies are proceeding in relation to the three dimensional structure of GPCR, lignads for GPCR, intracellular signal transduction pathways through GPCR, and the functions thereof and the like.

GPCR is a receptor for light, odor and flavor, while it is also a hormone and neurotransmitter receptor, and works as an important sensor of cells in living organisms ranging from yeasts to humans.

When GPCR receives stimulation from a ligand, it binds to G protein that is present inside the cell. G protein is a protein that couples with GPCR to function as a signal transducer. G protein is broadly classified into a several kinds of families based on functions on various factors (hereunder, referred to as "effector") involved in signal transduction in the intracellular signal transduction pathways and difference in the genes encoding the protein. The G proteins that belong to each family are trimers comprising three subunits called α, β and γ, normally with guanosine 5'-diphosphate (GDP) bound specifically to α-subunit. GDP-bound G protein is an inactive form that does not exhibit an action to an effector. When GPCR is stimulated by a ligand, an exchange reaction occurs between GDP bound to G protein and guanosine 5'-triphosphate (GTP) present in the cell, in which the GDP is released from G protein followed by binding of GTP to G protein to form GTP-bound G protein. GTP-bound G protein, which is referred to as an active form, rapidly dissociates into α-subunit bound with GTP (αGTP) and a dimer (βγ) comprising β- and γ- subunits. αGTP and βγ directly act on an effector (for example, a calcium ion channel or a potassium ion channel) to activate the intracellular signal transduction pathway, and consequently induce various cell responses.

Amongst the GPCR superfamily, human brain angiogenesis inhibitor 2 (hereunder, abbreviated as hBAI2) is classified into class B (secretin like), and its gene is registered in GenBank under the accession number AB 005298.

There have been reported a sequence information and expression distribution of hBAI2 (Non-Patent Literature 1), but neither the function of hBAI2 nor its involvement in disease has been reported. However, based on a structural comparison with BAI1, a homolog of BAI2, it is considered that thrombospondin type I domain (hereunder referred to as "TSP-I domain") is present in the extracellular domain (Non-Patent Literature 2). TSP-I domain is a characteristic domain recognized in thrombospondin in a region comprising the amino acid sequence from position 385 to position 522, and is known to be involved in the extracellular matrix of thrombospondin and to be an important functional domain for angiogenesis inhibiting activity

Regarding hBAI1, there has been some reports indicating its specifically high expression in human brain tissue, the presence of a domain with angiogenesis inhibiting activity in the extracellular region, a possibility to undergo expression control by p53, and the like, which suggests that this gene may make any contribution in a mechanism relating to angiogenesis in the brain (Non-Patent Literature 1-4).

Regarding mouse BAI2, a corresponding gene to hBAI2, it is reported that a negative correlation is observed in the expression amount between BAI2 and vascular endothelial growth factor (VEGF) in brain tissue of cerebral ischemia model rat, which allows a consideration that, similarly to hBAI1, mouse BAI2 may be involved in angiogenesis. Specifically, expression of BAI2 was decreased after suffering the hypoxic state which was followed by increase of expression of VEGF. Further, a splicing variant of mouse BAI2 is reported to exist (non-patent document 3).

Both hBAI1 and hBAI2 are predicted to belong to the GPCR family from the sequence information; however, no report can be found that mentions their functions as GPCR, including information regarding a ligand.

Meanwhile, cholecystokinin (hereunder, abbreviated as "CCK") is known as a gastrointestinal hormone released from endocrine cell in duodenal mucous membrane. CCK is secreted along with fat intake, and promotes gallbladder contraction and pancreatic enzyme secretion. It exhibits actions in the digestive organs including gallbladder contraction, promotion of pancreatic enzyme secretion and stimulation of intestinal movement. CCK is also considered a signaling substance that imparts a sensation of satiety to cerebral neurons.

CCK is known to be sulfated at the seventh tyrosine residue from the C-terminal side. Post-translational processing of CCK generates fragments of several lengths based on the different cleavage sites on the N-terminal side, such as CCK-4, CCK-8, CCK-12, CCK-33 and CCK-58. It has been verified that the physiological activity and amount of each of these fragments are different. Further, cerulein that is extracted from the skin of frog has been reported as a compound that has a similar chemical structure to CCK and exhibits the same biological activity.

CCK is widely distributed in the brain. For example, it has been observed in large amounts in brain cortex, hippocampus, amygdaloid body, and hypothalamus. Further, its action in the central nerves, such as involvement in anxiety, analgesia, sedation, food intake control, memory and learning, is also reported. CCK is partially co-localized with DA (Dopamine) and GABA (γ-aminobutyric acid), and its interaction with 5HT (serotonin; 5-hydroxytryptamine)-activated nerve system and the like has also been reported. It has also been reported that release of CCK is regulated by GABA. CCK-8 and CCK-4 have mainly been reported as CCK that is present in the brain with exhibiting bioactivity. CCK-8 that is present in the brain is a cholecystokinin octapeptide sulfated form (CCK-8S) in which the seventh tyrosine residue from the C-terminal side is sulfated.

Recently, it has been reported that CCK is essential for memory retention. For example, it has been clarified that absence of CCK-8S makes it difficult to recall memory to conscious level and translate it into action, and that CCK-4 (a C-terminal tetrapeptide of CCK-33) obstructs mnemonic retrieval.

CCK-A receptor and CCK-B receptor have been reported as CCK receptors. These are both G-protein coupled receptors. Expression of CCK-A receptor is detected in tissues and cells originating in the alimentary canal, leukocytes in the blood, and the like. CCK-A receptor is involved in alimentary regulation through promotion of effectors such as phospholipase C and adenyl cyclase in the intracellular signal transduction pathway. Meanwhile, expression of CCK-B receptor is detected in tissues and cells originating in the brain and alimentary canal, leukocytes in the blood, and the like. CCK-B receptor is also referred to as "gastrin receptor", and is involved in alimentary regulation and cell proliferation through promotion of effectors such as phospholipase C and intracellular calcium ion influx in the intracellular signal transduction pathway. In recent years, attention is being focused on the relation between CCK-B receptor and anxiety.

Non-Patent Literature 1: Shiratsuchi, T. et al., "Cytogenetics and cell genetics", 1997, Vol. 79, p. 103-108.
Non-Patent Literature 2: Nishimori, H. et al., "Oncogene", 1997, Vol. 15, p. 2145-2150.
Non-Patent Literature 3: Kee, H. J. et al., "Journal of Cerebral Blood Flow and Metabolism", 2002, Vol. 22, p. 1054-1067.
Non-Patent Literature 4: Kaur, B. et al., "American Journal of Pathology", 2003, Vol. 162, p. 19-27.

### DISCLOSURE OF THE INVENTION

GPCR works as an important sensor of cells *in vivo,* and is a leading target molecule in developing remedies for various diseases. Although a large number of GPCRs have already been found, identification of a novel GPCR can bring expectations for a large contribution in the field of pharmaceutical development.

An object of the present invention is to provide a gene encoding a novel protein having an equivalent function to GPCR and the protein. Further, another object of the present invention is to provide a method for producing the protein. Furthermore, the other object of the present invention is to provide a recombinant vector containing the gene and a transformant in which the recombinant vector is introduced. Further, the other object of the present invention is to find out a relation of the gene and protein to a disease, and to provide an effective means for prevention and/or treatment of the disease. Furthermore, the other object of the present invention is to provide a method for identifying a compound for use in prevention and/or treatment of the disease.

The present inventors conducted concentrated studies to solve the above problems and found a protein that works as a functional membrane protein receptor having a seven-span transmembrane domain and can be considered to be a GPCR as well as a gene encoding the protein, and succeeded in acquiring the gene product thereof using the gene. The present inventors demonstrated that the protein was expressed on cell membrane in animal cells that expressed the gene, and that a cell response was produced by ligand stimulation through intracellular signal transduction. It was also clarified that the protein interacted with a protein involved in intracellular signal transduction in the C-terminal region thereof, and also that it had three TSP-I domains that were known to be associated with an angiogenesis inhibiting function in its amino acid sequence. Further, it was demonstrated that CCK-8S acted as a ligand of the functional membrane protein receptor.

In the present invention, a splicing variant of the gene was also found out. Then, the present inventors demonstrated that a protein encoded by the splicing variant was expressed on a cell membrane of an animal cell as a protein encoded by the gene did, and caused a cell response by the ligand stimulation through an intracellular signal transduction.

Further, the present invention demonstrated that an experimental system in which an animal cell expressing the gene was stimulated by the ligand to cause a cell response can be used to identify a compound that inhibits the function of a protein encoded by the gene, i.e., the cell response.

Further, the present invention revealed that the gene is strongly expressed in the brain tissues, particularly in brain cortex, hippocampus, and amygdaloid body. Further, it has found out that the gene and a splicing variant thereof are involved in depression.

The present invention was achieved based on these findings.

Thus, the present invention relates to a method for identifying a compound having an anti-depressant action that is an antagonist of a protein encoded by a DNA represented by a nucleotide sequence as set forth in SEQ ID NO: 1 in the sequence listing or of a protein homolog thereof, comprising using at least one member selected from the following: a DNA represented by a nucleotide sequence as set forth in SEQ ID NO: 1 in the sequence listing or a DNA homolog thereof, a protein encoded by the DNA and a cell containing the DNA.

The present invention also relates to a method for identifying a compound having an anti-depressant action that is an antagonist of any one protein selected from the group consisting of a protein encoded by a DNA represented by a nucleotide sequence as set forth in SEQ ID NO: 1 in the sequence listing and splicing variants of the protein, comprising using at least one member selected from the following: a DNA represented by a nucleotide sequence as set forth in SEQ ID NO: 1 in the sequence listing, a DNA represented by any one of nucleotide sequences of splicing variants of the DNA, a protein encoded by the DNA, and a cell containing the DNA.

The present invention further relates to a method for identifying a compound having an anti-depressant action that is an antagonist of any one protein selected from the group consisting of a protein encoded by a DNA represented by a nucleotide sequence as set forth in SEQ ID NO: 1 in the sequence listing and splicing variants of the protein, comprising contacting a cell containing a DNA represented by any one of the nucleotide sequences as set forth in SEQ ID NO: 1, 15, 17, 19 and 21 in the sequence listing with a test compound, measuring a function of a protein encoded by the DNA that is expressed on a cell membrane of the cell, comparing with a case where the cell is not made to contact with a test compound, and determining that a test compound which reduces or eliminates the function of the protein is to be a compound having anti-depressant action.

The present invention still further relates to the aforementioned identifying method, wherein the function of a protein encoded by the DNA that is expressed on a cell membrane of a cell is a function causing an increase in intracellular calcium concentration in response to addition of a ligand of the protein.

The present invention also relates to the aforementioned identifying method, wherein the function of a protein encoded by the DNA that is expressed on a cell membrane of a cell is a function causing a change in membrane potential in response to addition of a ligand of the protein.

The present invention further relates to the aforementioned identifying method, wherein the ligand used therein is a peptide selected from the group consisting of:
(i) cholecystokinin octapeptide sulfated form (SEQ ID NO: 14, hereunder referred to as CCK-8S),
(ii) a peptide having mutations such as deletion, substitution, or addition of one or a few amino acids in amino acid sequence of CCK-8S and having an equivalent function to CCK-8S; and
(iii) a peptide containing the amino acid sequence of the peptide described in (i) or (ii) and having an equivalent function to CCK-8S.

The present invention still further relates to a DNA represented by the nucleotide sequence as set forth in SEQ ID NO: 15 in the sequence listing or a complementary strand thereof.

The present invention also relates to a DNA represented by the nucleotide sequence as set forth in SEQ ID NO: 17 in the sequence listing or a complementary strand thereof.

The present invention further relates to a recombinant vector comprising the DNA represented by the nucleotide sequence as set forth in SEQ ID NO: 15 in the sequence listing, or the DNA represented by the nucleotide sequence as set forth in SEQ ID NO: 17 in the sequence listing, or a complementary strand of the DNA.

The present invention still further relates to a transformant into which the aforementioned recombinant vector is introduced.

The present invention also relates to a protein encoded by the DNA represented by the nucleotide sequence as set forth in SEQ ID NO: 15 in the sequence listing, or the DNA represented by the nucleotide sequence as set forth in SEQ ID NO: 17 in the sequence listing, or a complementary strand of the DNA.

The present invention further relates to protein represented by the amino acid sequence as set forth in SEQ ID NO: 16 in the sequence listing.

The present invention still relates to a protein represented by the amino acid sequence as set forth in SEQ ID NO: 18 in the sequence listing.

The present invention also relates to a method for producing the aforementioned protein, comprising culturing a transformant into which an expression recombinant vector comprising the DNA represented by the nucleotide sequence as set forth in SEQ ID NO: 15 in the sequence listing, or the DNA represented by the nucleotide sequence as set forth in SEQ ID NO: 17 in the sequence listing, or a complementary strand ofthe DNA.

The present invention further relates to a reagent kit comprising at least any one of the following: a DNA represented by the nucleotide sequence as set forth in SEQ ID NO: 1 in the sequence listing and splicing variants of the DNA, a recombinant vector comprising any one DNA selected from the DNA and the splicing variants of the DNA, a transformant into which the recombinant vector is introduced, a protein encoded by the DNA, and an antibody that recognizes the protein.

The present invention still further relates to the aforementioned reagent kit, wherein the DNA represented by the nucleotide sequence as set forth in SEQ ID NO: 1 in the sequence listing and splicing variants of the DNA is a DNA represented by any one of the nucleotide sequences as set forth in SEQ ID NO: 1, 15 and 17 in the sequence listing, and the protein encoded by the DNA is a protein represented by any one of the amino acid sequences as set forth in SEQ ID NO: 2, 16 and 18 in the sequence listing.

The present invention also relates to a method for improving depression state, comprising inhibiting the function and/or expression of any one protein selected from the group consisting of a protein encoded by a DNA represented by the nucleotide sequence as set forth in SEQ ID NO: 1 in the sequence listing and splicing variants of the protein.

The present invention further relates to a method for preventing and/or treating depression, comprising using the aforementioned method for improving depression state.

The present invention still further relates to an assay method for use in diagnosing depression, comprising employing a DNA selected from the group consisting of a DNA represented by a nucleotide sequence as set forth in SEQ ID NO: 1 in the sequence listing and splicing variants of the DNA, and/or, the protein encoded by the DNA, as a marker, and performing quantitative or qualitative analysis thereof.

The present invention also relates to a method for diagnosing depression, comprising employing a DNA selected from the group consisting of a DNA represented by a nucleotide sequence as set forth in SEQ ID NO: 1 in the sequence listing and a splicing variant of the DNA, and/or, the protein encoded by the DNA, as a marker, and performing quantitative or qualitative analysis thereof.

According to the present invention, it is possible to provide a protein that works as a functional membrane protein receptor having a seven-span transmembrane domain which is considered to be a GPCR and a DNA encoding the protein. The protein is expressed on a cell membrane when expressed in a cell, and activates an intracellular signal transduction in response to ligand stimulation to cause a cell response.

According to the present invention, it is possible to carry out the elucidation of the signal transduction pathway and the cellular function both of which the present protein participates in as well as the regulation thereof Moreover, the present invention allows for the prevention and/or treatment of a disease attributable to an abnormality in the present protein and/or DNA, for example, depression.

### BRIEF DESCRIPTION OF THE DRAWINGS

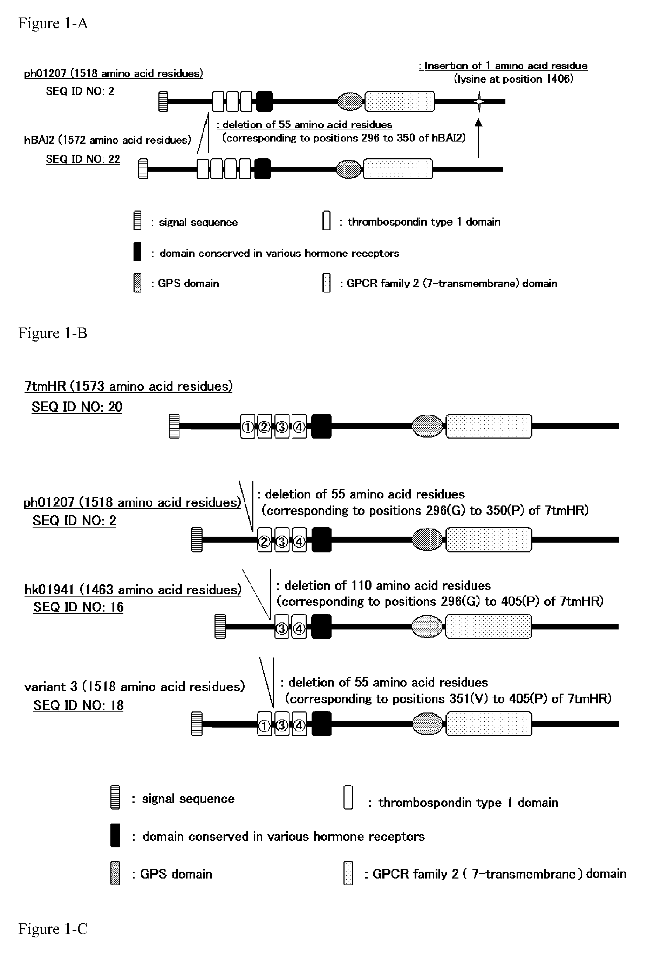

Figure 1-A is a schematic diagram illustrating a comparison between a functional domain of a protein represented by the amino acid sequence as set forth in SEQ ID NO: 2 and that of a protein encoded by hBAI2. (Example 1)

Figure 1-B is a schematic diagram illustrating a comparison between a structural characteristic of a protein represented by the amino acid sequence as set forth in SEQ ID NO: 2 and that of a splicing variant thereof. The term "ph01207" means a protein represented by the amino acid sequence as set forth in SEQ ID NO: 2. The terms "7tmHR", "hk01941" and "variant 3" each indicate a splicing variant of a protein represented by the amino acid sequence as set forth in SEQ ID NO: 2. (Example 6)

Figure 1-C shows a comparison between an amino acid sequence of a protein represented by the amino acid sequence as set forth in SEQ ID NO: 2 and that of a splicing variant thereof. In the figure, the thrombospondin type I domain (TSP-I domain) was double underlined, and the transmembrane domain was underlined. The term "ph01207" means a protein represented by the amino acid sequence as set forth in SEQ ID NO: 2. The terms "7tmHR", "hk01941" and "variant 3" each indicate a splicing variant of a protein represented by the amino acid sequence as set forth in SEQ ID NO: 2. (Example 6)

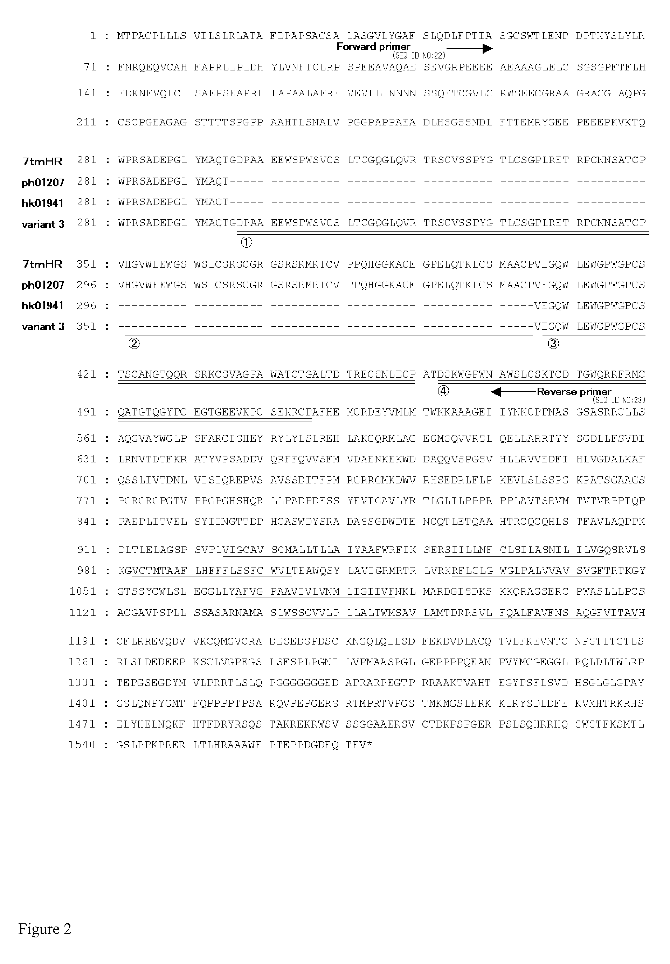

Figure 2 shows that, when cDNA clone ph01207 was expressed in CHO-K1 cells, an animal cell line, as a FLAG-tag fusion protein and a HA-tag fusion protein, respectively, these proteins expressed on the cell membrane (the left panel and right panel, respectively). Detection of the protein on the cell membrane was analyzed by fluorocytometry using anti-FLAG-tag antibody, anti-HA-tag antibody and an FITC-labeled second antibody (FITC-anti-mouse IgG antibody). In the figure, the region shown in black indicates cells that expressed each tag fusion protein, and the region shown in white indicates control cells that did not express the proteins. (Example 2)

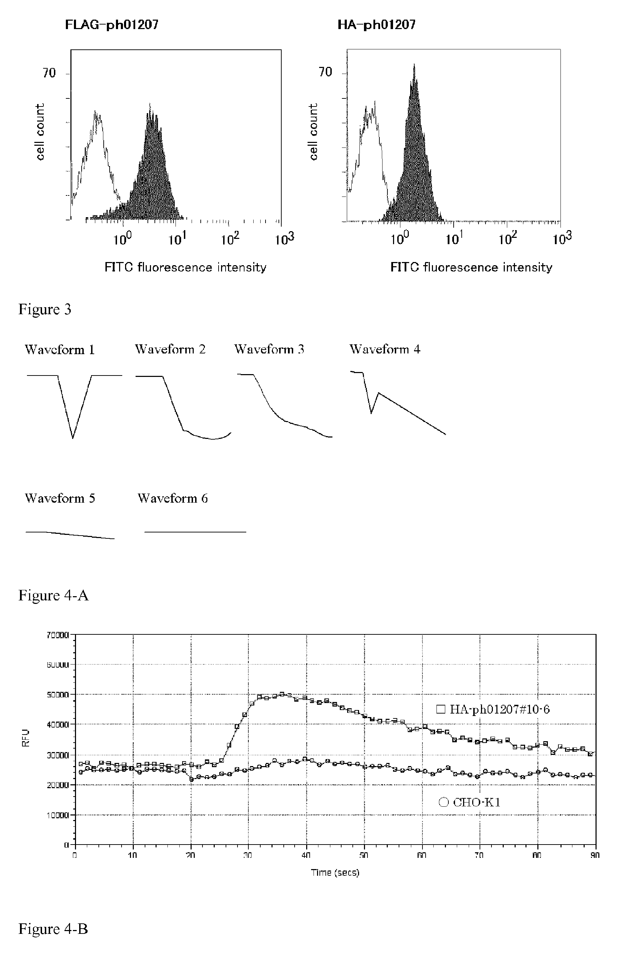

Figure 3 shows waveforms that can be observed when the ligand response of GPCR is measured by changes in membrane potential of cells (waveform produced by GPCR-specific response (waveform 1), waveforms produced by artificial elements and the like (waveform 2-4), and waveforms recognized at the time of no response (waveforms 5 and 6)). (Example 3)

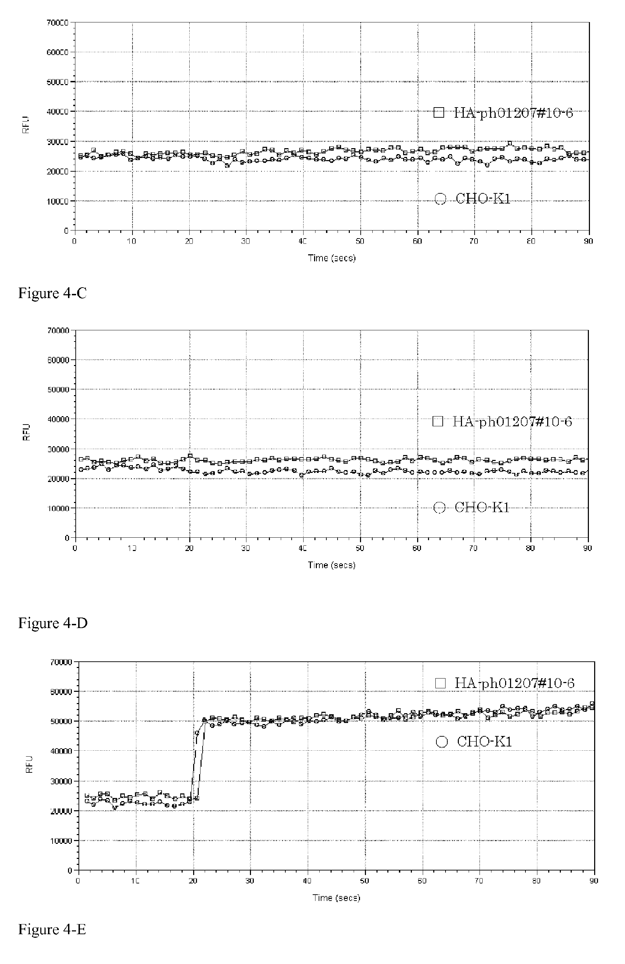

Figure 4-A shows that intracellular Ca²⁺ concentration increased with addition of 1 nM CCK-8S (SEQ ID NO: 14) in CHO-K1 cell line (HA-ph01207#10-6) stably expressing cDNA clone ph01207 as a HA-tag fusion protein. In contrast, in CHO-K1 cell line that was not transfected with this cDNA, an increase in intracellular Ca²⁺ concentration with addition of CCK-8S (SEQ ID NO: 14) was not observed. The horizontal axis shows the time (Time (sec)) from the start of measurement of intracellular Ca²⁺ concentration. The longitudinal axis shows fluorescence intensity (RFU) of fluorescent dye that reflects Ca²⁺ concentration. (Example 5)

Figure 4-B shows that an increase in intracellular Ca²⁺ concentration with addition of 1 nM CCK-8NS (CCK-8 Nonsulfated form) was not observed in CHO-K1 cell line (HA-ph01207#10-6) stably expressing cDNA clone ph01207 as a HA-tag fusion protein. CCK-8NS is a CCK octapeptide consisting of the same amino acid sequence as CCK-8S, but its seventh tyrosine residue from the C-terminus is not sulfated. An increase in intracellular Ca²⁺ concentration with addition of CCK-8NS was also not observed in a CHO-K1 cell line that was not transfected with cDNA clone ph01207. The horizontal axis shows the time (Time (sec)) from the start of measurement of intracellular Ca²⁺ concentration. The longitudinal axis shows fluorescence intensity (RFU) of fluorescent dye that reflects Ca²⁺ concentration. (Example 5)

Figure 4-C shows that an increase in intracellular Ca²⁺ concentration with addition of 1 nM CCK-4 was not observed in CHO-K1 cell line (HA-ph01207#10-6) stably expressing cDNA clone ph01207 as a HA-tag fusion protein. An increase in intracellular Ca²⁺ concentration with addition of CCK-4 was also not observed in CHO-K1 cell line that was not transfected with the cDNA. The horizontal axis shows the time (Time (sec)) from the start of measurement of intracellular Ca²⁺ concentration. The longitudinal axis shows fluorescence intensity (RFU) of fluorescent dye that reflects Ca²⁺ concentration. (Example 5)

Figure 4-D shows that intracellular Ca²⁺ concentration increased with addition of 10 µM calcium ionophore A23187 in CHO-K1 cell line (HA-ph01207#10-6) stably expressing cDNA clone ph01207 as a HA-tag fusion protein. An increase in intracellular Ca²⁺ concentration with addition of A23187 was also observed in CHO-K1 cell line that was not transfected with the cDNA. The horizontal axis shows the time (Time (sec)) from the start of measurement of intracellular Ca²⁺ concentration. The longitudinal axis shows fluorescence intensity (RFU) of fluorescent dye that reflects Ca²⁺ concentration. (Example 5)

Figure 4-E shows intracellular Ca²⁺ concentrations after addition of a buffer in CHO-K1 cell line (HA-ph01207#10-6) stably expressing cDNA clone ph01207 as a HA-tag fusion protein and CHO-K1 cell line that was not transfected with the cDNA. The horizontal axis shows the time (Time (sec)) from the start of measurement of intracellular Ca²⁺ concentration. The longitudinal axis shows fluorescence intensity (RFU) of fluorescent dye that reflects Ca²⁺ concentration. (Example 5)

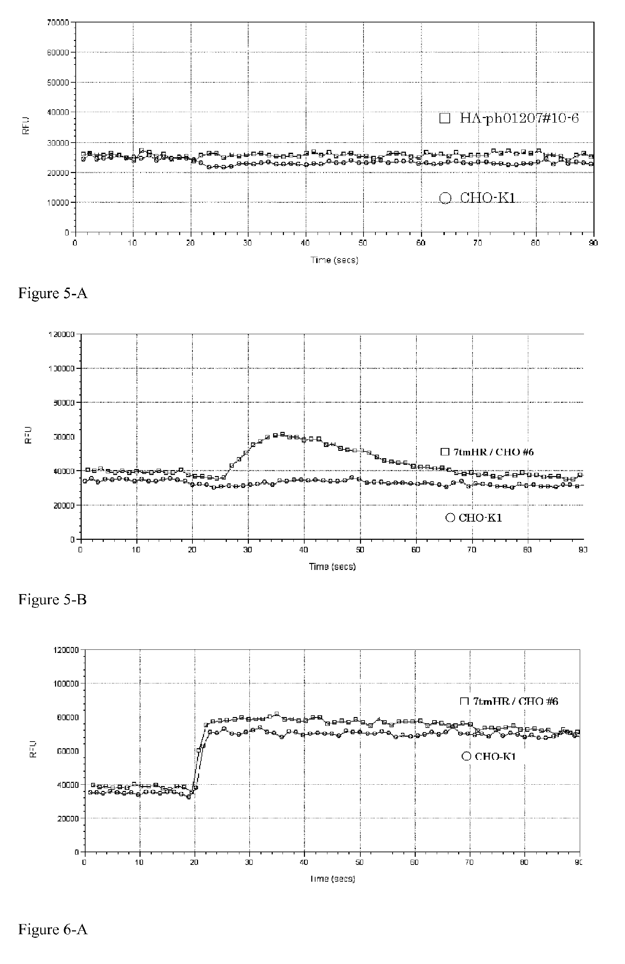

Figure 5-A shows typical results of increase in intracellular Ca²⁺ concentration by 1nM CCK-8S (SEQ ID NO: 14) in 7tmHR stably expressing cell line. Increase in intracellular Ca²⁺ concentration by CCK-8S (SEQ ID NO: 14) was not observed in a host cell to which 7tmHR expression vector was not transfected. In the figure, the term "7tmHR/CHO #6" refers to 1 clone of 7tmHR stably expressing cell line, and the term "CHO-K1" refers to the host cell. The horizontal axis shows the time (Time (sec)) from the start of measurement of intracellular Ca²⁺ concentration. The longitudinal axis shows fluorescence intensity (RFU) of fluorescent dye that reflects Ca²⁺ concentration. (Example 8)

Figure 5-B shows an increase in intracellular Ca²⁺ concentration by 20 µM calcium ionophore A23187 in 7tmHR stably expressing cell line. An increase in intracellular Ca²⁺ concentration by A23187 was observed even in the host cell to which 7tmHR expression vector was not transfected. In the figure, the term "7tmHR/CHO #6" refers to 1 clone of 7tmHR stably expressing cell line, and the term "CHO-K1" refers to a host cell. The horizontal axis shows the time (Time (sec)) from the start of measurement of intracellular Ca²⁺ concentration. The longitudinal axis shows fluorescence intensity (RFU) of fluorescent dye that reflects Ca²⁺ concentration. (Example 8)

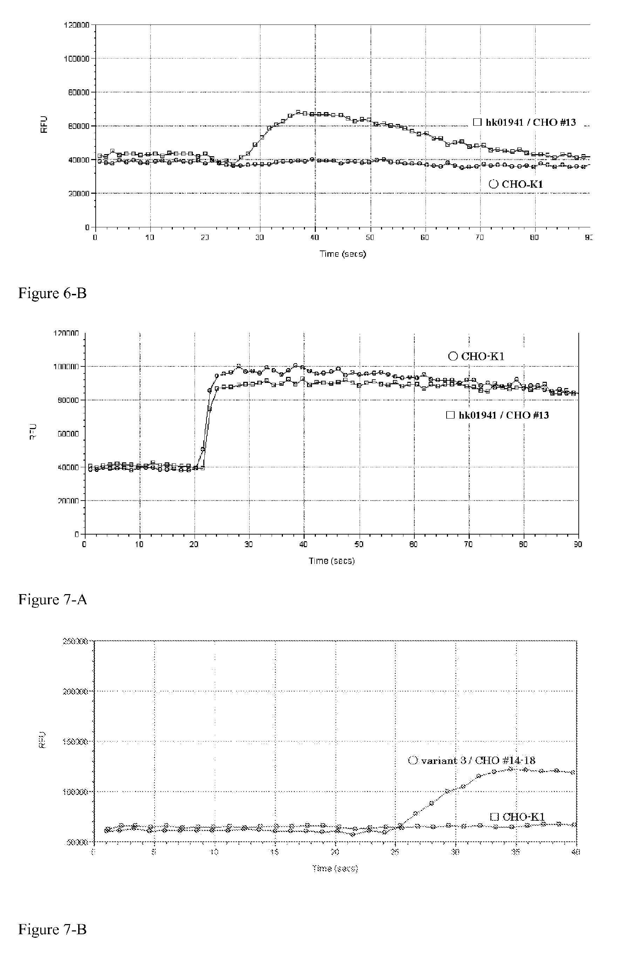

Figure 6-A shows typical results of increase in intracellular Ca²⁺ concentration by 1nM CCK-8S (SEQ ID NO: 14) in hk01941 stably expressing cell line. Increase in intracellular Ca²⁺ concentration by CCK-8S (SEQ ID NO: 14) was not observed in a host cell to which hk01941 expression vector was not transfected. In the figure, the term "hk01941/CHO #13" refers to 1 clone of hk01941 stably expressing cell line, and the term "CHO-K1" refers to the host cell. The horizontal axis shows the time (Time (sec)) from the start of measurement of intracellular Ca²⁺ concentration. The longitudinal axis shows fluorescence intensity (RFU) of fluorescent dye that reflects Ca²⁺ concentration. (Example 8)

Figure 6-B shows an increase in intracellular Ca²⁺ concentration by 20 µM calcium ionophore A23187 in hk01941 stably expressing cell line. An increase in intracellular Ca²⁺ concentration by A23187 was observed even in a host cell to which hk01941 expression vector was not transfected. In the figure, the term "hk01941/CHO #13" refers to 1 clone of hk01941 stably expressing cell line, and the term "CHO-K1" refers to the host cell. The horizontal axis shows the time (Time (sec)) from the start of measurement of intracellular Ca²⁺ concentration. The longitudinal axis shows fluorescence intensity (RFU) of fluorescent dye that reflects Ca²⁺ concentration. (Example 8)

Figure 7-A shows typical results of increase in intracellular Ca²⁺ concentration by 1nM CCK-8S (SEQ ID NO: 14) in variant 3 stably expressing cell line. Increase in intracellular Ca²⁺ concentration by CCK-8S (SEQ ID NO: 14) was not observed in a host cell to which variant 3 expression vector was not transfected. In the figure, the term "variant 3/CHO#14-18" refers to 1 clone of variant 3 stably expressing cell line, and the term "CHO-K1" refers to the host cell. The horizontal axis shows the time (Time (sec)) from the start of measurement of intracellular Ca²⁺ concentration. The longitudinal axis shows fluorescence intensity (RFU) of fluorescent dye that reflects Ca²⁺ concentration. (Example 9)

Figure 7-B shows an increase in intracellular Ca²⁺ concentration by 20 µM calcium ionophore A23187 in variant 3 stably expressing cell line. An increase in intracellular Ca²⁺ concentration by A23187 was observed even in a host cell to which variant 3 expression vector was not transfected. In the figure, the term "variant 3/CHO#14-18" refers to 1 clone of variant 3 stably expressing cell line, and the term "CHO-K1" refers to the host cell. The horizontal axis shows the time (Time (sec)) from the start of measurement of intracellular Ca²⁺ concentration. The longitudinal axis shows fluorescence intensity (RFU) of fluorescent dye that reflects Ca²⁺ concentration. (Example 9)

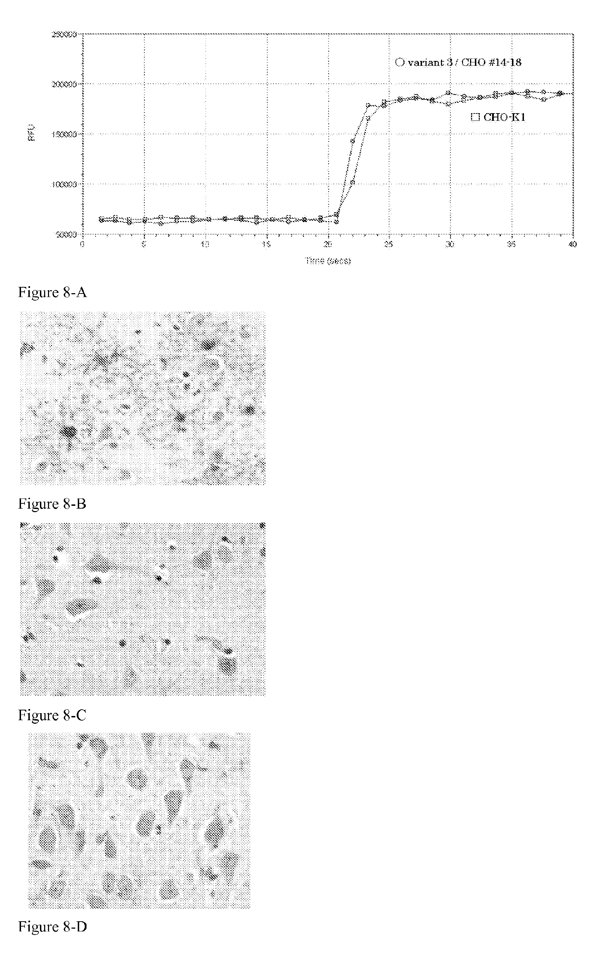

Figure 8-A shows that ph01207 gene is strongly expressed in protoplasmic astrocyte in human amygdaloid body with data analyzed by tissue immunostaining using an anti-human BAI2 polyclonal antibody. (Example 10)

Figure 8-B shows that ph01207 gene is strongly expressed in neuron and glia in amygdaloid body with data analyzed by tissue immunostaining using an anti-human BAI2 polyclonal antibody. (Example 10)

Figure 8-C shows that ph01207 gene is strongly expressed in neuron in CA2 region in hippocampus with data analyzed by tissue immunostaining using an anti-human BAI2 polyclonal antibody. (Example 10)

Figure 8-D is a view showing that ph01207 gene is strongly expressed in neuron in CA1 region in hippocampus with data analyzed by tissue immunostaining using an anti-human BAI2 polyclonal antibody. (Example 10)

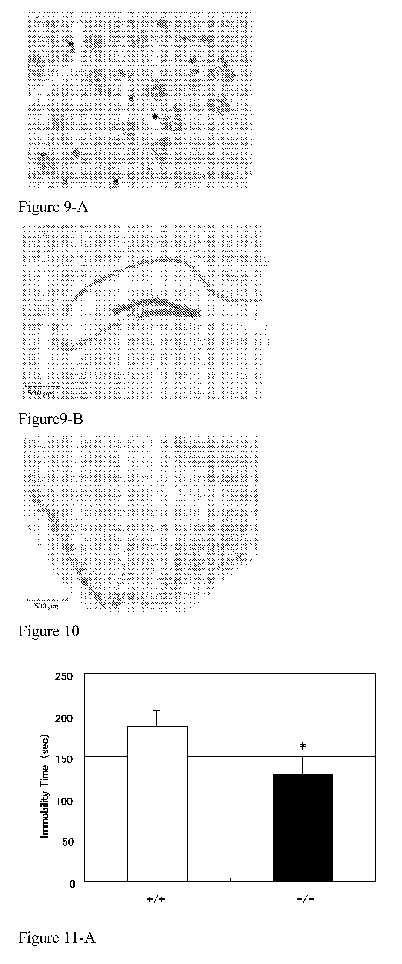

Figure 9-A shows that, in hippocampus of an F1 heterozygous mutant mouse prepared using ES cell which was introduced with a targeting vector for targeting mouse BAI2 gene, LacZ-Neo gene contained in the targeting vector was strongly expressed, which was detected by LacZ expression analysis. This result indicates that LacZ-Neo fragment was inserted into target site of mouse BAI2 gene, namely the gene was destroyed, and that mouse BAI2 gene is expressed in hippocampus. (Example 11)

Figure 9-B shows that, in amygdaloid body of an F1 heterozygous mutant mouse prepared using ES cell which was introduced with a targeting vector for targeting mouse BAI2 gene, LacZ-Neo gene contained in the targeting vector was strongly expressed, which was detected by LacZ expression analysis. This result indicates that LacZ-Neo fragment was inserted into target site of mouse BAI2 gene, namely the gene was destroyed, and that mouse BAI2 gene is expressed in amygdaloid body. (Example 11)

Figure 10 shows immobility time of a BAI2 knockout mouse and a wild type mouse in the tail suspension test. In the figure, "+/+" refers to a wild type mouse and "-/-" refers to a BAI2 knockout mouse. The tail suspension test was carried out using 16 wild type mice and 10 BAI2 knockout mice. Results are expressed by average (sec) ± standard deviation of immobility time of each mouse. In the figure, asterisk shows that a significant difference (P < 0.05) was obtained in statistical processing using t-test. (Example 11)

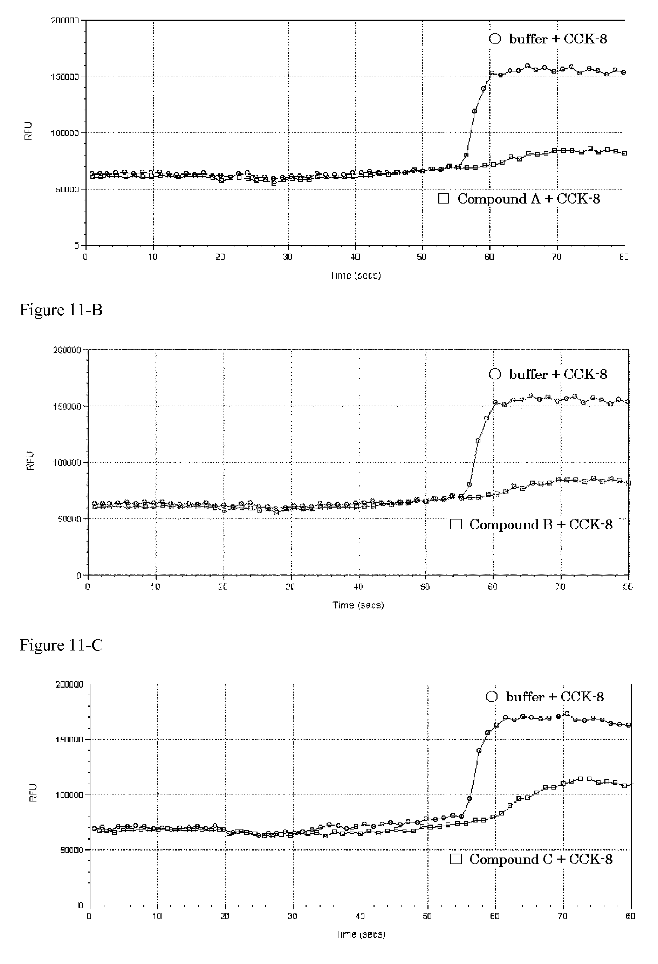

Figure 11-A shows that, in CHO-K1 cell line in which cDNA clone ph01207 is stably expressed, increase in intracellular Ca²⁺ concentration by 10 nM CCK-8S was inhibited by addition of 10 µg/mL compound A. As a control, the buffer was added instead of the compound A. The compound A or the buffer was added 15 sec after the start of measurement, and CCK-8S was added 35 sec after addition of the compound A or the buffer. The horizontal axis shows time (Time (sec)) from the start of measurement of intracellular Ca²⁺ concentration. The longitudinal axis shows fluorescence intensity (RFU) of fluorescent dye that reflects Ca²⁺ concentration. (Example 12)

Figure 11-B shows that, in CHO-K1 cell line in which cDNA clone ph01207 is stably expressed, increase in intracellular Ca²⁺ concentration by 10 nM CCK-8S was inhibited by addition of 10 µg/mL compound B. As a control, the buffer was added instead of the compound B. The compound B or the buffer was added 15 sec after the start of measurement, and CCK-8S was added 35 sec after addition of the compound B or the buffer. The horizontal axis shows time (Time (sec)) from the start of measurement of intracellular Ca²⁺ concentration. The longitudinal axis shows fluorescence intensity (RFU) of fluorescent dye that reflects Ca²⁺ concentration. (Example 12)

Figure 11-C shows that in CHO-K1 cell line in which cDNA clone ph01207 is stably expressed, increase in intracellular Ca²⁺ concentration by 10 nM CCK-8S was inhibited by addition of 10 µg/mL compound C. As a control, the buffer was added instead of the compound C. The compound C or the buffer was added 15 sec after the start of measurement, and CCK-8S was added 35 sec after addition of the compound C or the buffer. The horizontal axis shows time (Time (sec)) from the start of measurement of intracellular Ca²⁺ concentration. The longitudinal axis shows fluorescence intensity (RFU) of fluorescent dye that reflects Ca²⁺ concentration. (Example 12)

### DETAILED DESCRIPTION

In the present specification, the term "protein" is sometimes used as a generic term that refers to an isolated or synthetic full length protein, an isolated or synthetic full length polypeptide, or an isolated or synthetic full length oligopeptide. In this case, a protein, polypeptide or oligopeptide has a minimum size of two amino acids. Hereunder, amino acids may be designated by one-letter codes or three-letter abbreviations.

The present invention relates to a protein that works as a functional membrane protein receptor having a seven-span transmembrane domain, and to a DNA encoding the protein. Specifically, the present invention relates to a protein that works as a G protein coupled receptor, and to a DNA encoding the protein.

As used herein, the phrase "membrane protein receptor" means a protein that is made of a protein having a domain for penetrating the lipid double layer of a biological membrane to be present in a cell membrane, and specifically recognizes various physiological active substances to transmit and express their actions. Here, the protein includes a glycoprotein. As used herein, the phrase "functional membrane protein receptor" means a membrane protein receptor having a function that receives the action of a ligand to cause a cell response via intracellular signal transduction. The membrane protein receptor has an extracellular domain interacting with the ligand, a domain for penetrating the lipid double layer of biological membrane, and an intracellular domain for mediating intracellular signal transduction.

As used herein, the phrase "G-protein coupled receptor (GPCR)" means a membrane protein receptor that activates G-protein by binding to the G-protein present in cells when stimulated by a ligand. The term "G-protein" refers to a protein that associates with GPCR, converts from GDP-binding type G-protein to GTP-binding type G-protein due to GDP/GTP exchange reaction and causes various cell responses as an intracellular signal transducer. The phrase "activates G-protein" means that by inducing and/or promoting the GDP/GTP exchange reaction, the conversion from GDP-binding type G-protein to GTP-binding type G-protein is induced and/or promoted, thereby various cell responses in which the G-protein associated GPCR is involved is induced and/or promoted.

As used herein, the term "ligand" means a physiological active substance specifically interacting with a membrane protein receptor.

As used herein, the term "interaction" means, for example, that two identical or distinct proteins specifically act with each other resulting in change, for example, an increase or a decrease in the function of one or both of the proteins. The phrase "specifically act" means that the proteins participating in the action acts more selectively with each other than with the other proteins. The interaction, for example, includes binding of two distinct proteins, or activation of one protein by another protein.

As used herein, the phrase "intracellular signal transduction" means a series of reactions generating changes, such as formation of second messenger in cells, change in intracellular ion concentration and phosphorylation of proteins, by action of a ligand to the membrane protein receptor. The phrase "intracellular signal transduction pathway" means a process of the series of reactions.

A DNA used in the present invention is, specifically, a DNA represented by the nucleotide sequence as set forth in SEQ ID NO: 1 or a DNA homolog of the DNA. In the present specification, the phrase "DNA homolog" means a DNA having sequence homology with the DNA of interest and encoding a protein which has a similarity with a protein encoded by the DNA in structural characteristic or biological function.

A protein used in the present invention is, preferably, a protein encoded by a DNA represented by the nucleotide sequence as set forth in SEQ ID NO: 1 or a protein homolog of the protein. In the present specification, the phrase "protein homolog" means a protein having sequence homology with the protein of interest and a similarity with the protein in structural characteristic or biological function.

In the present invention, a DNA and protein are preferably a DNA and protein that originate in human, but can be a DNA and protein originating in mammals which have an equivalent function and a structural homology to a human-derived DNA and protein, for example, a DNA and protein that originate in mouse, horse, sheep, cow, dog, monkey, cat, bear, rat or rabbit.

The DNA represented by the nucleotide sequence as set forth in SEQ ID NO: 1 is a DNA encoding a protein that works as a functional membrane protein receptor having a seven-span transmembrane domain. A specific example of a protein encoded by the DNA represented by the nucleotide sequence as set forth in SEQ ID NO: 1 is preferably a protein represented by the amino acid sequence as set forth in SEQ ID NO: 2.

The protein encoded by the DNA represented by the nucleotide sequence as set forth in SEQ ID NO: 1 has three TSP-I domains, one GPS (GPCR proteolytic site) domain and one seven-span transmembrane domain as the structural characteristics (see Fig. 1-A and Fig. 1-B). The seven-span transmembrane domain is also referred to as the GPCR family-2 domain and is a structural characteristic of a G-protein coupled receptor as GPS domain is. The TSP-I domain is a characteristic domain found in thrombospondin and is known to be an important functional domain for involvement of thrombospondin with extracellular matrix and antiangiogenic function thereof.

The gene product of the DNA represented by the nucleotide sequence as set forth in SEQ ID NO: 1 exhibited actually a function as the membrane protein receptor. Specifically, it was observed in the animal cells in which the DNA represented by the nucleotide sequence as set forth in SEQ ID NO: 1 was expressed, that the protein encoded by the DNA was expressed on the cell membrane, and that a cell response via intracellular signal transduction was caused by ligand stimulation such as CCK-8S (SEQ ID NO: 14) stimulation.

In addition, it was observed that the gene product of the DNA represented by the nucleotide sequence as set forth in SEQ ID NO: 1 interacted with MAGUK family proteins DLG2, DLG3 and DLG4, or AIP1, MAGI3 and the like at C-terminal region thereof. MAGUK family proteins have a PDZ domain that recognizes the last C-terminal amino acid sequence of a target protein in protein-protein interaction, and are considered to localize on the cell membrane to interact with a membrane protein such as a receptor or an ion channel and participate in signal transduction from these membrane proteins to contribute to intercellular adhesion and the like. Interaction has similarly been observed between the gene product of hBAI2 gene that is homologous to the DNA represented by the nucleotide sequence as set forth in SEQ ID NO: 1 and MAGUK family proteins. Meanwhile, it has been reported that hBAI1, an hBAI2 homologue, binds to BAP1 (BAI1-associated protein 1), one of the MAGUK family proteins, through a partial sequence (QTEV: SEQ ID NO: 3) of the distal region of hBAI1 (Shiratsuchi, T. et al., "Biochemical and Biophysical Research Communications", 1998, Vol. 247, p.597-604).

The amino acid sequence (QTEV) as set forth in SEQ ID NO: 3 is conserved in the C-terminal region of both the protein encoded by the DNA represented by the nucleotide sequence as set forth in SEQ ID NO: 1 and the protein encoded by hBAI2 (SEQ ID NO: 21) having a sequence homology with the gene. Therefore, the inventors consider that the both proteins interact with a protein having a PDZ domain at this sequence segment.

A DNA homolog of the DNA represented by the nucleotide sequence as set forth in SEQ ID NO: 1 is preferably a DNA that has sequence homology to the DNA represented by the nucleotide sequence as set forth in SEQ ID NO: 1 and encodes a protein that exhibits an equivalent function to GPCR by the action of CCK-8S (SEQ ID NO: 14).

The DNA homolog of the DNA represented by the nucleotide sequence as set forth in SEQ ID NO: 1 is preferably exemplified by a splicing variant of the DNA represented by the nucleotide sequence as set forth in SEQ ID NO: 1.

The protein having similarity with a protein encoded by the DNA represented by the nucleotide sequence as set forth in SEQ ID NO: 1 in structural characteristic or biological function is preferably exemplified by a splicing variant of the protein encoded by the DNA represented by the nucleotide sequence as set forth in SEQ ID NO: 1.

As used herein, the phrase "splicing variant" means two or more kinds of mature mRNAs generated by selective splicing of the mRNA precursor of a certain gene transcribed from a genome in the expression of the gene in a eukaryote, the complementary DNAs of the mature mRNAs, or the proteins translated from the mature mRNAs. Expression of a gene in a eukaryote is carried out by forming a mature mRNA through splicing of the mRNA precursor transcribed from a region composed of exons present on the genome in scattered fashion and introns present between the exons. Further, a protein is produced by translation of the mature mRNA. The term "splicing" means a process where an intron is cut out from an mRNA precursor at a splice site (a boundary point between the intron and the exon) to form a mature mRNA. At the time of splicing, the splice sites some times happen to change in position and combination to generate two or more kinds of mature mRNAs, that is, a so-called selective splicing. As a result of the selective splicing, in many cases, two or more kinds of proteins are produced from the one gene.

The splicing variant of the DNA represented by the nucleotide sequence as set forth in SEQ ID NO: 1 can be two or more kinds of mature mRNAs generated by selective splicing of a mRNA precursor transcribed from a genome of the gene consisting of the DNA, or the complementary DNAs of the mature mRNAs.

The splicing variant of the DNA represented by the nucleotide sequence as set forth in SEQ ID NO: 1 can be preferably exemplified by a DNA represented by any one of the nucleotide sequences as set forth in SEQ ID NOs: 15, 17, 19 and 21.

The DNA represented by the nucleotide sequence as set forth in SEQ ID NO: 19 encodes a protein having the longest amino acid sequence among proteins each encoded by the DNA represented by any one of the nucleotide sequences as set forth in SEQ ID NO: 1, 15, 17, 19 and 21.

The splicing variant of the protein encoded by the DNA represented by the nucleotide sequence as set forth in SEQ ID NO: 1 is not limited to the splicing variant exemplified above and includes any of DNAs as long as it is a DNA that has homology in sequence and similarity in structural characteristics to the DNA, and encodes a protein having an equivalent biological function to the protein encoded by the DNA.

The splicing variant of the protein encoded by the DNA represented by the nucleotide sequence as set forth in SEQ ID NO: 1 is proteins translated from two or more kinds of mature mRNAs generated by selective splicing of a mRNA precursor of a gene encoding the protein that transcribed from a genome.

The splicing variant of the protein encoded by the DNA represented by the nucleotide sequence as set forth in SEQ ID NO: 1 can be preferably exemplified by a protein encoded by the DNA represented by any one of the nucleotide sequences as set forth in SEQ ID NOs: 15, 17, 19 and 21.

The protein encoded by the DNA represented by any one of the nucleotide sequences as set forth in SEQ ID NOs: 15, 17, 19 and 21 can be preferably exemplified by a protein represented by any one of the amino acid sequences as set forth in SEQ ID NOs: 16, 18, 20 and 22.

The protein represented by the amino acid sequence as set forth in SEQ ID NO: 20 is a protein having the longest amino acid sequence among proteins represented by the amino acid sequences as set forth in SEQ ID NO: 2, 16, 18, 20 and 22.

The splicing variant of the protein encoded by the DNA represented by the nucleotide sequence as set forth in SEQ ID NO: 1 is not limited to the splicing variant exemplified above, and includes any of proteins as long as it is a protein that has homology in sequence and similarity in structural characteristics to the protein encoded by the DNA, and further has an equivalent biological function to that of the protein.

In other words, the splicing variant of the protein encoded by the DNA represented by the nucleotide sequence as set forth in SEQ ID NO: 1 includes any of proteins, as long as it is a protein that has homology in sequence and similarity in the structural characteristics to the protein represented by the amino acid sequence as set forth in SEQ ID NO: 2, and has an equivalent biological function to that of the protein.

In the present specification, "homology in sequence" is suitably presented by normally 50% or more homology with the entire sequence of a nucleotide sequence or an amino acid sequence, and preferably at least 70% homology therewith. The suitable sequence homology is more preferably greater than 70%, further preferably is 80% or more, still further preferably is 90% or more, and still more preferably is 95% or more.

Examples of a DNA that has sequence homology to the DNA represented by the nucleotide sequence as set forth in SEQ ID NO: 1 include a DNA comprising a nucleotide sequence having a mutation including a deletion, substitution, addition or insertion of one or more, for example 1 to 100, preferably 1 to 30, more preferably 1 to 20, further preferably 1 to 10, and particularly preferably 1 or a few nucleotides in the nucleotide sequence of the DNA represented by the nucleotide sequence as set forth in SEQ ID NO: 1. A preferable DNA is a DNA of this kind that encodes a protein having the above described biological function. The degree of mutation and the location thereof and the like are not particularly limited, as long as a DNA having the mutation has similar structural characteristics as the above DNA and has a biological function that is equivalent to that of the protein encoded by the DNA comprising the nucleotide sequence represented by SEQ ID NO: 1.

A DNA having this kind of mutation may be a natural DNA or may be a DNA obtained by introduction of mutation on the basis of a gene existing in nature. Techniques for introducing mutation are known, for example, site-directed mutagenesis, genetic homologous recombination, primer extension, and polymerase chain reaction (hereunder, abbreviated as PCR), and these techniques can be used independently or in suitable combinations thereof. For example, mutation may be introduced in accordance with a method described in publications (Sambrook et al., Eds., "Molecular Cloning, A Laboratory Manual, 2nd Edition", 1989, Cold Spring Harbor Laboratory; Muramatsu S., Ed., "Labomanual Genetic Engineering", 1988, Maruzen Co., Ltd.) or by modifying these methods, and Ulmer's technique (Ulmer, K. M., "Science", 1983, Vol. 219, p.666-671) may also be utilized.

As a further example of the DNA used in the present invention, a DNA that hybridizes with the DNA represented by the nucleotide sequence as set forth in SEQ ID NO: 1 and a splicing valiant of the DNA under stringent conditions may also be mentioned. The hybridization conditions can, for example, be accordance with a method described in publications (Sambrook et al., Eds., "Molecular Cloning, A Laboratory Manual, 2nd Edition", 1989, Cold Spring Harbor Laboratory) or the like. More specifically, the phrase "under stringent conditions" refers to, for example, conditions of heating at 42 °C in a solution containing 6× SSC, 0.5% SDS and 50% formamide, and then washing at 68 °C in a solution containing 0.1× SSC and 0.5% SDS. As long as these DNAs are DNAs that hybridize with the DNA represented by the nucleotide sequence as set forth in SEQ ID NO: 1 and a splicing valiant of the DNA, they are not necessary to be DNAs having the complementary sequence thereof. Preferably, the DNA is a DNA encoding a protein that has an equivalent function to that of the protein encoded by the DNA represented by the nucleotide sequence as set forth in SEQ ID NO: 1 and a splicing valiant ofthe DNA.

Examples of a protein that has sequence homology to the protein encoded by the DNA represented by the nucleotide sequence as set forth in SEQ ID NO: 1 include a protein comprising an amino acid sequence having a mutation including a deletion, substitution, addition or insertion of one or more, for example 1 to 100, preferably 1 to 30, more preferably 1 to 20, further preferably 1 to 10, and particularly preferably one or a few amino acids in the amino acid sequence represented by SEQ ID NO: 2 and having the aforementioned biological function. The degree of mutation of the amino acids and the positions and the like thereof are not particularly limited, as long as the protein having the mutation has an equivalent function to that ofthe protein represented by the amino acid sequence as set forth in SEQ ID NO: 2.

A protein having the mutation may be a protein that was naturally produced by, for example, mutation or posttranslational modification, or may be a protein obtained by introduction of mutation based on a gene existing in nature. Techniques for introducing mutation are known, for example, site-directed mutagenesis, genetic homologous recombination, primer extension, and PCR, and these techniques can be used independently or in suitable combinations thereof. For example, a mutation may be introduced in accordance with a method described in publications (Sambrook et al., Eds., "Molecular Cloning, A Laboratory Manual, 2nd Edition", 1989, Cold Spring Harbor Laboratory; Muramatsu Masami., Ed., "Labomanual Genetic Engineering", 1988, Maruzen Co., Ltd.) or by modifying these methods, and Ulmer's technique (Ulmer, K. M., "Science", 1983, Vol. 219, p.666-671) can also be utilized. When introducing mutation, from the viewpoint of not altering the fundamental properties (physical properties, function, physiological activity, immunological activity or the like) of the protein, for example, mutual substitution among homologous amino acids (polar amino acids, nonpolar amino acids, hydrophobic amino acids, hydrophilic amino acids, positively charged amino acids, negatively charged amino acids and aromatic amino acids and the like) is easily conceivable.

Examples of the structural characteristic of the DNA include a seven-span transmembrane domain coding region and a TSP-I domain coding region. Besides, examples of the structural characteristic of the protein include a seven-span transmembrane domain and a TSP-I domain. A preferable DNA or protein has sequence homology in these types of regions or domains of preferably at least 70%, more preferably greater than 70%, further preferably is 80% or more, still further preferably is 90% or more, and still more preferably is 95% or more. It is further preferable that these domains retain a function thereof, for example, a function of localizing a protein that comprises the domain on a membrane or an angiogenesis inhibiting function.

In addition, examples of the structural characteristic of the DNA include a conserved region encoding an amino acid sequence (QTEV) as set forth in SEQ ID NO: 3 that are present in the 3'-terminal region of the DNA. The structural characteristics of the protein encoded by the DNA can be exemplified by a conserved amino acid sequence (QTEV) as set forth in SEQ ID NO: 3 that are present in the C-terminal region of the protein.

As an example of functions equivalent to the biological functions of the protein encoded by the DNA represented by the nucleotide sequence as set forth in SEQ ID NO: 1, a function as a membrane protein receptor can be mentioned. The phrase "function as a membrane protein receptor" refers to a function of being expressed as a membrane protein when expressed in an animal cell and promoting intracellular signal transduction to induce a cell response when receiving the action of a ligand. For example, an equivalent function to a GPCR may be mentioned. The phrase "equivalent function to a GPCR" refers to a function of binding to G protein when receiving the action of a ligand and activating the G protein to promote intracellular signal transduction resulting in induction of a cell response.

As specific examples of a cell response, a change in cell membrane potential or a change in intracellular calcium concentration can be mentioned. A change in cell membrane potential or a change in intracellular calcium concentration can be measured by a known method. A change in cell membrane potential can be detected, for example, by expressing the DNA represented by the nucleotide sequence as set forth in SEQ ID NO: 1 or a splicing valiant of the DNA in Xenopus laevis oocyte, measuring the amount of the membrane protein receptor-specific generation of current in the presence and absence of ligand stimulation, and comparing the amounts of current. A change in intracellular calcium concentration can be detected, for example, by making a cell incorporate a fluorescent substance that is capable of binding to calcium ion, eliciting a fluorescence phenomenon by excitation light in the presence and absence of ligand stimulation, and comparing the fluorescence amounts.

Examples of a ligand include a sample prepared from a cell or a biological tissue in which expression of the DNA represented by the nucleotide sequence as set forth in SEQ ID NO: 1 or a splicing valiant of the DNA was recognized. Sample preparation can be carried out, for example, by culturing cells or tissue according to a known method, and then employing a method such as centrifuging or the like to obtain the culture supernatant, or a method which disrupts or lyses the cells or tissue by a known method. Besides, a ligand can be purified for use from these samples by a known protein purification method, for example, gel filtration chromatography. Specific examples of a ligand include, but are not limited to, culture supernatant of the HeLa cell line that was used in the present example, and any substance can be used as a ligand as long as it can act on the gene product of the DNA that expressed in a cell to induce a cell response.

More preferably, CCK-8S (SEQ ID NO: 14) can be exemplified as a ligand.

As an example of functions equivalent to the biological functions of the protein encoded by the DNA represented by the nucleotide sequence as set forth in SEQ ID NO: 1, a function of interacting with a protein that has a guanylate kinase activity and/or a cell adhesion function, for example, a MAGUK family protein, may be also mentioned. Specific examples of MAGUK family protein include DLG2, DLG3 DLG4, AIP1 and MAGI3.

As described above, the DNA represented by the nucleotide sequence as set forth in SEQ ID NO: 1 and a splicing variant of the DNA are DNAs encoding a protein that works as a functional membrane protein receptor having a seven-span transmembrane domain. Regarding the structural characteristics, the protein encoded by the DNA has several, preferably two to four TSP-I domains, one GPS domain and one seven-span transmembrane domain (see Fig. 1-A and Fig. 1-B).

The DNA represented by the nucleotide sequence as set forth in SEQ ID NO: 1 comprises a nucleotide sequence of 4557 bp that contains an open reading frame (ORF) encoding 1518 amino acid residues (SEQ ID NO: 2) that have a portion predicted to be a signal sequence (20 amino acid residues from N- terminus).

The protein encoded by the DNA represented by the nucleotide sequence as set forth in SEQ ID NO: 1 is preferably exemplified by a protein represented by the amino acid sequence as set forth in SEQ ID NO: 2.

The protein encoded by the DNA represented by the nucleotide sequence as set forth in SEQ ID NO: 1 comprises 1518 amino acid residues (SEQ ID NO: 2) having a portion predicted to be a signal sequence (20 amino acid residues from N-terminus) and has a GPS domain and a seven-span transmembrane domain (seven-span transmembrane domain) in amino acid sequence thereof in addition to three TSP-I domains (see Fig. 1-A and Fig. 1-B). The amino acid sequence of this protein is identical with the amino acid sequence of the protein encoded by the DNA represented by the nucleotide sequence as set forth in SEQ ID NO: 19 except for the deleted 55 amino acid residues including one TSP-I domain at N- terminal side. The deleted 55 amino acid residues correspond to those from glycine (G) at position 296 to proline (P) at position 350 in the amino acid sequence of the protein represented by the amino acid sequence as set forth in SEQ ID NO: 20. The three TSP-I domains respectively comprise the region from histidine (His) at position 297 to proline (Pro) at position 350, the region from glutamic acid (Glu) at position 352 to proline (Pro) at position 405, and the region from aspartic acid (Asp) at position 408 to proline (Pro) at position 461, in the amino acid sequence represented by SEQ ID NO: 2. The seven transmembrane domains respectively comprise the region from valine (Val) at position 870 to phenylalanine (Phe) at position 890, the region from serine (Ser) at position 899 to glycine (Gly) at position 919, the region from valine (Val) at position 928 to leucine (Leu) at position 948, the region from arginine (Arg) at position 970 to threonine (Thr) at position 990, the region from alanine (Ala) at position 1012 to phenylalanine (Phe) at position 1032, the region from leucine (Leu) at position 1087 to alanine (Ala) at position 1107, and the region from valine (Val) at position 1114 to valine (Val) at position 1134 in the amino acid sequence represented by SEQ ID NO: 2.

The DNA represented by the nucleotide sequence as set forth in SEQ ID NO: 15 comprises a nucleotide sequence of 4389 bp that contains ORF encoding 1463 amino acid residues (SEQ ID NO: 16) that have a portion predicted to be signal sequence (20 amino acid residues from N-terminus).

The protein encoded by the DNA represented by the nucleotide sequence as set forth in SEQ ID NO: 15 is preferably exemplified by a protein represented by the amino acid sequence as set forth in SEQ ID NO: 16.

The protein encoded by the DNA represented by the nucleotide sequence as set forth in SEQ ID NO: 15 comprises 1463 amino acid residues having a portion predicted to be a signal sequence (20 amino acid residues from N-terminus), and has a seven-span transmembrane domain, two TSP-I domains and one GPS domain. (See Fig. 1-B). The amino acid sequence of this protein is identical with the protein encoded by the DNA represented by the nucleotide sequence as set forth in SEQ ID NO: 19 except for the deleted 110 amino acid residues containing two TSP-I domains at N-terminal side. The deleted 110 amino acid residues correspond to those from glycine (G) at position 296 to proline (P) at position 405 in the amino acid sequence of the protein represented by the amino acid sequence as set forth in SEQ ID NO: 20.

The DNA represented by the nucleotide sequence as set forth in SEQ ID NO: 17 comprises a nucleotide sequence of 4554 bp that contains ORF encoding 1518 amino acid residues (SEQ ID NO: 18) that have a portion predicted to be signal sequence (20 amino acid residues from N-terminus).

The protein encoded by the DNA represented by the nucleotide sequence as set forth in SEQ ID NO: 17 is preferably exemplified by a protein represented by the amino acid sequence as set forth in SEQ ID NO: 18.

The protein encoded by the DNA represented by the nucleotide sequence as set forth in SEQ ID NO: 17 comprises 1518 amino acid residues (SEQ ID NO: 18) having a portion predicted to be a signal sequence (20 amino acid residues from N-terminus), and has a seven-span transmembrane domain, three TSP-I domains and one GPS domain. (See Fig. 1-B). The amino acid sequence of this protein is identical with the protein encoded by the DNA represented by the nucleotide sequence as set forth in SEQ ID NO: 19 except for the deleted 55 amino acid residues containing one secondary TSP-I domain from N-terminal side. The deleted 55 amino acid residues correspond to those from valine (V) at position 351 to proline (P) at position 405 in the amino acid sequence of the protein encoded by the DNA represented by the nucleotide sequence as set forth in SEQ ID NO: 19.

The DNA represented by the nucleotide sequence as set forth in SEQ ID NO: 19 is referred to as 7tmHR (seven transmembrane helix receptor) gene (GenBank, Accession NO: AB065648). This DNA comprises a nucleotide sequence of 4719 bp that contains ORF encoding 1573 amino acid residues (SEQ ID NO: 20) having a portion predicted to be signal sequence (20 amino acid residues from N-terminus).

The protein encoded by the DNA represented by the nucleotide sequence as set forth in SEQ ID NO: 19 is preferably exemplified by a protein represented by the amino acid sequence as set forth in SEQ ID NO: 20.

The protein encoded by the DNA represented by the nucleotide sequence as set forth in SEQ ID NO: 19 is referred to as 7tmHR (GenBank, Accession NO: AB065648). This protein comprises 1573 amino acid residues and has a seven-span transmembrane domain, four TSP-I domains and one GPS domain in amino acid sequence thereof (see Fig. 1-B).

The DNA represented by the nucleotide sequence as set forth in SEQ ID NO: 21 is a known human DNA and is referred to as hBAI2 gene (GenBank, Accession No: AB005298). This DNA comprises a nucleotide sequence of 5399 bp that contains ORF encoding 1572 amino acid residues (SEQ ID NO: 22) having a portion predicted to be signal sequence (20 amino acid residues from N-terminus).

The protein encoded by the DNA represented by the nucleotide sequence as set forth in SEQ ID NO: 21 is preferably exemplified by a protein represented by the amino acid sequence as set forth in SEQ ID NO: 22.

The protein encoded by the DNA represented by the nucleotide sequence as set forth in SEQ ID NO: 21 is a known human protein and is referred to as hBAI2 (GenBank, Accession No: AB005298). This protein comprises 1572 amino acid residues and has a seven-span transmembrane domain, four TSP-I domains and one GPS domain. (See Fig. 1-A). The amino acid sequence of this protein is identical with the amino acid sequence of the protein encoded by the DNA represented by the nucleotide sequence as set forth in SEQ ID NO: 19 except for deletion of one amino acid residue corresponding to lysine at position 1461 in C-terminal region.

DNAs represented by any one of nucleotide sequences as set forth in SEQ ID NO: 1, 15, 17, 19 and 21 have homology to each other as mentioned above as well as the proteins encoded by the DNAs do, and conserve a TSP-I domain, GSP domain and seven-span transmembrane domain.

The inventors consider that the DNAs represented by any one of the nucleotide sequences as set forth in SEQ ID NO: 1, 15, 17, 19 and 21 and the proteins encoded by the DNAs are splicing variants from view points of homology in the sequence and similarity in structural characteristics.

The proteins represented by any one of amino acid sequences as set forth in SEQ ID NO: 2, 16, 18, 20 and 22 have homology to each other and conserve TSP-I domain, GPS domain and seven-span transmembrane domain. The inventors consider that these proteins are splicing variants from viewpoints of homology in sequence and similarity in structural characteristics.

A gene product of the DNA represented by any one of nucleotide sequences as set forth in SEQ ID NO: 15, 17 and 19 was actually exhibited the function as a membrane protein receptor. Specifically, it was observed that an animal cell expressing the gene product generated a cell response by CCK-8S (SEQ ID NO: 14) stimulation via intracellular signal transduction as similar to an animal cell expressing the gene product of the DNA represented by the nucleotide sequence as set forth in SEQ ID NO: 1.

A DNA utilized in the present invention can be a DNA represented by the nucleotide sequence that comprises any of the nucleotide sequences of the aforementioned DNAs, for example, the nucleotide sequences as set forth in SEQ ID NO: 1, 15, 17, 19 and 21.

In addition, a DNA utilized in the present invention can be a DNA fragment represented by a partial nucleotide sequence that is present in a designated region of the DNA represented by the nucleotide sequence as set forth in SEQ ID NO: 1 or of a splicing variant of the DNA. Such a DNA fragment is useful for use as primers or probes for detecting the DNA represented by the nucleotide sequence as set forth in SEQ ID NO: 1, or as primers for producing the DNA. The primer preferably consists of 15 to 30 nucleotides, and more preferably 20 to 25 nucleotides. The probe preferably consists of 8 to 50 nucleotides, more preferably 17 to 35 nucleotides, and further preferably 17 to 30 nucleotides. If the length of a primer or a probe is longer than a suitable length, the specificity decreases due to an increase in false hybridization. Further, if the length is shorter than a suitable length, the specificity decreases due to the occurrence of mismatches.

A designated region of the DNA represented by the nucleotide sequence as set forth in SEQ ID NO: 1 or of a splicing variant of the DNA is preferably exemplified by a region encoding a fragment of the protein that is encoded by the DNA and contains the site to which a ligand acts. A DNA fragment represented by a partial nucleotide sequence that is present in a region encoding a fragment containing a site to which a ligand acts can be used in the production of a fragment containing a site to which the ligand acts. A fragment containing a site to which a ligand acts is useful for detecting an action of a ligand to the protein utilized in the present invention, for example, binding between the protein and the ligand, or for identifying a compound that promotes or inhibits the action. Alternatively, the fragment is useful for identifying a compound having the same action as a ligand to the protein, i.e. an agonist. The minimum unit of this kind of DNA fragment preferably comprises five or more consecutive nucleotides in the region, more preferably ten or more nucleotides, and further preferably 20 or more nucleotides.

A DNA fragment represented by a partial nucleotide sequence that is present in a designated region of the DNA represented by the nucleotide sequence as set forth in SEQ ID NO: 1 or of a splicing variant of the DNA is also useful for use as an antisense oligonucleotide that inhibits expression of the DNA represented by the nucleotide sequence as set forth in SEQ ID NO: 1 or the splicing variant of the DNA, when it is a DNA fragment complementary to a sense strand encoding a protein. Since it is known that a DNA fragment consisting of approximately 20 nucleotides can generally inhibit expression of a gene, the antisense oligonucleotide consists of preferably 15 or more nucleotides, and more preferably 20 or more nucleotides.

These DNA fragments can be prepared according to a known chemical synthesis method by designing a fragment having a sequence in interest in accordance with the nucleotide sequence information of the DNA represented by the nucleotide sequence as set forth in SEQ ID NO: 1 or a splicing variant of the DNA. As a simple and convenient method, an automated DNA/RNA synthesizer can be used for preparing the DNA fragments.

A protein utilized in the present invention can be a protein represented by the amino acid sequence that comprises any of the nucleotide sequences of the aforementioned proteins, for example, the nucleotide sequences as set forth in SEQ ID NO: 2, 16, 18, 20 and 22.

In addition, a protein utilized in the present invention can be a fragment represented by a partial amino acid sequence that is present in a designated region of the protein. Such a fragment of the protein is useful for use as an antigen to produce an antibody against the protein.

A designated region of the protein used in the present invention can be preferably a site in the protein where a ligand acts to. A fragment containing a site where a ligand acts to is useful for detecting action of ligand to the protein, for example, for detecting binding between the protein and the ligand, or identifying a compound that promotes or inhibits the action. Alternatively, the fragment is useful for identifying a compound having a similar action as a ligand to the protein, that is, an agonist. Further, among fragments containing a site where a ligand acts to, a fragment that inhibits an interaction between the ligand and the protein is useful as a compound for inhibiting induction of a function of the protein by the action of the ligand.

A fragment represented by a partial amino acid sequence present in a designated region of the protein used in the present invention preferably comprises, as a minimum unit, five or more, more preferably eight or more, further preferably twelve or more, and still further preferably fifteen or more consecutive amino acids. These fragments can be prepared according to a known chemical synthesis method by designing a fragment having the target sequence in accordance with the amino acid sequence information of the protein.

A protein used in the present invention may be a protein prepared from cells in which a gene encoding the protein was expressed by genetic engineering techniques or from biological samples, or may be a synthetic product in a cell-free system or a chemical synthesis product. The protein can be further purified from these. The protein can also be a protein that is expressed in a cell that contains a gene encoding the protein. The cell can be a transformant obtained by transfection with a vector containing a gene encoding the protein.

A protein used in the present invention can be modified to the extent that no significant functional change is involved, such as modification of its constituent amino groups, carboxyl groups, or the like, for example, by an amidation or the like. Further, the protein may be labeled with the other protein or the like, that is added to the N-terminal or C-terminal, directly or indirectly via a linker peptide or the like by means of genetic engineering techniques, or the like. Labeling is preferably conducted in a way not to inhibit the fundamental properties of the present protein. Examples of the protein or the like to be added include, but are not limited to, enzymes such as GST, β-galactosidase, HRP or ALP, tag peptides such as His-tag, Myc-tag, HA-tag, FLAG-tag or Xpress-tag, fluorescent substances such as fluorescein isothiocyanate or phycoerythrin, maltose-binding protein, Fc fragment of immunoglobulin and biotin. Labeling can also be carries out using radioactive isotope. One or more kinds of labeling substances in combination can be added to the present protein. These labeling substances allow the detection and/or purification of the present protein to become easier, by measuring the substance itself, or the function thereof. In addition, these substances allow, for example, the detection of the binding of the present protein to the other protein

### (Preparation of DNA)

The DNA used in the present invention can be readily acquired by known genetic engineering techniques (refer to Sambrook et al., Eds., "Molecular Cloning, A Laboratory Manual, 2nd Edition", 1989, Cold Spring Harbor Laboratory; Muramatsu S., Ed., "Labomanual Genetic Engineering", 1988, Maruzen Co., Ltd.) based on sequence information of the DNA. For example, the DNAs represented by the nucleotide sequences as set forth in SEQ ID NO: 1, 15, 17, 19 and 21 in the sequence listing can be obtained by known genetic engineering techniques based on their sequence information.

More specifically, the DNA used in the present invention can be acquired by preparing a cDNA library in accordance with an ordinary method from a suitable origin in which expression of the DNA of the invention is confirmed, and then selecting a desired clone from the library using a specific suitable probe or primer to the DNA. Examples of the cDNA origin include various cells or tissues in which expression of the DNA is confirmed, or cultured cells derived from these. For example, the origin of the DNA represented by the nucleotide sequences as set forth in SEQ ID NO: 1 and splicing variants thereof can be human brain tissues and brain cells.

Isolation of total RNA from these origins, isolation and purification of mRNA, acquisition of cDNA and the cloning thereof and the like can each be carried out in accordance with an ordinary method. Further, a cDNA library can be constructed for use from commercially available polyA⁺RNA derived from the human brain, fetal brain or cerebral hippocampus. A method for selecting a desired clone from a cDNA library is also not particularly limited, and a commonly used method can be used. Examples thereof include a plaque hybridization method or colony hybridization method that uses a probe capable of binding selectively to the target DNA sequence, and a combination of these methods. As a probe used herein, a DNA or the like that was chemically synthesized on the basis of information regarding the nucleotide sequence of the DNA represented by the nucleotide sequence as set forth in SEQ ID NO: 1 or splicing variants of the DNA can generally be used. A sense primer and antisense primer that were designed on the basis of the nucleotide sequence information of the present DNA can also be used as this kind of probe.

Selection of a target clone from a cDNA library can be carried out, for example, by confirming an expression protein for each clone utilizing a known protein expression system, and using the biological function thereof as an indicator.

In addition, a DNA/RNA amplification method employing PCR (Ulmer, K. M., "Science", 1983, Vol. 219, pp.666-671; Ehrlich, H. A., Ed., "PCR Technology. Principles and Applications for DNA Amplification", 1989, Stockton Press; Saiki, R. K., et al., "Science", 1985, Vol. 230, pp.1350-1354) can be favorably utilized to acquire the DNA. When it is difficult to acquire full length cDNA from a cDNA library, a RACE method ("Jikken Igaku (Experimental Medicine)", 1994, Vol. 12, No. 6, p. 615-618), and particularly the 5'-RACE method (Frohman, M. A., "Proceedings of The National Academy of Sciences of The United States of America", 1988, Vol. 85, No. 23, pp. 8998-9002) or the like can be favorably employed. Primers to be used for PCR can be suitably designed based on the nucleotide sequence information of the DNA, and obtained by synthesis in accordance with an ordinary method. Isolation and purification of amplified DNA/RNA fragments can be carried out according to an ordinary method. For example, isolation and purification of amplified DNA/RNA fragments can be carried out by gel electrophoresis or the like.

Determination of the nucleotide sequence of DNA can be carried out by an ordinary method, for example, the dideoxy method ("Proceedings of The National Academy of Sciences of The United States of America", 1977, Vol. 74, pp.5463-5467) or the Maxam-Gilbert method (Methods in Enzymology", 1980, Vol. 65, p.499-560), or by simply using a commercially available sequencing kit, or the like.

The DNA may also be a DNA having one or more genes of, for example, enzymes such as glutathione S-transferase (GST), β-galactosidase, horseradish peroxidase (HRP) or alkaline phosphatase (ALP), or tag peptides such as His-tag, Myc-tag, HA-tag, FLAG-tag or Xpress-tag that are ligated to the 5'-terminal side or 3'-terminal side, as long as the function thereof, for example, expression of the protein encoded by the DNA or the function of the expressed protein, is not inhibited. Ligation of these genes can be carried out by using conventional gene manipulation techniques, and is useful to facilitate detection of a gene or mRNA.

### (vector)

A recombinant vector containing the DNA used in the present invention can be obtained by inserting the DNA into a suitable vector DNA. The recombinant vector may be any kind of recombinant vector, as long as it is a recombinant vector into which the DNA used in the present invention is incorporated.

A vector DNA is not particularly limited as long as it can be replicated within a host, and it can be suitably selected in accordance with the kind of host and purpose of use. The vector DNA may be extracted from a substance existing in nature, or may be one in which one part of a DNA segment other than a segment necessary for replication has been deleted. As typical examples, vector DNA derived from a plasmid, a bacteriophage or a virus may be mentioned. Examples of plasmid DNA include a plasmid derived from Escherichia coli, a plasmid derived from Bacillus subtilis, and a plasmid derived from yeast. Examples of a bacteriophage DNA include λ phage. Examples of vector DNA derived from a virus include a vector derived from an animal virus such as retrovirus, vaccinia virus, adenovirus, papovavirus, SV 40, fowlpox virus, and pseudorabies virus, or a vector derived from an insect virus such as baculovirus. Other examples thereof include vector DNA derived from a transposon, an insertion element, or a yeast chromosome element. Alternatively, vector DNA obtained by combining two or more of these, for example, vector DNA (cosmid or phagemid or the like) produced by combining genetic elements of a plasmid and a bacteriophage may be employed. Further, an expression vector or cloning vector or the like can also be used in accordance with the object.

A vector is required to have a target gene that is incorporated in such a way as to allow the function of the gene to appear, and contains at least the target gene sequence and a promoter as components thereof In addition to these components, as desired, one or a plurality of genetic sequences in combination selected from genetic sequences that encode information relating to replication and control, may be incorporated into the vector DNA by using a well-known method. Such genetic sequences can be exemplified by a ribosome binding sequence, terminator, signal sequence, cis element such as an enhancer, splicing signal, and a selective marker. A selective marker can be exemplified by dihydrofolate reductase gene, ampicillin-resistant gene and neomycin-resistant gene.

As a method of incorporating the target gene sequence into the vector DNA, any known method can be employed. For example, a method may be used which comprises treating the target gene sequence with suitable restriction enzymes to cleave it at specific sites, and then mixing it with a similarly treated vector DNA for ligation using a ligase. Alternatively, a desired recombinant vector can also be obtained by ligating a suitable linker to the target gene sequence, and then inserting it into the multi-cloning site of a vector suitable for the desired purpose.

### (transformant)

A transformant can be obtained by introducing a vector DNA containing the DNA used in the present invention into a host. When using an expression vector as the vector DNA, the DNA can be expressed, and a protein encoded by the DNA can also be produced. The transformant may further incorporate one or more kinds of vector DNAs containing a desired gene other than the present DNA.

Both prokaryotes and eukaryotes can be used as a host. Examples of the prokaryote include bacteria belonging to the *Escherichia* genus, such as, *Escherichia coli,* bacteria belonging to the *Bacillus genus*, such as, *Bacillus subtilis,* bacteria belonging to the *Pseudomonas genus*, such as, *Pseudomonas putida*, and bacteria belonging to the *Rhizobium* genus, such as, *Rhizobium meliloti.* Examples of the eukaryote include yeasts such as *Saccharomyces cerevisiae* and *Schizosaccharomyces pombe,* insect cells such as Sf9 and Sf2l, and animal cells such as monkey kidney-derived cells, (COS cells, Vero cells), Chinese hamster ovary cells (CHO cells), mouse L cells, rat GH3 cells, human FL cells or 293 EBNA cells, and Xenopus laevis oocyte. Preferably, animal cells are used.

Introduction of vector DNA into the host cell can be performed according to a known method, for example, by applying a standard method described in publications (Sambrook et al., Eds., "Molecular Cloning, A Laboratory Manual, 2nd Edition", 1989, Cold Spring Harbor Laboratory). When gene stability is a consideration, it is preferable to use a method that integrates the gene onto a chromosome. Meanwhile, it is convenient to use an autonomous replication system that utilizes an extranuclear gene. Specifically, calcium phosphate transfection, DEAE-dextran mediated transfection, microinjection, cationic lipid-mediated transfection, electroporation, transduction, scrape loading, ballistic introduction, infection, and the like, may be mentioned.

When employing an animal cell as the host, it is preferable that the recombinant vector is capable of autonomous replication within the cell and is composed of a promoter, RNA splice site, target gene, polyadenylated site and a transcription terminating sequence. As desired, it may also contain an origin of replication. As a promoter, SRα promoter, SV 40 promoter, LTR promoter, CMV promoter and the like can be used, and early gene promoter of cytomegalovirus and the like can also be used. As a method for introducing the recombinant vector into an animal cell, preferably, for example, electroporation, the calcium phosphate technique, lipofection or the like is used.

When employing a prokaryote as the host, it is preferable that the recombinant vector is capable of autonomous replication within the bacterium and is composed of a promoter, a ribosomal binding sequence, the target gene and a transcription terminating sequence. It may also contain a gene that regulates the promoter.

When employing bacteria as the host, the promoter is not particularly limited and any promoter may be used as long as it can work in a host such as Escherichia coli. For example, a promoter derived from Escherichia coli or a phage, such as trp promoter, lac promoter, PL promoter or PR promoter may be mentioned. An artificially designed and modified promoter such as tac promoter may also be used. A method for introducing a recombinant vector into bacteria is not particularly limited as long as it is a method that introduces the DNA into the bacteria. Preferable examples thereof include a method using calcium ion, the electroporation method, or the like.

When using yeast as a host, the promoter is not particularly limited and any promoter may be used as long as it can work in yeast. Examples thereof include gal1 promoter, gal10 promoter, heat shock protein promoter, MFα1 promoter, PH05 promoter, PGK promoter, GAP promoter, ADH promoter, and AOX1 promoter. A method for introducing a recombinant vector into yeast is not particularly limited as long as it is a method that introduces the DNA into the yeast. Preferable examples thereof include the electroporation method, the spheroplast method, the lithium acetate method, or the like.

When using an insect cell as the host, preferable examples of a method for introducing a recombinant vector include the calcium phosphate method, the lipofection method and the electroporation method.

As a specific example of the transformant obtained by trasnfecting with a vector DNA containing the DNA represented by the nucleotide sequence as set forth in SEQ ID NO: 1 in the sequence listing, HA-ph01207#10-6 cell line may be mentioned. HA-ph01207#10-6 cell line was established by transfecting the CHO-K1 cell line with a vector that allows for the expression of the DNA comprising the nucleotide sequence of the ORF of the DNA represented by the nucleotide sequence as set forth in SEQ ID NO: 1 from which a segment presumed to encode a signal sequence consisting of 20 amino acid residues from the N-terminus of the amino acid sequence represented by SEQ ID NO: 2 is excluded, as an N-terminal HA-tag fusion protein. The HA-ph01207#10-6 cell line stably expresses the N-terminal HA-tag fusion protein. A specific method for producing this cell line is described in detail in Example 2.

The HA-ph01207#10-6 cell line was deposited with the International Patent Organism Depositary of the National Institute of Advanced Industrial Science and Technology (Japan, Tsukuba Central 6, 1-1, Higashi 1-chome, Tsukuba, Ibaraki) on August 19, 2004 under Accession NO: FERM BP-10101. The existence of this cell line was confirmed by experiments at the International Patent Organism Depositary on September 22, 2004.

### (Method for producing the protein)

The protein used in the present invention can be produced, for example, by ordinary genetic engineering techniques based on the nucleotide sequence information of the gene encoding the protein (see, Sambrook et al., Eds., "Molecular Cloning, A Laboratory Manual, 2nd Edition", 1989, Cold Spring Harbor Laboratory; Muramatsu Masami., Ed., "Labomanual Genetic Engineering", 1988, Maruzen Co., Ltd.; Ulmer, K. M., "Science", 1983, Vol. 219, p.666-671; Ehrlich, H. A., Ed., "PCR Technology. Principles and Applications for DNA Amplification", 1989, Stockton Press). For example, the protein can be acquired by preparing a cDNA library in accordance with an ordinary method from various cells or tissues in which expression of the gene encoding the protein is confirmed or cultured cells derived from these, for example, human brain tissue, amplifying the gene encoding the protein using suitable primers that are specific to the gene, and inducing expression of the obtained gene by known genetic engineering techniques.

More specifically, for example, the protein can be produced by culturing a transformant transfected with a vector DNA containing the aforementioned DNA, and then recovering the protein of interest from the obtained culture. Cultivation of the transformant can be carried out according to a known culture conditions and culture method that are best suited to the respective hosts. The protein itself that are expressed by the transformant, or a function thereof can be used as an indicator for carrying out the cultivation. Alternatively, the protein itself or the protein amount thereof that is produced in the host or outside the host can be used as an indicator for carrying out the cultivation. Further, a subculture or a batch culture can be also carried out with employing the amount of the transformant in the culture medium as an indicator.

When the protein in interest expresses within the cell of the transformant or on the cell membrane, the protein in interest may be extracted from the disrupted transformant. Further, when the protein in interest is secreted outside the transformant, the culture medium can be used as it is or the culture medium can be used after removing the transformant by centrifugation or the like.

A protein used in the present invention can also be produced according to an ordinary chemical synthesis method. For example, solid phase synthesis, solution phase synthesis and the like are known as methods of chemically synthesizing a protein, and any of these methods can be used. These kinds of protein synthesis methods more specifically include a so-called stepwise elongation method that sequentially binds each amino acid, one at a time, to elongate a chain based on the amino acid sequence information, and a fragment condensation method that previously synthesizes fragments comprising several amino acids and subsequently subjects the respective fragments to a coupling reaction. Synthesis of the protein can be performed by either of these methods. A condensation method used for the above described protein synthesis can also be carried out according to an ordinary method, and examples thereof include an azide method, mixed anhydride method, DCC method, active ester method, oxidation-reduction method, DPPA (diphenylphosphoryl azide) method, DCC + additive (1-hydroxybenzotriazole, N-hydroxysuccinamide, N-hydroxy-5-norbornane-2,3-dicarboxyimide and the like) method, and Woodward's method. A protein obtained by chemical synthesis can be suitably purified in accordance with various kinds of common purification methods as described above.

A protein used in the present invention can be cleaved using a suitable peptidase for fragmentation, which consequently allows production of fragments of the protein.

As desired, the protein can be isolated and/or purified by various isolation methods that utilize the physical properties or chemical properties thereof Isolation and/or purification can be carried out employing a function of the protein as an indicator. Examples of an isolation method include, ammonium sulfate precipitation, ultrafiltration, gel chromatography, ion-exchange chromatography, affinity chromatography, high performance liquid chromatography, and dialysis. These methods may be used independently or in suitable combinations thereof. It is preferably recommended to employ a method that utilizes a specific antibody to the protein that is prepared based on the amino acid sequence information of the protein to specifically adsorb the protein, for example, affinity chromatography utilizing a column with the antibodies bound thereto.

### (Antibody)

The antibody can be produced using the protein used in the present invention or the fragment thereof as an antigen. The antigen may be the protein or a fragment thereof, and consists of at least eight, preferably at least ten, more preferably at least twelve and further preferably fifteen or more amino acids. In order to produce an antibody that is specific to the protein used in the present invention and/or a fragment thereof, a region comprising a characteristic amino acid sequence of the protein used in the present invention or a fragment thereof is preferably used. The amino acid sequence of this region need not necessarily be homologous or identical with a sequence of the protein or a fragment thereof, and a site that is exposed outward on the tertiary structure thereof is preferable, and even if the amino acid sequence of the exposure site is not continuous on the primary structure, it is sufficient if the amino acid sequence is continuous with respect to the exposure site. The antibody is not particularly limited as long as it can specifically bind to or recognize the protein used in the present invention and/or a fragment thereof immunologically. The presence or absence of this binding or recognition can be determined by a known antigen-antibody binding reaction.

A known antibody producing method can be utilized for production of the antibody. For example, the antibody can be obtained by administering to an animal an antigen alone, or an antigen bound to a carrier, with or without an adjuvant, and thereby inducing immunity, such as a humoral response, and/or a cell response. A carrier is not particularly limited as long as it does not itself exhibit an adverse action against the host and is capable of enhancing antigenicity. Examples thereof include cellulose, polymeric amino acids, albumin and keyhole limpet hemocyanin. Examples of the adjuvant include Freund's complete adjuvant (FCA), Freund's incomplete adjuvant (FIA), Ribi (MPL), Ribi (TDM), Ribi (MPL + TDM), Bordetella pertussis vaccine, muramyldipeptide (MDP), aluminium adjuvant (ALUM), and combinations of these. As an animal for immunization, mouse, rat, rabbit, goat, horse or the like is preferably used.

A polyclonal antibody can be acquired from serum of an animal that was administered with the antigen by using a known antibody recovery method. As a preferable example of an antibody recovery method, immunoaffinity chromatography may be mentioned.

A monoclonal antibody can be produced by recovering antibody-producing cells (for example, lymphocytes derived from spleen or lymph nodes) from an animal that was administered with the antigen, and introducing transforming means that uses known immortalized cells (for example, myeloma strain of the P3-X63-Ag8 line). For example, antibody-producing cells are fused with permanently proliferating cells by a known method to produce a hybridoma which is subsequently subjected to cloning and screening for a hybridoma that produces an antibody that specifically recognizes the protein used in the present invention. The antibody is then recovered from culture solution of that hybridoma.

A polyclonal antibody or a monoclonal antibody that can recognize and bind with the protein used in the present invention can be utilized as an antibody for purification of the protein, reagent, labeling marker, or the like. In particular, an antibody that inhibits the function of the protein, or an antibody that binds to the protein and exhibits a ligand-like action for the protein can be used for regulating the function of the protein. These antibodies are useful for elucidating, inhibiting, improving and/or treating various kinds of diseases attributable to an abnormality in the protein and the function thereof

### (Membrane protein receptor)

The protein used in the present invention is a protein that functions as a membrane protein receptor and was able to induce a cell response to CCK-8S (SEQ ID NO: 14) when expressed in animal cells. That is, CCK-8S (SEQ ID NO: 14) is one of ligands to a membrane protein receptor comprising the protein. Hereunder, a membrane protein receptor comprising the protein may also be referred to as "membrane protein receptor of the present invention".

The cell response caused by CCK-8S (SEQ ID NO: 14) in animal cells that expressed the protein used in the present invention was observed in, specifically, the above mentioned HA-ph01207#10-6 cell line in which the DNA represented by the nucleotide sequence as set forth in SEQ ID NO: 1 in the sequence listing was stably expressed. More specifically, an increase in intracellular calcium concentration was observed by action of CCK-8S (SEQ ID NO: 14) in the HA-ph01207#10-6 cell line (see Example 5). The degree of increase in intracellular calcium concentration in the cell line caused by 1nM of CCK-8S (SEQ ID NO: 14) was roughly equal to that caused by calcium ionophore A23187 as a positive control. In CHO-K1 cell line that did not express the DNA, this kind of increase in intracellular calcium concentration caused by CCK-8S (SEQ ID NO: 14) was not observed. Meanwhile, the HA-ph01207#10-6 cell line did not respond to a peptide (CCK-8 Nonsulfated form, hereunder referred to as "CCK-8NS") in which the seventh tyrosine residue from C-terminus was not sulfated, even though the peptide was a CCK octapeptide consisting of the same amino acid sequence as CCK-8S (SEQ ID NO: 14). Further, the HA-ph01207#10-6 cell line did not respond to a tetrapeptide (CCK-4) consisting of the amino acid residues up to fourth residue from the C-terminus of CCK-8S (SEQ ID NO: 14). That is to say, an increase in intracellular calcium concentration caused by 1 nM of CCK-8NS or 1 nM of CCK-4 was not observed. It was thus clarified that the HA-ph01207#10-6 cell line responds specifically to CCK-8S (SEQ ID NO: 14) and functions as a membrane protein receptor. Furthermore, since the HA-ph01207#10-6 cell line responded to CCK-8S (SEQ ID NO: 14) but not to CCK-8NS, the inventors consider that sulfated seventh tyrosine residue from the C-terminus of the amino acid sequence of CCK-8S (SEQ ID NO: 14) is important for the ligand action of CCK-8S (SEQ ID NO: 14).

The CCK-8S (SEQ ID NO: 14)-induced cell response was also observed in cells in which the DNA represented by the nucleotide sequence as set forth in SEQ ID NO: 15, the DNA represented by the nucleotide sequence as set forth in SEQ ID NO: 17, or the DNA represented by the nucleotide sequence as set forth in SEQ ID NO: 19 was expressed. More specifically, increased intracellular calcium concentration in these cells was observed by an action of 1 nM CCK-8S (SEQ ID NO: 14) (Example 8). Meanwhile, such an increase in intracellular calcium concentration by CCK-8S (SEQ ID NO: 14) was not observed in CHO-K1 cell line in which the DNA represented by the nucleotide sequence as set forth in SEQ ID NO: 15, the DNA represented by the nucleotide sequence as set forth in SEQ ID NO: 17, or the DNA represented by the nucleotide sequence as set forth in SEQ ID NO: 19 was not expressed.

The protein encoded by the DNA represented by any one of nucleotide sequences as set forth in SEQ ID NO: 1, 15, 17 and 19 was different to each other in number of repeats of TSP-I domain in the N-terminal extracellular region, but has the same amino acid sequence except for the domain (Fig.1-B). A cell that was made to express the DNA represented by any one of nucleotide sequences as set forth in SEQ ID NOs: 1, 15, 17 and 19 showed a cell response by CCK-8S (SEQ ID NO: 14) (Example 5, Example 8, and Example 9). Therefore, the inventors consider that TSP-1 domain does not largely participate in the binding of CCK-8S to the protein encoded by the DNA represented by any one of the nucleotide sequences as set forth in SEQ ID NOs: 1, 15, 17 and 19, and in the intracellular signal transduction caused by the binding.

Since CCK-8S (SEQ ID NO: 14) caused a cell response even at low concentration of 1 nM in the HA-ph01207#10-6 cell line and in cells in which the DNA represented by the nucleotide sequence as set forth in SEQ ID NO: 15, SEQ ID NO: 17, or SEQ ID NO: 19 was expressed, it can be considered that CCK-8S (SEQ ID NO: 14) actually causes a cell response *in vivo* through the protein encoded by the DNA. That is, the inventors consider that CCK-8S (SEQ ID NO: 14) may be one of *in vivo* ligands to a functional membrane protein receptor of the present invention that is presumed to be GPCR. Further, the membrane protein receptor exhibited a cell response such as a change in membrane potential in *Xenopus laevis* oocyte when culture supernatant of HeLa cells was used as the ligand source.

In addition to CCK-8S (SEQ ID NO: 14), a peptide that comprises an amino acid sequence having a mutation including a deletion, substitution, addition or insertion of one or several amino acids in the amino acid sequence of CCK-8S (SEQ ID NO: 14) and has an equivalent function to CCK-8S is also included in the scope of the ligand of the membrane protein receptor of the present invention. The phrase "equivalent function to CCK-8S" refers to a function that induces a biological function of the membrane protein receptor of the present invention, and more specifically to a function that induces a cell response, such as an increase in intracellular calcium concentration or a change in membrane potential, in a cell expressing the membrane protein receptor of the present invention. Since the sulfated seventh tyrosine residue from the C-terminus of the amino acid sequence of CCK-8S (SEQ ID NO: 14) is important for the ligand action of CCK-8S (SEQ ID NO: 14), it is preferable for the ligand of the membrane protein receptor of the present invention to retain the sulfated tyrosine residue therein. A protein having the mutation may be a protein that was naturally produced by, for example, a mutation or posttranslational modification, or may be a protein obtained by introducing a mutation based on a gene existing in nature. Techniques for introducing a mutation are known, and the above described methods can be used. From the viewpoint of not altering the fundamental properties (physical properties, functions, physiological activity, immunological activity or the like) of the protein in interest, for example, mutual substitution among homologous amino acids (polar amino acids, nonpolar amino acids, hydrophobic amino acids, hydrophilic amino acids, positively charged amino acids, negatively charged amino acids and aromatic amino acids and the like) is easily conceivable. Further, peptides that contain CCK-8S (SEQ ID NO: 14) or contain a peptide comprising an amino acid sequence having a mutation of one or a few amino acids in the amino acid sequence of CCK-8S (SEQ ID NO: 14) and having an equivalent function to CCK-8S are included in the scope of the ligand of the membrane protein receptor of the present invention. In this kind of peptide, it is preferable that the sulfated tyrosine residue present at the seventh position from the C-terminus of CCK-8S is retained.

Tissue expression of CCK is observed mainly in brain tissues, especially in brain cortex, hippocampus, amygdaloid body, and hypothalamus (see Table 3).

The inventors found out that tissue expression of a DNA encoding the protein used in the present invention was observed in, specifically, brain cortex, hippocampus and amygdaloid body remarkably strongly (see Example 10 and Table 3). Since distribution of the expression of the DNA encoding the protein used in the present invention is identical with that of CCK, the inventors consider that a ligand of the membrane protein receptor is CCK, for example, CCK-8S (SEQ ID NO: 14).

Since one of the ligands to the membrane protein receptor of the present invention is CCK-8S (SEQ ID NO: 14) as described above, the inventors consider that the membrane protein receptor of the present invention is involved in neural functions such as memory retention in which CCK-8S (SEQ ID NO: 14) is thought to participate. A reduction in the quantity and/or function of CCK-8S (SEQ ID NO: 14) or the disappearance thereof causes the appearance of pathologic symptoms such as difficulty in recalling memory to a conscious level and translation into action. The inventors consider that CCK-8S (SEQ ID NO: 14) acts to this kind of neural function through the membrane protein receptor of the present invention. Therefore, diseases or symptoms accompanying this kind of impairment of memory function can be alleviated by an agonist of the membrane protein receptor of the present invention. Examples of this kind of disease include diseases accompanying impairment of neural function such as memory. Specifically for example, dementia and Alzheimer's disease and the like may be mentioned.

The inventors therefore consider that the membrane protein receptor of the present invention participates as a CCK receptor in physiological functions such as anxiety, analgesia, sedation, ingestion suppression, memory and learning, in which CCK has been assumed to be involved. Furthermore, CCK is reported to exhibit various actions in digestive organs, and is considered to work as a signaling substance that imparts a sensation of satiety to brain neurons. The inventors therefore consider that CCK-8S, a member of the CCK family, also acts as a signaling substance that imparts a sensation of satiety to brain neurons. The inventors consider that a quantitative and functional decline in CCK-8S causes obesity due to a decline in a sensation of satiety. The inventors consider that the membrane protein receptor of the present invention is involved in this kind of obesity as a CCK receptor. Further, it is reported that CCK-8S administration to diabetes patients resulted in promotion of increase in insulin amounts and suppression of increase in postcibal glucose amounts (Bo, A. et al., "The Journal of Clinical Endocrinology & Metabolism", 2000, Vol. 85, pp. 1043-1048). Thus, there is a possibility that the functional membrane protein receptor of the present invention is associated with diabetes. Accordingly, an agonist or antagonist of the membrane protein receptor of the present invention can alleviate a disease or symptoms accompanied with impairment of this kind of physiological function. More specifically, an agonist or antagonist of the membrane protein receptor of the present invention can be used as an effective ingredient of an anti-anxiety drug, an analgesic preparation or a preventive and/or therapeutic agent for diseases caused by various kinds of abnormalities of the central nervous system. Specific examples of these kinds of diseases include dementia, Parkinson's disease, panic syndrome, drug dependence, obesity, diabetes and the like.

More specifically, the inventors consider that the membrane protein receptor of the present invention is involved in neurological diseases such as depression, from viewpoints of expression distribution of the DNA encoding the receptor protein and results of experiments using knockout mouse of a splicing variant of the DNA.

"Depression", also referred to as depressive illness, is emotional mental disorder with chief complaints of emotional disturbance such as sorrow feeling or the like, thinking disturbance such as inhibition of thought or the like, hypobulia, behavioral suppression, sleep disorder, daily fluctuation of depression state or the like. It is said that reduction in the neurotransmitter in the brain is responsible for depression. Further, it is said that biological factors, psychological factors, social and environmental factors are involved in the development of this disorder.

"Depression state" refers to symptoms generally observed in depression, for example, emotional disturbance such as sorrow feeling or the like, thinking disturbance such as inhibition of thought or the like, hypobulia, behavioral suppression, sleep disorder or the like.

The expression of a DNA encoding the protein used in the present invention was observed strongly in brain cortex, hippocampus and amygdaloid body as mentioned above (see Example 10 and Table 3). It is reported that amygdaloid body is involved in depression (Whalen P.J. et al., "Seminars in Clinical Neuropsychiatry", 2002, Vol. 7, No. 4, p.234 242; Drevets W.C. et al., "Annals of the New York Academy of Sciences", 2003, Vol. 985, p. 420 444; Nestler E.J. et al., "Neuron", 2002, Vol. 34, No. 1 p. 13 25).

A BAI2 gene knockout mouse showed a contra depression-like phenotype in the tail suspension test (see Example 11). BAI2 gene is a splicing variant of the DNA encoding the protein used in the present invention. The tail suspension test is a common technique as a test method for investigating a depression phenotype and is used as the test system for studying the association with depression such as assessment of anti-depressant drug or the like (Steru L. et al., "Psychopharmacology (Berl)", 1985, Vol. 85, No. 3, p. 367-370; Crowley J.J. et al., "Pharmacological Biochemical Behavior",2004, Vol. 78, No. 2, P.269-274; Nielsen D.M. et al., "European Journal of Pharmacology", 2004, Vol. 499, Nos. 1-2, P.135-146).

The BAI2 gene knockout mouse showed a contra depression-like phenotype in the tail suspension test, but did not show increase in any behavioral activity in other behavioral examinations. In addition, the knockout mouse did not show any significant difference compared with wild type mouse with respect to many examination items including physiological examination, pathological examination, anatomical examination or the like.

Because of destroyed BAI2 gene in a BAI2 gene knockout mouse, BAI2 gene and splicing variants thereof are not expressed. In other words, a contra-depression state was induced in the mouse lacking the gene products of BAI2 gene and splicing variants thereof From the results thus obtained, the inventors consider that the gene products of BAI2 gene and splicing variants thereof are involved in depression.

The inventors consider that even in human, BAI2 gene and a splicing variant thereof, i.e., the DNA encoding the protein used in the present invention and splicing variants thereof are involved in depression.

Meanwhile, two kinds of GPCRs, CCK-A receptor (also referred to as "CCK1 receptor") and CCK-B receptor (also referred to as "CCK2 receptor"), have been reported as CCK receptors (Herranz, R., "Medicinal Research Reviews", 2003, Vol. 23, No. 5, pp. 559-605, Review). Both of these belong to class Arhodopsin-like GPCR.

The membrane protein receptor of the present invention are different from CCK-A receptor and CCK-B receptor in ligand affinity and expression distribution, therefore, the inventors believe that the present membrane protein receptor works for a physiological action different from that of CCK-A receptor and CCK-B receptor. Specifically, the present membrane protein receptor has similar ligand affinity as that of CCK-A receptor with different expression distribution, and has similar expression distribution as that of CCK-B receptor with different ligand affinity

The DNA encoding the protein used in the present invention is expressed highly and specifically in brain tissues, therefore, the inventors consider that the present membrane protein receptor comprising the protein is mainly involved in action of CCK in the central nervous system in comparison to CCK-A receptor which is lowly expressed in brain tissues. Further, the inventors consider that the membrane protein receptor of the present invention may show a different physiological activity in the brain from that of CCK-B receptor, since it is different in ligand affinity from CCK-B receptor expressed in brain tissues.

Further, a contra-depression state was observed in the knockout mouse of the present membrane protein receptor, as mentioned above. In the meantime, there have been some reports describing phenotypes of a CCK-A receptor gene knockout mouse, a CCK-B receptor gene knockout mouse and a double knockout mouse of CCK-A receptor gene and CCK-B receptor gene, as well as a report describing an enhanced behavioral activity in the CCK-B receptor gene knockout mouse. However, there is no report indicating a possible association between these knockout mice and depression.

Although the present membrane protein receptor, CCK-A receptor and CCK-B receptor are receptors all of which is responsive to CCK, the inventors consider that the present membrane protein receptor alone among them is a membrane protein receptor associated with depression from viewpoints of their tissue expression distribution and phenotypes of the knockout mice. Further, the inventors consider that CCK-A receptor is not involved in physiological activity of CCK in brain tissue because of its low expression in brain tissue. Although CCK-B receptor is expressed in brain tissue, there is no report that suggests possible association between depression and phenotypes of knockout mice of the gene encoding CCK-B receptor protein.

CCK-A receptor is strongly expressed mainly in digestive organs, and is observed to be expressed in a part of brain tissues. The ligand affinity of CCK-A receptor is in the order of CCK-8S >> CCK-8NS, gastrin > CCK-4. CCK-A receptor responds strongly to CCK-8S (SEQ ID NO: 14), but does not have any ligand specificity.

CCK-B receptor is expressed widely in brain tissues as well as digestive organs. The ligand affinity of CCK-B receptor is in the order of CCK-8S ≥ CCK-8NS, gastrin > CCK-4, indicating that it has lower selectivity than CCK-A receptor.

The phenotypes of a CCK-A receptor knockout mouse have been reported, such as abnormal homeostasis of body temperature regulation or the like (Nomoto S. et al., "American journal of physiology. Regulatory integrative and comparative physiology", 2004, Vol. 287, No. 3, R556-61), an increased behavioral activity (Miyasaka K. et al., "Neuroscience Letters", 2002, Vol. 335, No. 2, p.115-118), and abnormal appetite regulation (Bi S. et al., "Neuropeptides", 2002, Vol. 36, Nos. 2-3, p.171-181) in addition to dysfunction in the digestive system such as biliary calculus, and abnormality of pancreatic enzyme secretion and gallbladder contraction, or the like.

The phenotypes of a CCK-B receptor knockout mouse have been reported in many reports, some of which demonstrate a central nervous system phenotype such as anxiety, pain, memory or the like (Noble F. et al., "Neuropeptides", 2002, Vol. 36, Nos. 2-3, P.157-170), in addition to abnormality of the digestive system such as abnormal gastric secretion and gastric mucosa malformation. More specifically, there have been reported reduced anxiety related behavior (Horinouchi Y et al., "European Neuropsychopharmacology", 2004, Vol. 14, No. 2, p.157-161), enhanced anxiety related behavior (Miyasaka K. et al., "Neuroscience Letters", 2002, Vo1. 335, No.2, p115-118), enhanced behavioral activity and memory impairment (Dauge V. et al., "Neuropsychopharmacology", 2001, Vol. 25, No. 5, p.690-698), dysalgesia and correlation with opioid system (Kurrikoff K. et al., "The European Journal of Neuroscience", 2004, Vol. 20, No. 6, p.1577-1586) or the like have been reported.

Further, the phenotypes of a double knockout mouse of CCK-A receptor and CCK-B receptor have been reported (Miyasaka K. et al., "Neuroscience Letters", 2002, Vol. 335, No. 2, p.115-118); however characteristic phenotypes of double knockout mouse has not been found.

The inventors consider that a DNA encoding the protein used in the present invention. i.e., the DNA represented by the nucleotide sequence as set forth in SEQ ID NO: 1 and splicing variants of the DNA, is involved in depression. For example, the inventors consider that depression state and depression are induced by such an abnormality that expression of the DNA represented by the nucleotide sequence as set forth in SEQ ID NO: 1 or splicing variants of the DNA is increased.

Since it can be considered that the DNA represented by the nucleotide sequence as set forth in SEQ ID NO: 1 and splicing variants of the DNA are involved in depression, it is possible to improve depression state and to recover depression by inhibiting a function and/or expression of any one of proteins selected from the group consisting of the protein encoded by the DNA and splicing variants of the protein.

The present invention relates to a method for improving depression state by inhibiting the function and/or expression of any one of proteins selected from the group consisting of the protein encoded by the DNA represented by the nucleotide sequence as set forth in SEQ ID NO: 1 and splicing variants of the protein.

The phrase "improving depression state" means that depression state is alleviated or cured compared with a state before the method for improving depression state is attempted. For example, this means that emotional disturbance, thinking disturbance, hypobulia, behavioral suppression, sleep disorder or the like is alleviated or cured.

Inhibition of the function and/or expression of the protein used in the present invention can be executed by, for example, a compound that inhibits the function and/or expression of the protein.

In the present specification, the phrases "a compound inhibiting a function of the protein used in the present invention" and "an antagonist of the protein" are used interexchangeably. In the present specification, "an antagonist" can be any compound that inhibits the function of the protein used in the present invention. Such compounds include a compound that binds to a receptor and inhibits an effect of an agonist, but is unable to exert an effect that is exhibited by the agonist even after the binding to the receptor, a compound that inhibits binding of a ligand to a receptor, or a compound that acts on the present protein as an inverse agonist. In recent years, it has been known that a receptor such as GPCR is converted from active type to inactive type, or from inactive type to active type, irrespective of an action of a ligand. In this specification, a substance that inhibits a step where a receptor such as GPCR is converted from inactive type to active type irrespective of an action of a ligand is referred to as an inverse agonist. It can be considered that the inverse agonist of the present protein also inhibits the function of the protein used in the present invention.

The antagonist of the protein used in the present invention binds to a membrane protein receptor comprising the present protein and inhibits an effect of a ligand. Since it is considered that depression state and depression are induced by an abnormality such as increase in expression of the DNA encoding the protein, the inventors consider that depression state and depression can be improved by inhibiting an action of a ligand to a receptor comprising the protein by the antagonist of the protein.

Thus, it is considered that the antagonist of the protein used in the present invention has an anti-depressant action. The phrase "Anti-depressant action" refers to an effect that improves depression state.

A compound that inhibits the function of the protein used in the present invention, for example, the antagonist of the protein is preferably an antagonist that inhibits the function caused by CCK-8S through the membrane protein receptor comprising the protein. Further, the antagonist may be an antagonist that inhibits the function caused by a peptide having an equivalent function to that of CCK-8S through the membrane protein receptor comprising the protein.

The antagonist of the protein used in the present invention can be obtained by a method for identifying a compound which will be explained later. As the antagonist of the protein if a ligand of the membrane protein receptor comprising the protein is a protein, a substance that is a partial peptide of the ligand and binds to the membrane protein receptor, but is unable to exert an effect like that exhibited by the ligand, can be used. A partial peptide of a ligand of the membrane protein receptor can be obtained by identifying the ligand and then designing and synthesizing a number of partial peptides from the amino acid sequence thereof, followed by selecting those having an activity as an antagonist from the synthesized partial peptides by a method for identifying a compound which is explained later.

As the antagonist of the protein used in the present invention, three kinds of compounds (structural formula (I), (II) and (III)) identified by a method for identifying a compound which is explained later can be exemplified (see Example 12).

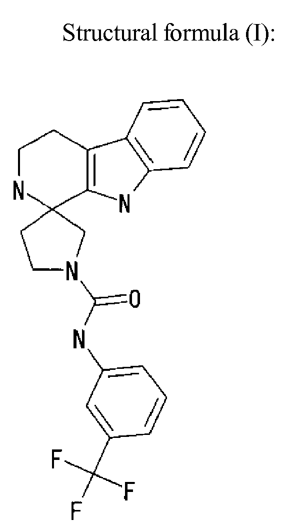

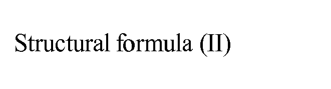

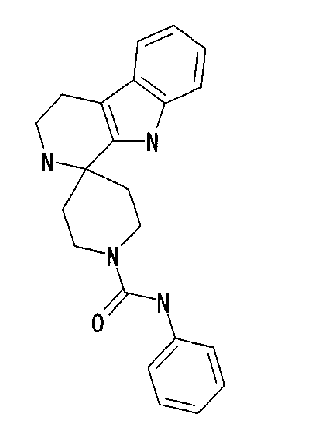

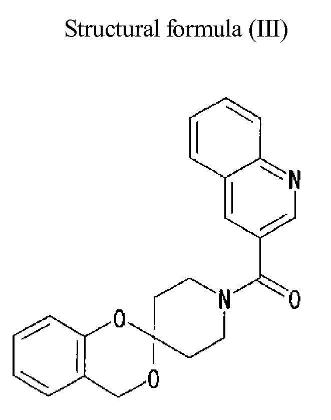

Further, the present invention can provide an agent for improving depression state, comprising a compound that inhibits the function and/or expression of any one of proteins selected from the group consisting of a protein encoded by the DNA represented by the nucleotide sequence as set forth in SEQ ID NO: 1 in the sequence listing and splicing variants of the protein. Hereunder, the agent for improving depression state may be referred to as the anti-depressant drug.

The phrase "An agent for improving depression state" or the phrase "antidepressant drug" refers to a drug having an effect for improving depression state.

### (Method for identifying a compound)

A method for identifying a compound that inhibits the function of the protein used in the present invention can be carried out utilizing a known pharmaceutical screening system using at least one member selected from the proteins, the DNAs, the recombinant vectors, the transformants, or the antibodies. The present identification methods allow screening for antagonists by drug design based on the structure of the present proteins, screening for an inhibitor of the expression at the gene level by utilizing a protein synthesis system, or screening for a substance recognized by an antibody by utilizing the antibody, or the like.

The method for identifying a compound that inhibits the function of the protein used in the present invention can be used as a method for identifying a compound having anti-depressant action. More preferably, the identification method can be used as a method for identifying a compound having an anti-depressant action that is an antagonist of any one of proteins selected from the group consisting of a protein encoded by the DNA represented by the nucleotide sequence as set forth in SEQ ID NO: 1 in the sequence listing and splicing variants of the protein. An anti-depressant action of the compound can be confirmed by carrying out test methods generally used for anti-depressant action, for example, a tail suspension test or a forced swimming test using a mouse. Specifically, if immobility time of a mouse administered with the compound is shortened in the tail suspension test compared with that of a mouse not administered with the compound, it can be determined that the compound has an anti-depressant action. Such a compound is useful as an anti-depressant drug.

Identification of a compound that inhibits the function of the protein used in the present invention can be carried out by using, specifically, for example, an experimental system capable of measuring a function of the protein. The identification method can be carried out by using the experimental system that allows the present protein coexist with a compound to be tested (test compound), under conditions allowing the interaction of the present protein with the test compound, followed by measuring the function of the present protein, subsequently, detecting the change of the function of the present protein, such as reduction, enhancement, disappearance, and appearance, in comparison to the measurement result obtained under absence of a test compound. The effect of the test compound that exerts on the function of the protein can be determined by comparing a function of the protein in the presence of the test compound with a function of the protein in the absence of the test compound. For example, if a function of the protein in the presence of the test compound is decreased compared with a function of the protein in the absence of the test compound, it can be determined that the test compound has an inhibitory action on a function of the protein.

As a function of the protein used in the present invention, binding to a ligand, activation of intracellular signal transduction mechanism and induction of cell response can be exemplified, since the protein functions as a membrane protein receptor. More specifically, binding to CCK-8S (SEQ ID NO: 14) and interaction with MAGUK family proteins or the like can be exemplified.

A method for identifying a compound that inhibits the binding of the protein used in the present invention to a ligand of the protein can be conducted by allowing the reaction between the protein and the ligand of the protein in the presence or absence of the test compound and then measuring the binding between the protein and the ligand. The protein used in the identification method can be a protein that expressed in a cell membrane of a cell containing a DNA encoding the protein. The cell may be a transformant obtained by transfecting with a vector containing a DNA encoding the protein. Measurement of the binding between the protein and a ligand of the protein can be performed utilizing various kinds of binding assays that are used in an ordinary pharmaceutical screening system. For example, measurement can be carried out by allowing a binding reaction between the ligand and the protein, separating a complex formed by binding of the ligand to the protein from the unbound free ligand and protein, and detecting the complex by a known method such as immunoblotting or the like. Further, measurement of binding can be carried out by allowing a binding reaction between the ligand and the protein, and then measuring the ligand bound to the protein using an anti-ligand antibody The anti-ligand antibody bound to the ligand can be detected using a secondary antibody labeled with HRP or biotin or the like. The ligand bound to the protein can also be detected using an anti-ligand antibody that is previously labeled with HRP or biotin or the like. Alternatively, the ligand bound to the protein can be measured by using a ligand that was previously labeled with a desired labeling substance as the ligand for use in the binding reaction with the protein for performing the above described identification method, and detecting the labeling substance. Any substance that is used in an ordinary binding assay can be utilized as the labeling substance, which is exemplified by GST, tag peptides such as His-tag, Myc-tag, HA-tag, FLAG-tag or Xpress-tag or the like, or fluorescence dye or the like. As a simple and convenient method, a radioactive isotope can be utilized.

The compound, which is obtained by a method for identifying a compound that inhibits the binding between the protein used in the present invention and a ligand of the protein, can be a compound that inhibits the function of the membrane protein receptor, since the protein is a membrane protein receptor.

The compound, which inhibits the binding between the protein used in the present invention and a ligand of the protein and inhibits a function of a membrane protein receptor comprising the protein, can be used as an antagonist of the membrane protein receptor. It can be determined whether or not the compound is a compound that inhibits the function of the membrane protein receptor of the present invention, by conducting a measurement of a change in the function of the membrane protein receptor caused by ligand in the presence and absence of the compound. When a change in function of the membrane protein receptor is not caused due to a compound, it can be determined that the compound is either a compound that binds to the membrane protein receptor, but does not induce a cell reaction through the membrane protein receptor, or a compound that acts on a ligand and inhibits the binding between the ligand and the membrane protein receptor. In contrast, when the change in function of the membrane protein receptor due to a compound is equivalent to a change in functions of the membrane protein receptor caused by ligand such as CCK-8S (SEQ ID NO: 14), it can be determined that the compound is an agonist that binds to the membrane protein receptor and induces cell response via the membrane protein receptor.

A method for measuring a function of the protein used in the present invention can be exemplified by a method employing an experimental system using a transformant that was made to express the protein, which comprises allowing the ligand to the protein to act after contacting a test compound to the transformant or in the presence of a test compound, and then measuring a change in a cell response produced in the transformant.

The method for measuring a function of the protein can be utilized to carry out a method for identifying a compound that inhibits the function of the protein such as activation of intracellular signal transduction mechanism or induction of a cell response. A compound that inhibits the function of the protein can be selected by detecting a change in the function, such as decrease, increase, disappearance or appearance, in comparison with measurement results obtained in the absence of a test compound. As a change in cell response generated in the transformant in which the protein is expressed, for example, a change in cell membrane potential or in intracellular calcium concentration or the like may be mentioned. When a test compound caused a change such as a decrease in cell membrane potential of the transformant or a decrease in intracellular calcium concentration, it can be determined that the test compound inhibits the function of the protein. Measurements of a change in cell membrane potential and in intracellular calcium concentration can be carried out using a known method. Further, measurement of a function of the protein can be carried out by measuring the interaction with a MAGUK family protein. Measurement of a change in interaction with a MAGUK family protein or measurement of a change in binding to a G protein can be carried out using a known method.

Actually, a cell transfected with a vector containing the DNA represented by the nucleotide sequence as set forth in SEQ ID NO: 1 in the sequence listing was used in an experimental system for measuring a cell response to carry out identification of a compound that exhibits an inhibitory effect to the cell response (see Example 12). In the experimental system, a cell response was induced by subjecting the cell to CCK-8S and measured by measuring a change in intracellular calcium concentration. As the test compound, SoftFocus GPCR Target-Directed Library (BioFocus) that is a compound library was used. As a result, three kinds of compounds that inhibit a cell response of the cell to CCK-8S were identified (aforementioned structural formulae (I), (II) and (III)). The inventors consider that these compounds act as an antagonist of a protein encoded by DNA represented by the nucleotide sequence as set forth in SEQ ID NO: 1.

From the results thus obtained, the inventors consider that an experimental system using a transformant that was made to express the present protein, for example, a system using the transformant which measures a change in an intracellular calcium concentration allows identification of an antagonist that inhibits a response to a ligand of the protein, such as CCK-8S.

A method for identifying a compound that can affect interaction between the protein used in the present invention and MAGUK family protein can be carried out, for example, by using the isolated present protein and MAGUK family protein and detecting a binding between the protein and MAGUK family protein by a known protein binding assay. More specifically, for example, a MAGUK family protein that is expressed as a GST-tag fusion protein by genetic engineering techniques and then is made to bind to glutathione-sepharose allows for quantitative measurement of the amount of the protein binding thereto using an antibody against the protein, for example an antibody that was labeled with an enzyme such as HRP or ALP, a radioactive isotope, a fluorescent substance, or biotin or the like. Alternatively, use of the protein fused with a tag peptide allows for quantitative measurement using an anti-tag antibody. Naturally, the protein may also be directly labeled with the above described enzyme, radioactive isotope, fluorescent substance, or biotin or the like. Alternatively, a secondary antibody that was labeled with the above described enzyme, radioactive isotope, fluorescent substance, or biotin or the like may be used. As a further alternative, DNA encoding the protein may be co-expressed with DNA encoding a MAGUK family protein using a suitable cell followed by detecting binding between the two substances using a pull-down method, which allows for measuring the interaction of the two substances.

A method of identifying a compound that affects interaction between a protein used in the present invention and a MAGUK family protein can also be carried out, for example, by using a two-hybrid method that comprises introducing into a yeast or a eukaryotic cell or the like a plasmid for expressing a fusion protein between the protein and a DNA binding protein, a plasmid for expressing a fusion protein between a MAGUK family protein and a transcription-activating protein, and a plasmid containing a reporter gene such as lacZ connected to an appropriate promoter gene, and comparing the expression amount of the reporter gene in the presence of a test compound with the expression amount of the reporter gene in the absence of the test compound. When the expression amount of the reporter gene in the presence of a test compound decreases in comparison to the expression amount of the reporter gene in the absence of the test compound, it can be determined that the test compound has an inhibitory action on binding between the protein and the MAGUK family protein. In contrast, when the expression amount of the reporter gene in the presence of a test compound increases in comparison to the expression amount of the reporter gene in the absence of the test compound, it can be determined that the test compound has a stabilizing action on binding between the protein and the MAGUK family protein.

Identification of a compound that affects interaction between a protein used in the present invention and a MAGUK family protein can also be performed using a surface plasmon resonance sensor such as the BIACORE system.

Further, identification of a compound that affects interaction between a protein used in the present invention and a MAGUK family protein can be carried out using a method that employs a scintillation proximity assay (SPA) or fluorescence resonance energy transfer (FRET).

Specific examples of a MAGUK family protein used in the identification method of the present invention include DLG2, DLG3 and DLG4, or AIP1 and MAGI3. As long as there is no affect on the interaction with the protein used in the present invention, the MAGUK family protein may have a partial deletion thereof, or may have a labeling substance such as another protein.

The method for identifying a compound that inhibits expression of the protein used in the present invention can be carried out utilizing a known pharmaceutical screening system using at least one member selected from the DNAs, the recombinant vectors and the transformants.

The method for identifying a compound that inhibits expression of the protein used in the present invention can be used as a method for identifying a compound that has anti-depressant action. That is, since a compound obtained by the identification method is considered to have anti-depressant action, it can be used as anti-depressant drug.

The method for identifying a compound that inhibits the expression of the protein used in the present invention can be carried out in an experimental system that allows for measuring expression of the DNA, by allowing the DNA to coexist with a test compound and measuring the expression thereof, subsequently detecting a change such as decrease or disappearance of the expression in comparison with results of measurements obtained in the absence of the test compound. Measurement of the expression of the protein can be performed by directly detecting a protein encoded for by the DNA, or can be carried out, for example, by introducing such a signal as an indicator of the expression into an experimental system and detecting the signal. As a signal, GST, a tag peptide such as, His-tag, Myc-tag, HA-tag, FLAG-tag or Xpress-tag, or a fluorescent dye, or the like can be used.

Specifically, the method for identifying a compound that inhibits the expression of the protein used in the present invention can be carried out, in an experimental system that uses a transformant transfected with an expression vector containing the DNA for expressing the protein and comprises contacting the transformant with a test compound followed by measuring an expressed protein. A compound that inhibits the expression of the protein can be selected by detecting a change such as decrease, or disappearance in expression in comparison with measurement results obtained in the absence of a test compound. Detection of the presence or absence of, or a change in the expression of the protein can be carried out by a known protein detection method, for example, Western blotting or the like. Further, detection of the presence or absence of, or a change in expression of the protein can be carried out by employing an indicator such as a biological function of the expressed protein or a cell response via the protein, for example, interaction with MAGUK family protein, a change in cell membrane potential and a change in intracellular calcium concentration that are generated when subjecting a ligand thereon.

Identification of a compound that inhibits expression of the protein used in the present invention can also be carried out by, for example, producing a vector in which a reporter gene is connected instead of the DNA downstream of a promoter region of a gene including the DNA, contacting a cell, for example, a eukaryotic cell, containing the vector with a test compound, and measuring the presence or absence of, or a change in, expression of the reporter gene. A gene that is ordinarily used in a reporter assay can be used as the reporter gene. For example, a gene having an enzyme activity such as luciferase, β-galactosidase or chloramphenicol acetyl transferase may be mentioned. Detection of the reporter gene expression can be carried out by detecting the activity of the gene product, for example, the enzyme activity in the case of the reporter genes exemplified above.

The method for identifying a compound that promotes a function of or expression of the protein used in the present invention can be carried out utilizing an experimental system or a measuring system that is used in the aforementioned identification method. For example, the method for identifying a compound that promotes a function of the protein can be carried out utilizing an experimental system for measuring the binding between the protein and a ligand of the protein, a method for measuring a function of the protein, or an experimental system for measuring the binding between the protein and MAGUK family protein. Further, the method for identifying a compound that promotes expression of the protein can be carried out utilizing an experimental system capable of measuring expression of the DNA used in the present invention. When a function of or expression of the protein is increased or generated by a test compound in such an experimental system or a measuring system, it can be determined that the test compound promotes a function of or expression of the protein.

The method for identifying an agonist of the membrane protein receptor of the present invention can be carried out utilizing an experimental system or a measuring system used in the aforementioned identification method. As the identification method of an agonist of the membrane protein receptor of the present invention, for example, such a method may be mentioned which uses an experimental system using the transformant in which the protein is expressed and comprises contacting the transformant with a test compound or allowing the transformant to coexist with a test compound followed by measuring a change in function produced in a transformant. An agonist of the membrane protein receptor can be selected by detecting the change in the function of the membrane protein receptor, for example, decrease, increase, disappearance, appearance or the like, in comparison with measurement results obtained in the absence of a test compound. An agonist can be selected more preferably by comparing the change in functional with a change in function observed by measuring a change in function of the membrane protein receptor caused by a ligand of the membrane protein receptor such as CCK-8S (SEQ ID NO: 14). Preferably, the agonist is a compound that brings a change in function of the membrane protein receptor that is equivalent to a change in function of the membrane protein receptor produced by a ligand such as CCK-8S (SEQ ID NO: 14). It is sufficient that the change in function of the membrane protein receptor produced by an agonist is equivalent to a change in function of the membrane protein receptor produced by a ligand such as CCK-8S (SEQ ID NO: 14), while there may be a quantitative difference. For example, the change function of the membrane protein receptor produced by an agonist may be weaker than a change in function of the membrane protein receptor produced by a ligand such as CCK-8S (SEQ ID NO: 14). It is preferable to select an agonist that induces an equal change in function. The change in function of the membrane protein receptor can be measured by employing a change in cell response via the membrane protein receptor of the transformant as an indicator. Accordingly, as a change in function that is equivalent to a change in function of the membrane protein receptor produced by a ligand such as CCK-8S (SEQ ID NO: 14), for example, an increase in intracellular calcium concentration via the membrane protein receptor in a transformant can be mentioned. Measurement of a change in intracellular calcium concentration can be carried out using a known method (see Example 5). In addition, as a change in function that is equivalent to a change in function of the membrane protein receptor produced by a ligand such as CCK-8S (SEQ ID NO: 14), a change in membrane potential via the membrane protein receptor in a transformant can be mentioned. Measurement of a change in membrane potential can be carried out using a known method (see Example 3). With respect to the ligand, either a sample including the ligand or the ligand itself that was obtained by the above described method of identifying a ligand can be used. Since CCK-8S (SEQ ID NO: 14) is considered to be an *in vivo* ligand of the protein, the use of CCK-8S (SEQ ID NO: 14) as a ligand is preferred. CCK-8S (SEQ ID NO: 14) can be produced by a common chemical synthesis method. Further, it can also be synthesized using a commercially available peptide synthesis apparatus.

The method for identifying an agonist of the membrane protein receptor of the present invention can also be carried out by using the above described identification method to determine whether or not a compound obtained by the method for identifying a compound that binds to the membrane protein receptor induces a change in function of the membrane protein receptor.

Identification of a ligand of a membrane protein receptor comprising the protein used in the present invention can be carried out by utilizing an experimental system and measuring system that is used in the aforementioned identification method. For example, it can be carried out by detecting the binding between a substance to be examined (hereunder, referred to as "test substance") and the protein by a known binding assay. Alternatively, it can be carried out in the identification method using cells that express the protein, by measuring a cell response of the cell induced when contacting the test substance with the protein. In the case that the cell response when contacting a test substance with the protein is changed, for example, promoted, occurred, decreased, or disappeared, in comparison to that when not contacting the test substance, it can be determined that the test substance is a ligand or includes a ligand. Specific examples of the cell response include a change in cell membrane potential or a change in intracellular calcium concentration. Measurement of cell membrane potential or intracellular calcium concentration can be carried out by a known method. Alternatively, the target ligand can be obtained in an identification method using cells that express the protein, by measuring the interaction between the protein and a MAGUK family protein as an indicator for the cell response. In the case that the interaction between the protein and a MAGUK family protein in the cell when contacting a test substance with the protein is changed such as promoted or occurred in comparison to that when not contacting the test substance, it can be determined that the test substance is a ligand or includes a ligand. Interaction between the protein and a MAGUK family protein can be detected by a known method such as immunoblotting or the like.

As a test substance which may be an object for identifying a ligand, for example, a sample prepared from a cell or a biological tissue in which expression of the DNA used in the present invention was observed may be used. Alternatively, various compounds that were derived from natural products or synthesized can be used as an object.

This kind of identification method is useful for determining whether or not a ligand is included in a sample. The identification method can also be effectively used in a process for purifying the ligand from a sample which was determined to contain the ligand. For example, when fractionating and purifying a sample using gel filtration chromatography or the like, it can be used to determine whether or not a ligand is included in the fractions.

### (Compounds)

Compounds identified by the identification method of the present invention can be utilized as inhibitors, antagonists, promoters or stabilizers or the like of a function of the protein used in the present invention, for example, binding to a ligand, activation of intracellular signal transduction, or induction of a cell response. Furthermore, the compounds can also be utilized as expression inhibitors or expression promoters of the protein used in the present invention at the gene level. These compounds can be prepared as medicaments by further selection in consideration of the balance between bioavailability and toxicity Further, these compounds are expected to exert a preventive effect and/or therapeutic effect for various kinds of pathologic symptoms attributable to an abnormality in a function of the protein and/or expression of DNA encoding the protein.

### (Pharmaceutical Composition)

The protein, DNA, recombinant vector, transformant, antibody, ligand and compound which are used in the present invention are useful as an active ingredient of a medicament or a pharmaceutical composition that is based on inhibiting, antagonizing, or promoting a function and/or expression of the protein.

A medicament or a pharmaceutical composition of the present invention can be used as an agent for preventing and/or treating diseases attributable to an abnormality in a function of the protein used in the present invention and/or the expression of DNA encoding the protein. The medicament or the pharmaceutical composition can also be used in a method for preventing and/or treating such diseases.

In the case of excess function of the protein used in the present invention and/or excess expression ofDNA encoding the protein, one method is to administer an effective dose of the inhibitor that inhibits the function of the protein and/or the expression of the DNA to a subject together with a pharmaceutically acceptable carrier to inhibit the function of the protein and thereby improve the abnormal symptoms. Further, the spontaneous expression of DNA encoding the protein may be inhibited using an expression block method. For example, expression of DNA encoding the protein can be inhibited by using a fragment of the DNA as an antisense oligonucleotide in gene therapy. A DNA fragment corresponding to a non-coding region of a present DNA as well as a DNA fragment corresponding to a coding region thereof is useful as an anti-sense oligonucleotide used herein. In order to specifically inhibit the expression of a present DNA, it is preferable to use a nucleotide sequence of a characteristic region of the DNA.

Examples of a disease attributable to abnormality in a function of the protein used in the present invention and/or expression of DNA encoding the protein include neurological diseases that are preferably exemplified by depression. Expression of the present protein is strongly observed in the brain tissues, particularly in brain cortex, hippocampus, and amygdaloid body, which corresponds to the distribution of CCK that may be a ligand of a functional membrane protein receptor comprising the protein. It is known that CCK is involved in physiological functions such as anxiety, analgesia, sedation, food intake control, memory and learning. Besides, in the tail suspension test using knockout mouse of BAI2 gene, the mouse exhibited a contra-depression-like phenotype. BAI2 gene is a splicing variant of DNA encoding the protein. Accordingly, the inventors consider that a splicing variant of DNA encoding the protein is involved in depression. The inventors consider that, for example, depression is induced by such an abnormality that expression of DNA encoding the protein or of a splicing variant of the DNA is increased.

The inventors consider that an inhibitor of a function and/or expression of the protein used in the present invention, for example, an antagonist of a membrane protein receptor comprising the protein, and a pharmaceutical composition containing an effective dose of the inhibitor are effective for alleviation, improvement, prevention and/or treatment of depression. The medicament or the pharmaceutical composition of the present invention can be used for preventing and/or treating depression. Specifically, the medicament or the pharmaceutical composition can be an agent for preventing and/or treating depression containing an effective dose of the inhibitor of the function and/or the expression of the present protein, for example, an antagonist of a membrane protein receptor comprising the present protein, as an active ingredient. In other words, the medicament or the pharmaceutical composition can be an agent for preventive and/or treating depression containing an effective dose of the aforementioned anti-depressant drug as an active ingredient. Administration of the aforementioned anti-depressant drug allows a method of preventing and/or treating depression to be carried out.

Examples of a disease attributable to abnormality of the function of the protein used in the present invention and/or the expression of DNA encoding the protein further include a disease attributable to angiogenesis inhibition and a disease accompanying angiogenesis inhibition, since the present protein has TSP-I domain in the amino acid sequence. It is reported that TSP-I domain is a domain responsible for a function of inhibiting angiogenesis. Therefore, the present protein is considered to have a function of inhibiting angiogenesis. Accordingly, the present protein is probably involved in a disease attributable to angiogenesis inhibition or a disease accompanying angiogenesis inhibition. In such a disease, the treatment thereof can be performed by promoting angiogenesis. Therefore, it is preferable to inhibit the function or expression of the present protein. Examples of such a disease include cerebral contusion and cerebral infarction.

For treatment of abnormal symptoms relating to a decrease or depletion of a function of a protein used in the present invention and/or expression of DNA encoding the protein, a method can be employed for one means, which comprises administering an effective dose of a promoter that promotes or stabilizes the function of the protein and/or expression of the DNA together with a pharmaceutically acceptable carrier, thereby to improve the abnormal symptoms. Alternatively, the protein can be produced in cells within the subject using gene therapy. A known method can be utilized for the gene therapy that utilizes the present DNA. For example, a method can be employed which comprises preparing a replication-defective retrovirus vector incorporating the present DNA or the RNA that is a transcription product of the DNA, and then treating cells originating from a subject ex vivo using the vector, subsequently introducing the cell into the subject.

A medicament of the present invention may be prepared as a medicament containing an effective dose of at least one member selected from the aforementioned protein, the aforementioned DNA, the aforementioned recombinant vector, the aforementioned transformant, the aforementioned antibody, the aforementioned ligand, or the aforementioned compound, as an effective ingredient. In general, it is preferable to prepare a pharmaceutical composition using one or more kinds of pharmaceutical carriers.

An amount of the effective ingredient contained in a pharmaceutical preparation of the present invention can be suitably selected from a wide range. In general, a suitable amount may fall within a range of approximately 0.00001 to 70 wt%, preferably approximately 0.0001 to 5 wt%.

A pharmaceutical carrier may be a diluent or excipient, which can be generally used in accordance with the form of use of the pharmaceutical preparation, such as, a filler, an extender, a binder, a wetting agent, a disintegrator, and/or a lubricant. These can be suitably selected and used in accordance with the form of use of the pharmaceutical preparation obtained.

The pharmaceutical carrier may be, for example, water, a pharmaceutically acceptable organic solvent, collagen, polyvinyl alcohol, polyvinylpyrrolidone, carboxyvinyl polymer, sodium alginate, soluble dextran, sodium carboxymethyl starch, pectin, xanthan gum, acacia gum, casein, gelatin, agar, glycerin, propylene glycol, polyethylene glycol, vaseline, paraffin, stearyl alcohol, stearic acid, human serum albumin, mannitol, sorbitol and lactose. One or a combination of two or more kinds of these carriers may be used in accordance with administration form.

As desired, various ingredients used in conventional protein preparations can be suitably used herein, such as, a stabilizer, a bacteriocide, a buffer agent, an isotonizing agent, a chelating agent, a pH adjuster, or a surfactant, for preparing the pharmaceutical preparation.

As a stabilizer, the following may be used: human serum albumin, common L-amino acids, sugars, and cellulose derivatives. These can be used independently or in combination with a surfactant, and the like. Use of these in such a combination may give increased stability to an effective ingredient. An L-amino acid is not particularly limited, and may be any one of glycine, cysteine, glutamic acid, and the like. A sugar is not particularly limited, and may be any one of the monosaccharides (such as glucose, mannose, galactose, and fructose), sugar alcohols (such as mannitol, inositol, and xylitol), disaccharides (such as sucrose, maltose, and lactose), polysaccharides (dextran, hydroxypropylstarch, chondroitin sulfate, and hyaluronic acid), derivatives thereof, and so on. A cellulose derivative is not particularly limited, and may be any one of methylcellulose, ethylcellulose, hydroxyethylcellulose, hydroxypropylcellulose, hydroxypropylmethylcellulose, sodium carboxymethylcellulose, and the like. A surfactant is also not particularly limited, and can be both an ionic surfactant and/or a non-ionic surfactant. Examples of a surfactant include polyoxyethyleneglycol sorbitan alkyl ester base, polyoxyethylene alkyl ether base, sorbitan monoacyl ester base, and a fatty acid glyceride base.

Examples of a buffer agent include boric acid, phosphoric acid, acetic acid, citric acid, ε-aminocaproic acid, glutamic acid and/or salts corresponding to these (for example, an alkali metal salt or an alkali earth metal salt of these, such as a sodium salt, potassium salt, calcium salt or magnesium salt).

Examples of an isotonizing agent include sodium chloride, potassium chloride, a saccharide, and glycerin.

Examples of a chelating agent include edetate sodium and citric acid.

The medicaments and the pharmaceutical compositions of the present invention can be used as solution preparations. Alternatively, they can be freeze-dried, so as to be preservable, and used by dissolving them in water, a buffered solution including saline, and the like, to adjust them to a suitable concentration, at the time of use.

Dosage range of the pharmaceutical composition is not particularly limited, and can be suitably selected in accordance with the following: effectiveness of the ingredients contained therein; the administration form; the route of administration; the type of disease; the characteristics of the subject (e.g., body weight, age, symptomatic conditions, and whether a subject is taking other pharmaceutical agents); and the judgment of a physician in charge. In general, a suitable dosage may fall, for example, within a range of about 0.01 µg to 100 mg, per 1 kg of the body weight of the subject, and preferably within a range of about 0.1 µg to 1 mg, per 1 kg of body weight. However, these dosages can be altered using conventional experiments for optimization of a dosage that are well known in the art. The aforementioned dosage can be divided for administration once to several times a day. Alternatively, periodic administration once every few days or few weeks can be employed.

When administering the pharmaceutical composition of the present invention, the pharmaceutical composition may be used alone, or may be used together with other compounds or medicaments required for treatment.

In terms of a route of administration, either systemic administration or local administration may be selected. In this case, a suitable administration route is selected in accordance with a disease, symptoms and the like. For example, parenteral administration including normal intravenous injection, intra-arterial administration, subcutaneous administration, intracutaneous administration, and intramuscular administration can be employed. Oral administration can be also employed. Further, transmucosal administration or dermal administration can be employed. In the case of use for cancer disease, it may be preferable to employ a direct administration into the tumor by injection, and the like.

In terms of an administration form, various forms can be selected in accordance with a purpose. Typical examples thereof include a solid administration form such as a tablet, pill, powder, powdered drug, subtle granule, granule or capsule, and a solution administration form such as an aqueous solution formulation, an ethanol solution formulation, a suspension, a lipid emulsion preparation, a liposome preparation, a clathrate such as cyclodextrin, a syrup and an elixir. These can be further classified, according to the administration route, into an oral formulation, parenteral formulation (drip injection formulation or injection formulation), nasal formulation, inhalant formulation, transvaginal formulation, suppositorial formulation, sublingual agents, eye drop formulation, ear drop formulation, ointment formulation, cream formulation, transdermal absorption formulation, transmucosal absorption formulation, and the like, which can be respectively blended, formed and prepared according to conventional methods.

When using the pharmaceutical composition of the present invention as a gene therapy agent, it is preferable in general to prepare the pharmaceutical composition as an injection, a drop or a liposome preparation. When preparing a gene therapy agent in a form containing a cell into which a gene was introduced, the gene therapy agent can also be prepared, for example, in a form in which the cell is suspended in phosphate buffered saline (pH 7.4), Ringer's solution, or an injectable solution of an intracellular composition solution. Alternatively, a gene therapy agent can also be prepared in a form that allows administration thereof together with a substance that enhances the efficiency of gene transfer, such as protamine. In the case of use as a gene therapy agent, the present pharmaceutical composition can be administered once a day, or can be divided for administration several times a day. Further, periodic administration once every few days or few weeks may also be employed. The administration method can be in accordance with a method used in a common gene therapy method.

### (Diagnostic Method)

The protein, the DNA, the recombinant vector, the transformant, the antibody or the compound, which are used in the present invention, can be used by itself as a means for diagnosing a disease, such as a diagnostic marker or a diagnostic reagent.

According to the present invention, for example, use of the nucleotide sequence of all or a part of the present DNA allows the specific detection of the presence or absence of an abnormality in a gene containing the DNA used in the present invention, or the presence or absence of expression thereof, in an individual, or in various kinds of tissues. The detection of the present DNA allows for a diagnosis of susceptibility to, onset of, and/or prognosis of, a disease attributable to the gene. The phrase "a disease attributable to the gene" refers to a disease resulted from an abnormality of the gene in amount and/or an abnormality of the gene in function. Examples of a disease attributable to the present gene include a neurological disease such as a depression or the like.

Diagnosis of a disease by gene detection can be carried out, for example, by detecting the presence of a nucleic acid corresponding to the gene, by determining the existing amount thereof, and/or by identifying a mutation therein, with respect to a test sample. In comparison to a normal control sample, a change in the existence of a nucleic acid corresponding to the target gene, and a quantitative change thereof, can be detected. Further, the deletion and insertion can be detected in comparison to a normal genotype by subjecting amplified products obtained by amplifying a nucleic acid corresponding to the target gene using a known method, for example, to the measurement of a change in size. Furthermore, a point mutation can be identified by hybridizing amplified DNA with, for example, the DNA used in the present invention that was labeled. Detection of an alteration or a mutation in such manner allows the above described diagnosis to be carried out.

A qualitative or quantitative measurement method for a target gene in a test sample, or a qualitative or quantitative measurement method for a mutation in the specific region of the gene can be carried out of the present invention.

A test sample is not particularly limited as long as it contains a nucleic acid of the target gene and/or a mutant gene thereof For example, the test sample may be a biological sample derived from a living organism, such as a cell, blood, urine, saliva, spinal fluid, biopsy tissue or autopsy material and the like. Alternatively, as desired, a nucleic acid may be extracted from a biological sample to prepare a nucleic acid sample for use. A nucleic acid may be a genomic DNA which is directly used to the detection. Alternatively, a nucleic acid may be enzymatically amplified by employing PCR, or other amplification methods, prior to analysis. RNA or cDNA may be similarly used. A nucleic acid sample may also be prepared according to various methods, for facilitating detection of a target sequence, for example, denaturation, digestion with restriction enzymes, electrophoresis, or dot blotting.

Any known gene detection method can be used for the detection method, and examples thereof include plaque hybridization, colony hybridization, Southern blotting, Northern blotting, the Nucleic Acid Sequence-Based Amplification (NASBA) method and RT-PCR. Measurement at the cell level utilizing in situ RT-PCR or in situ hybridization or the like can also be used. Methods that can be used for detection of the target gene are not limited to the methods described above, and any known gene detection method can be used.

In such a gene detection method, a fragment of the DNA used in the present invention having a property as a probe or having a property as a primer is useful for carrying out isolation and/or amplification of the target gene or the mutant gene thereof The phrase "DNA fragment having a property as a probe" refers to a DNA fragment that consists of a characteristic sequence of the DNA and is capable of specifically hybridizing only to the target DNA. The phrase "DNA fragment having a property as a primer" refers to a DNA fragment that consists of a characteristic sequence of the DNA and is capable of specifically amplifying only the present DNA. Further, when detecting a mutant gene capable of being amplified, a primer or a probe having a sequence with a predetermined length, which contains a mutation site within the gene, is prepared and used. A probe and a primer may have a nucleotide sequence consisting of, preferably, from about 5 to 50 nucleotides, more preferably, from about 10 to 35 nucleotides, and even more preferably, from about 15 to 30 nucleotides. A labeled probe is normally used as the probe, but the unlabeled probe can also be used. Alternatively, the detection can also be carried out by measuring the specific binding to a ligand that was labeled directly or indirectly. Various methods are known for labeling a probe and a ligand. For example, nick translation, random priming, or a method utilizing kinase treatment, may be used. Labeling substances suitable for use include a radioactive isotope, biotin, a fluorescent substance, a chemiluminescent substance, an enzyme, an antibody, and the like.

PCR is preferable as a gene detection method, from the viewpoint of sensitivity. Any well-known method of PCR can be employed, as long as it is a method that uses a primer capable of specifically amplifying the target gene. For example, RT-PCR may be employed. In addition, various modified PCR methods used in the art can be applied.

In addition to detection of a gene, PCR allows quantitative measurement of the target gene and/or a mutant gene thereof Such an assay method may be exemplified by a competitive assay such as a Multi-channel Simplex Stimulated Annealing (MSSA) method, or PCR-SSCP which is known as a mutation detection method that utilizes a change in mobility accompanying a structural change of a single-stranded DNA.

According to the present invention, for example, use of a protein used in the present invention allows the specific detection of the presence or absence of an abnormality in the protein itself and in its function, in an individual or in various kinds of tissues. The detection of an abnormality in the present protein and in its function allows a diagnosis of susceptibility to, onset of, and/or prognosis of, a disease attributable to the gene.

The diagnosis of a disease by detecting a protein can be carried out, for example, by detecting the presence of a protein, by determining the existing amount thereof, and/or by detecting a mutation therein, with respect to a test sample. That is to say, the present protein and/or a mutant thereof are quantitatively or qualitatively determined. In comparison to a normal control sample, a change in the existence of the target protein, and a quantitative change thereof, can be detected. Alternatively, in comparison to a normal control sample, a mutation can be detected, for example, by determining an amino acid sequence. The detection of such a change or a mutation allows the aforementioned diagnosis. The test sample is not particularly limited as long as it contains the target protein and/or a mutant thereof For example, a biological sample derived from a living organism, such as blood, serum, urine, biopsy tissue, and the like, may be used.

Determination of the protein used in the present invention and of the protein having a mutation can be carried out by using the following: the present protein, for example, a protein represented by the amino acid sequence as set forth in SEQ ID NO: 2 in the sequence listing, or an amino acid sequence having a deletion, substitution, insertion or addition of one or more amino acids in the amino acid sequence of the protein, or a fragment of these, or antibodies against the protein or a fragment thereof.

Any protein detection methods, or protein quantitation methods which are well known in the art, can be used for quantitative or qualitative measurement of the protein. For example, the amino acid sequence analysis of a target protein allows a detection of a mutant protein. More preferably, an antibody (a polyclonal antibody or a monoclonal antibody) may be used for detecting the difference in a target protein sequence, or the presence or absence of a target protein.

According to the present invention, a qualitative or quantitative measurement method for the protein used in the present invention in a test sample, or a qualitative or quantitative measurement method for a mutation in a specific region of the protein can be conducted.

Specifically, the aforementioned detection may be carried out by subjecting a test sample to immunoprecipitation, using specific antibodies raised against a target protein, and then analyzing the target protein by Western blotting or immunoblotting. Further, the detection of a target protein in a paraffin tissue section, or a frozen tissue section, may be carried out by means of immuno-histochemical techniques using antibodies raised against the target protein.

The preferable methods of detecting a target protein or a mutant thereof, may be, for example, enzyme-linked immunosorvent assay (ELISA), radio immuno assay (RIA), immunoradiometric assay (IRMA), and immunoenzymometric assay (IEMA), including a sandwich method using a monoclonal antibody and/or a polyclonal antibody. Alternatively, radio immuno assay, competitive binding assay, and the like may be employed.

### (Reagents and Reagent Kit)

The protein, the DNA, the recombinant vector, the transformant and the antibody, which are used in the present invention, can each be used by itself as a reagent or the like. For example, each of these can be used as a reagent for use in the method of identifying a compound of the present invention or the method for determining a protein and/or DNA of the invention. The reagent is useful, for example, in elucidating an intracellular signal transduction pathway in which the present protein or the DNA participates, as well as for fundamental research and the like relating to diseases and the like attributable to an abnormality in the protein and/or the DNA.

Specifically, the reagent kit of the present invention may be a reagent kit that contains at least one member selected from the following: a DNA represented by any one of nucleotide sequences as set forth in SEQ ID NO: 1, 15 and 17 in the sequence listing, a recombinant vector containing the DNA, a transformant in which the recombinant vector is introduced, a protein encoded by the DNA, and an antibody that recognizes the protein. More specifically, a reagent kit may be exemplified which comprises at least one member selected from the following: a DNA represented by any one of nucleotide sequences as set forth in SEQ ID NO: 1, 15 and 17 in the sequence listing, a recombinant vector containing the DNA, a transformant in which the recombinant vector is introduced, a protein represented by any one of amino acid sequences as set forth in SEQ ID NO: 2, 16 and 18, and an antibody that recognizes the protein.

When using these as a reagent, these may contain a substance such as a buffer solution, a salt, a stabilizer, and/or an antiseptic agent. In this connection, known formulation means may be introduced in accordance with the respective properties at the time of formulation.

The present invention provides a reagent kit including at least one member selected from the following: the protein, the DNA, the recombinant vector, the transformant and the antibody which are used in the present invention. When these are composed in a reagent kit, the kit may contain a substance necessary for carrying out a measurement, such as a labeling substance for detecting the present protein or the DNA, an agent for detecting the labeling substance, a reaction diluent, a standard antibody, a buffer solution, a washing agent, a reaction terminating solution and the like. As a labeling substance, the aforementioned proteins for labeling, the aforementioned substances for chemical modification, and the like, can be used. The labeling substance may be previously linked to the present protein or the DNA.

The reagent kit according to the present invention can be used the above described identification methods and measurement methods. The present invention can also be used as a testing agent as well as a testing kit in a testing method that uses the above described measurement methods. It can also be used as a diagnostic agent as well as a kit for diagnosis in a diagnostic method that uses the above described measurement methods.

Hereinafter, the present invention may be explained more specifically with the Examples, but it is not limited to the following Examples.

### EXAMPLE 1

### (Construction of human brain-derived cDNA library and isolation of gene)

A cDNA library was constructed according to an ordinary method employing commercially available polyA⁺ RNA derived from the human brain, fetal brain and brain hippocampus (Clontech Inc.: catalog Nos. 6516-1, 6525-1, and 6578-1) as starting material. The nucleotide sequences of cDNA clones were determined after isolating cDNA fragments by dbEST analysis. Specifically, a human brain-derived cDNA library was constructed in accordance with the method of Ohara et al. (Ohara, O. et al., "DNA Research", 1997, Vol. 4, p. 53-59)., from which approximately 50,000 recombinants were randomly selected. Then, with respect to approximately 30,000 cDNA clones among them, their 5 '-terminal and 3 '-terminual nucleotide sequences were determined. Further, approximately 1,100 cDNA clones were selected mainly by in-vitro transcription translation experiments, and their nucleotide sequences were determined according to the method of Ohara et al.

cDNA clones, whose entire nucleotide sequences were determined, were subjected to a conventional analysis method using a computer program to predict an ORF, resulting in acquisition of a cDNA clone having a seven-span transmembrane domain in this region.

The identified cDNA clone ph01207 is a DNA (SEQ ID NO: 1) having a nucleotide sequence with a total length of 4557 bp, containing an ORF that encodes 1518 amino acid residues containing a segment presumed to be a signal sequence consisting of 20 amino acid residues from the N terminus. A homology search indicated that ph01207 is a splice variant, having a deletion of a region that encodes 55 amino acid residues at the N-terminal region in the sequence of hBAI2 (GenBank accession number AB005298), and also having an insertion of one amino acid residue in a region on the C-terminal side that is lysine at position 1406 in the amino acid sequence represented by SEQ ID NO: 2 (Figure 1-A).

The protein encoded by ph01207 was found to have such a structural characteristics as a GPS domain and a seven-span transmembrane domain in addition to three TSP-I domains.

Meanwhile, the protein encoded by hBAI2 is deemed to have a GPS domain and a GPCR family-2 domain in addition to four TSP-I domains. It was thus clarified that the protein encoded by ph01207 lacks 55 amino acid residues corresponding to a region containing one TSP-I domain on the N-terminal region side ofhBAI2.

A tissue expression analysis for ph01207 gene revealed specific high expression of the gene in all normal brain tissues, for example, brain cortex (temporal pole, motor cortex), hippocampus, amygdaloid body and the like. Further, enhanced expression of the gene was observed in ovarian cancer, liver cancer, and adrenal cancer in comparison to the respective normal tissue.

### EXAMPLE 2

### (Production of ph01207 expressing cell line and expression of DNA in the cell line)

The clone ph01207 that was identified in Example 1 was used to express the protein encoded by ph01207 as an N-terminal epitope-tag fusion protein.

First, an expression vector containing the ph01207 gene was constructed. The clone ph01207 that was identified in Example 1 was used to clone the DNA that encodes the amino acid sequence not containing a segment presumed to be a signal sequence consisting of 20 amino acid residues from the N terminus, into pDONR201 by PCR and restriction enzyme treatment to construct a ph01207 entry vector. PCR was carried out using oligonucleotides, as primers, respectively represented by the nucleotide sequences as set forth in SEQ ID NOS: 4 to 11 in the sequence listing, and using Pfu turbo DNA polymerase (Stratagene) as polymerase.

Two kinds of vector, p3×FLAG-CMV9-attR and T8HA-attR/pCINeo, were prepared as N-terminal epitope-tag fusion type expression vectors. p3×FLAG-CMV9-attR is a vector obtained by making p3×FLAG-CMV9 (Sigma Inc.) compatible to the Gateway system using the Gateway Vector Conversion System (Invitrogen Corp.). T8HA-attR/pCINeo was constructed using pCINeo (Promega Corp.), synthetic oligos (T8SP-HA(SEQ ID NO: 12) and T8SP-HA as (SEQ ID NO: 13)) and the Gateway Vector Conversion System in accordance with information in the literature (Koller, K. J., et al., "Analytical Biochemistry", 1997, Vol. 250, p. 51-60). Both vectors have a secretory signal sequence (FLAG: PPTLS, HA: T8) before (N-terminal side) epitope-tag. An N-terminal FLAG-tag or HA-tag fusion type expression vector was constructed using these vectors by LR reaction with Mammalian Expression system with Gateway technology (Invitrogen Corp.). The constructed expression vectors were subjected to restriction enzyme treatment and sequence analysis, and were found to have neither nucleotide substitution nor nucleotide deletion in a coding region of the introduced sequence.

Each of the thus constructed two kinds of expression vector was transfected into the CHO-K1 cell line using FuGENE 6 (Roche), followed by cultivation with a culture medium including G418, that is DMEM/F12 medium containing 400 µg/mL G418 and 10% fetal calf serum, to select an expression cell line. The viable expression cell lines were subjected to further selection by cell enzyme immunoassay (cell EIA) using peroxidase (POD)-labeled anti-epitope-tag antibodies, anti-FLAG M2-POD antibody and anti-HA-POD antibody: 3F10. The selected cell lines were subjected to further selection by fluorocytometry (FCM) analysis using primary antibody (anti-FLAG M2 antibody and anti-HA antibody: clone HA-7) and fluorescein isothiocyanate (FITC)-labeled secondary antibody (FITC-anti-mouse IgG antibody), to obtain expression cell lines.

As the result of the FCM analysis, 8 clones that express a protein (FLAG-tag fusion protein) recognized by anti-FLAG antibody on the cell membrane and 4 clones that express a protein (HA-tag fusion protein) recognized by anti-HA antibody were obtained. Each of the clones showed a clearly stronger fluorescent signal produced by binding of an antibody recognizing FLAG-tag or HA-tag, in comparison to the host cell line (CHO-K1), which revealed that the gene of interest was expressed therein. Representative results are shown in Figure 2.

The result of the FCM analysis revealed that the N-terminal epitope-tag fusion type ph01207 gene product was expresses on the cell membrane.

Among the clones which were observed to express HA-tag fusion protein, a cell line designated as HA-ph01207#10-6 was deposited with the International Patent Organism Depositary of the National Institute of Advanced Industrial Science and Technology (Japan) on August 19, 2004 under Accession NO: FERM BP-10101. The existence of this cell line was confirmed by experiment at the International Patent Organism Depositary on September 22, 2004. The HA-ph01207#10-6 cell line is a cell line that was established by transfecting CHO-K1 cell line with a vector for expressing the DNA, which was represented by a nucleotide sequence of the open reading frame (ORF) of the DNA represented by the nucleotide sequence as set forth in SEQ ID NO: 1 but not contained a segment presumed to be a signal sequence consisting of 20 amino acid residues from the N terminus of the amino acid sequence represented by SEQ ID NO: 2, as an N-terminal HA-tag fusion protein. It stably expresses the N-terminal HA-tag fusion protein.

### EXAMPLE 3

### (Functional analysis of ph01207 gene product)

Functional analysis of the ph01207 gene product was carried out by adding a ligand to Xenopus laevis oocyte in which ph01207 was expressed, and then measuring a cell response. The cell response was measured using six each for control oocyte having no introduced gene and oocyte in which the gene, ph01207 cDNA clone, was expressed, by adding a ligand to the oocyte and then measuring variations in the membrane potential of the oocyte. Culture supernatant of the HeLa cell line in which expression of ph01207 was observed was used as the ligand. The culture supernatant was prepared by culturing 1.2 × 10⁶ cells of HeLa cells in DMEM containing 10% fetal calf serum for two days, followed by collecting the culture supernatant, and then subjected to filtration with a filter (0.45 µm). Two kinds of culture supernatant with different lots prepared in the same manner were used as the ligand (hereunder, referred to as ligand sample 1 and 2).

The Ligand sample 1 or 2 was added to the oocyte in which the ph01207 gene was expressed and the control oocyte to measure membrane potential variations. Evaluation was carried out using variations in current amount and waveforms showing variations in current amount. When a variation in the current amount was 0.2 µA or more and a waveform of a GPCR-specific pattern was observed, it was determined that a response to ligand stimulation was generated. The GPCR-specific pattern means a pattern of waveform 1 shown in Figure 3. Patterns of waveforms 2-4 shown in Figure 3 are waveform patterns generated by artificial factors, for example, influence of some kind of component such as a solvent, a high concentration ligand, or the like, whereby it was determined that a response generated was not against ligand stimulation. Further, when the patterns like waveforms 5 and 6 were observed, it was determined that a response to ligand stimulation was not generated.

Consequently, variations in the current amount were observed only in the ph01207 expressing cells (Table 1 and Table 2). The characters "ND" in Table 2 indicate that the current variation amount was less than 0.2 µA which means that a response was not observed. As shown in Table 1, a response to ligand stimulation was observed in all six samples of the ph01207 expressing cell lines. In contrast, there was completely no response to the ligand in the control cells (Table 2). The inventors therefore considered that the response of the ph01207 expressing cells produced by ligand stimulation is a specific response to the expressed receptor. A similar result was obtained when using either of the ligand samples 1 and 2.

**Table 1**

| | ph01207 Expressing Oocyte | | | | | | | | |
|---|---|---|---|---|---|---|---|---|---|
| Ligand sample | Rate of response | Representative Waveform | Mean amount of current variation S.D. | Amount of current variation (µA) | | | | | |
| | | | | 1 | 2 | 3 | 4 | 5 | 6 |
| 1 | 6/6 | GPCR | 1.21±0.54 | 1.33 | 1.81 | 1.30 | 0.50 | 0.63 | 1.70 |
| 2 | 6/6 | GPCR | 0.48±0.24 | 0.66 | 0.24 | 0.29 | 0.58 | 0.82 | 0.27 |

**Table2**

| | Control Oocyte | | | | | | | | |
|---|---|---|---|---|---|---|---|---|---|
| Ligand sample | Rate of response | Representative Waveform | Mean amount of current variation ± S.D | Amount of current variation (µA) | | | | | |
| | | | | 1 | 2 | 3 | 4 | 5 | 6 |
| 1 | 0/6 | ND | | ND | ND | ND | ND | ND | ND |
| 2 | 0/6 | ND | | ND | ND | ND | ND | ND | ND |

Since a cell response to ligand stimulation was generated by expression of the ph01207 gene, the inventors believe that the ph01207 gene product is a GPCR having a function that activates the intracellular signal transduction pathway in response to a ligand.

### EXAMPLE 4

### (Analysis of protein interaction of ph01207 gene product)

Analysis of protein interaction of the ph01207 gene product was examined using the yeast two hybrid system.

Based on ph01207 sequence information and hBAI2 sequence information, baits were set out respectively at 4 sites in the N-terminal region and 2 sites in the C-terminal region for the ph01207 gene product, and the N-terminal region which corresponds to a region consisting of 55 amino acid residues lacked in ph01207 and the C-terminal region which corresponds to a region having an insertion of one amino acid residue in ph01207 for the hBAI2 gene product. Then, screening was performed for cDNA libraries derived from brain, hippocampus, breast cancer and prostate cancer, heart, and skeletal muscle that were selected according to a report (Non-Patent Literature 1) showing the distribution of hBAI2 expression.

When using the baits designed based on the C-terminal region of the ph01207 gene product having an insertion of one amino acid residue corresponding to the position 1461 in the amino acid sequence represented by SEQ ID NO: 20, and the C-terminal region of the hBAI2 gene product without insertion of the amino acid residue, either provided preys such as DLG2, DLG3, DLG4, AIP1, MAGI3 and the like that are the MAGUK family proteins. In addition, HOMER2, Citron, SYNE-1, KIF5A and KIFAP3 were obtained as prey that is specific to the hBAI2 gene product.

The bait designed based on the N-terminal region of the ph01207 gene product having a deletion of 55 amino acid residues provided only BAT1 (HLA-B associated transcript 3) as prey. Meanwhile, the bait designed based on the N-terminal region of the hBAI2 gene product having no deletion of the aforementioned amino acid residues provided KCNN2 (potassium intermediate/small conductance calcium-activated channel, subfamily N, member 2) and the like

It was thus revealed that the ph01207 gene product is different from the hBAI2 gene product in the interacting proteins detected by the yeast two hybrid system, except for MAGUK family proteins.

The ph01207 gene product and the hBAI2 gene product were found to interact with MAGUK family proteins in their respective C-terminal regions. MAGUK family proteins are present in cytoplasm and bind to membrane proteins such as receptors or ion channels that are present in the cell membrane to participate in signal transduction from these membrane proteins. The inventors therefore consider that the ph01207 gene product and the hBAI2 gene product are functional membrane protein receptors that participate in intracellular signal transduction through MAGUK family proteins.

### EXAMPLE 5

### (Determination of CCK-8-induced change in intracellular calcium concentration in ph01207 expressing cell line)

Change in intracellular calcium (Ca²⁺) concentration caused by CCK-8S (SEQ ID NO: 14), CCK-8NS or CCK-4 was determined using the ph01207 expressing cell lines prepared in Example 2. The cell lines prepared in Example 2 are eight lines in which the protein encoded by ph01207 expresses as a FLAG-tag fusion protein and four lines in which the protein encoded by ph01207 expresses as a HA-tag fusion protein. Among them, HA-ph01207#10-6 cell line was used in this example, which was obtained by cell cloning of one cell line that stably expressed the protein encoded by ph01207 as a HA-tag fusion protein. CCK-8NS is a CCK octapeptide represented by the same amino acid sequence as CCK-8S (SEQ ID NO: 14), but not sulfated at the seventh tyrosine residue from the C-terminal thereof. CCK-4 is a tetrapeptide consisting of the amino acid residues from the C-terminus to the fourth residue of CCK-8S (SEQ ID NO: 14).

A specific method for measuring a CCK-8S (SEQ ID NO: 14) induced change in intracellular Ca²⁺ concentration in the HA-ph01207#10-6 cell line and the results thereof are described below. HA-ph01207#10-6 cell line was seeded in 96-well plates with a black wall surface and transparent bottom at 2×10⁴ cells/100 µL medium/well, and cultured at 37°C in the presence of 5% CO₂. DMEM/F12 (Gibco) containing 10% fetal calf serum (Moregate BioTech) was used as medium. On the next day, 50 µL of the medium was removed from each well, followed by addition of 50 µL of loading buffer to each well for reaction at room temperature for 1.5 h so as to make the cells uptake a fluorescent dye contained in the loading buffer. The loading buffer was prepared by dissolving component A of the FLIPR Calcium 3 Assay Kit (Molecular Devices Corp.) in a solution composed of 9.9 mL of component B and 0.1 mL of 500 mM probenecid (Sigma) added thereto. After the reaction, a time-dependent change in the fluorescence intensity upon addition of CCK-8S (Peptide Institute) was measured for each well using FLEXstation (Molecular Devices Corp.). Measurement was also performed in a similar manner for CCK-8NS (Peptide Institute, Osaka, Japan) and CCK-4 (Peptide Institute). A 0.1 mM solution of CCK-8S (SEQ ID NO: 14) was prepared by dissolving 0.52 mg thereof in 4.5 mL of 1% NaHCO₃ (Wako). A 0.1 mM solution of CCK-8NS was prepared by dissolving 0.53 mg thereof in 0.50 mL of dimethylsulfoxide (DMSO, Sigma), followed by addition of 4.50 mL of double distilled water thereto. A 0.2 mM solution of CCK-4 was prepared by dissolving 0.54 mg thereof in 0.46 mL of DMSO, followed by addition of 4.09 mL of double distilled water thereto. CCK-8S (SEQ ID NO: 14), CCK-8NS and CCK-4 were each diluted with phosphate buffered saline (PBS) into a 5 nM solution, and then 25 µL of each were added for the measurement (final concentration 1nM). As a positive control that induces an increase in intracellular Ca²⁺ concentration, A23187 (Calbiochem, CA) was used at a final concentration of 10 µM. As a negative control, CHO-K1 cell line that was used as a host in production of the HA-ph01207#10-6 cell line was used. Since the CHO-K1 cell line is not transfected with the ph01207 expression vector, it does not express the ph01207 gene product.

The results showed that the intracellular Ca²⁺ concentration of the HA-ph01207#10-6 cell line subjected to stimulation with CCK-8S (SEQ ID NO: 14) (Figure 4-A) was increased in comparison to that of the HA-ph01207#10-6 cell line not subjected to the stimulation (Figure 4-E). However, an increase in the intracellular Ca²⁺ concentration ofthe HA-ph01207#10-6 cell line was not observed by stimulation with CCK-8NS or CCK-4 (Figure 4-B and Figure 4-C). Further, an increase in the intracellular Ca²⁺ concentration of the HA-ph01207#10-6 cell line was observed by stimulation with A23187 (Figure 4-D).

The level of increase in the intracellular Ca²⁺ concentration of the HA-ph01207#10-6 cell line caused by 1 nM CCK-8S (SEQ ID NO: 14) (Figure 4-A) was roughly equal to the level of increase in the intracellular Ca²⁺ concentration caused by A23187 that was used as a positive control (Figure 4-D).

In contrast, the CHO-K1 cell line showed no increase in intracellular Ca²⁺ concentration by stimulation with any one of CCK-8S (SEQ ID NO: 14), CCK-8NS and CCK-4 (Figure 4-A, Figure 4-B and Figure 4-C). However, an increase in the intracellular Ca²⁺ concentration of CHO-K1 cells upon stimulation with A23187 was observed (Figure 4-D).

These results revealed that the HA-ph01207#10-6 cell line responded specifically to CCK-8S (SEQ ID NO: 14). It was also found that the protein encoded by ph01207 that was expressed in the HA-ph01207#10-6 cell line is involved in the increase in the intracellular Ca²⁺ concentration in the cell line caused by CCK-8S (SEQ ID NO: 14). The inventors therefore believe that CCK-8S (SEQ ID NO: 14) is a ligand for the ph01207 gene product.

Further, the HA-ph01207#10-6 cell line exhibited a sufficiently high cell response to 1 nM CCK-8S (SEQ ID NO: 14). Since CCK-8S (SEQ ID NO: 14) induced a biological response in the HA-ph01207#10-6 cell line at such a low concentration as 1 nM, the inventors believe that CCK-8 actually induces a cell response through the protein encoded by ph01207 in vivo. That it, the inventors believe that CCK-8S (SEQ ID NO: 14) is one of the in vivo ligands of ph01207 that is predicted to be a GPCR.

### EXAMPLE 6

### (Identification of splicing variant of ph01207 gene)

A splicing variant of ph01207 gene was acquired by carrying out cloning by RT-PCR.

Acquisition of a splicing variant of ph01207 gene was carried out as follows. First, cDNA library was constructed by an ordinary method using human brain-derived polyA(+) RNA (Clontech) as the starting material, followed by isolation of cDNA fragment by dbEST analysis and determination of the nucleotide sequence of cDNA clone. Specifically, RT-PCR was carried out using Superscript first-strand synthesis system for RT-PCR (Invitrogen) in 10 µL reaction system containing 0.1 µg of polyA(+) RNA to construct a cDNA library. Using this cDNA as a template, PCR was carried out using an oligonucleotide consisting of the nucleotide sequence as set forth in SEQ ID NO: 23 and an oligonucleotide consisting of the nucleotide sequence as set forth in SEQ ID NO: 24 as primers. PCR using these primers allows amplification of DNA of a region encoding an amino acid sequence from aspartic acid (D) at position 44 to cysteine (C) at position 475 of hBAI2 (SEQ ID NO: 22), and DNA corresponding to the region in a splicing variant of hBAI2 (SEQ ID NO: 22) (see Fig, 1-C). A recombination between each clone obtained and ph01207 cDNA clone was carried out by ScaI and XhoI, and thereby three kinds of full length cDNA clones were obtained. The nucleotide sequence of each clone was determined by sequence analysis.

As a result, three kinds of full length cDNA clones that are considered to be splicing variants of ph01207 gene from viewpoints of sequence homology and structural similarity were obtained. These cDNA clones are referred to as 7tmHR gene, hk01941 gene and variant 3 gene, respectively. All of these splicing variants have different numbers of repeats of TSP-I domain in the N-terminal extracellular region (Fig. 1-B). 7tmHR gene among these splicing variants encodes the longest protein.

7tmHR gene comprises a nucleotide sequence (SEQ ID NO: 19) of 4719 bp containing an ORF that encodes 1573 amino acid residues (SEQ ID NO: 20) having a segment presumed to be a signal sequence consisting of 20 amino acid residues from the N terminus. The 7tmHR gene has been registered in GenBank as Accession No: AB065648. The protein encoded by this DNA has a seven-span transmembrane domain, and has four TSP-I domains and one GPS domain. (See Fig. 1-B). The protein has the same amino acid sequence as hBAI2 (GenBank, Accession NO: AB005298), except for the insertion of one amino acid residue in the C-terminal side region in comparison to the sequence ofhBAI2. The insertion of one amino acid residue was found between glutamic acid (E) at position 1460 and valine (V) at position 1461 in the amino acid sequence of hBAI2, and the inserted amino acid residue was lysine. The inserted lysine is placed at position 1461 in the amino acid sequence of the protein (SEQ ID NO: 20) that was encoded by 7tmHR gene. From viewpoints of sequence homology and structural similarity, 7tmHR gene as well as the protein encoded by the gene was deemed to be a splicing valiant of hBAI2 gene.

hk01941 gene is a novel gene comprising a nucleotide sequence (SEQ ID NO: 15) of 4389 bp containing an ORF that encodes 1463 amino acid residues (SEQ ID NO: 16) having a segment presumed to be a signal sequence consisting of 20 amino acid residues from the N terminus. The protein encoded by this DNA has a seven-span transmembrane domain, and has two TSP-I domains and one GPS domain. (See Fig. 1-B). The amino acid sequence of the protein encoded by this DNA is same as that of the protein (SEQ ID NO: 20) encoded by 7tmHR gene, except for the deletion of 110 amino acid residues containing two TSP-I domains in the N-terminal side. The deleted 110 amino acid residues corresponds to those from glycine (G) at position 296 to proline (P) at position 405 in the amino acid sequence (SEQ ID NO: 20) of the protein encoded by 7tmHR gene.

Variant 3 gene is a novel gene comprising a nucleotide sequence (SEQ ID NO: 17) of 4554 bp containing an ORF that encodes 1518 amino acid residues (SEQ ID NO: 18) having a segment presumed to be a signal sequence consisting of 20 amino acid residues from the N terminus. The protein encoded by this DNA has a seven-span transmembrane domain, and has three TSP-I domains and one GPS domain. (See Fig. 1-B). The amino acid sequence of the protein encoded by the DNA is same as that of the protein (SEQ ID NO: 20) encoded by 7tmHR gene, except for the deletion of 55 amino acid residues containing the second one of TSP-I domains from the N-terminal side. The deleted 55 amino acid residues corresponds to those from valine (V) at position 351 to proline (P) at position 405 in the amino acid sequence (SEQ ID NO: 20) of the protein encoded by 7tmHR gene.

ph01207 gene encodes the protein having the amino acid sequence that is same as that of the protein (SEQ ID NO: 20) encoded by 7tmHR gene, except for the deletion of 55 amino acid residues containing one TSP-I domain in the N-terminal side. The deleted 55 amino acid residues corresponds to those from glycine (G) at position 296 to proline (P) at position 350 in the amino acid sequence (SEQ ID NO: 20) of the protein encoded by 7tmHR gene.

### EXAMPLE 7

### (Construction of cell line stably expressing a splicing variant of ph01207 gene)

A cell line stably expressing a splicing variant of ph01207 gene was constructed and used to study for a CCK-8S induced cell response in the stably expressing cell line. The splicing variants used were three kinds of splicing variants acquired in EXAMPLE 6, which were 7tmHR gene, hk01941 gene and variant 3 gene.

First, an expression vector of each of full length cDNA clones was constructed by using pcDNA3.1 (+) (Invitrogen) that was an expression vector for animal cells to carry out a recombinant reaction with each of full length cDNA clone by Asp718I and NotI. Expression vectors thus obtained were referred to as 7tmHR/pcDNA3.1, hk01941/pcDNA3.1 and variant 3/pcDNA3.1.

A 7tmHR stably expressing cell line and an hk01941 stably expressing cell line were prepared by transfecting a CHO-K1 cell line with 7tmHR expression vector (7tmHR/pcDNA3.1) and hk01941 expression vector (hk01941/pcDNA3.1), respectively. Specifically, the expression vector was mixed with Lipofectamine 2000 (may be abbreviated to as LF2000, Invitrogen) (4 µg, DNA/250 µL DMEM/F12 medium + 10µL LF 2000/250 µL DMEM/F12 medium), and then added to CHO-K1 cell line cultured in 2 mL medium/well of 6-well plates for transfection . On the next day, the cells were collected and seeded at a cell density of one cell/well in 96-well plates to carry out culturing in selection medium (DMEM/F12 medium containing 10% FCS) containing 400 µg/mL of G418 to screen expressing cell lines. Further, the screened expression cell lines were subjected to FCM analysis using anti-hBAI2 antibody and Western blotting to confirm expression of each of introduced genes.

### EXAMPLE 8

### (Functional analysis of cell line stably expressing a splicing variant of ph01207 gene)

A cell line stably expressing a splicing variant of ph01207 gene was used for measuring a CCK-8S-induced change in intracellular calcium (Ca²⁺) concentration by the same method described in Example 5. As a cell line stably expressing a splicing variant of ph01207 gene, the stably expressing cell line constructed in Example 7 was used. Further, a cell line stable expressing hk01941 gene was used to conduct the same investigation. As a cell line stably expressing hk01941 gene, the stably expressing cell line constructed in Example 7 was used. As a control, a host cell which was transfected with neither 7tmHR expression vector nor hk01941 expression vector was used.

The cell line was seeded in 96-well plates with a black wall surface and transparent bottom at 3 × 10⁴ cells/100 µL medium/well, and cultured overnight at 37°C in the presence of 5% CO₂. On the next day, 50 µL of the medium was removed from each well, followed by addition of 50 µL of loading buffer to each well to allow for a reaction at room temperature for 1 h so as to allow cells to incorporate fluorescent dye contained in the loading buffer. The loading buffer was prepared by the same method as described in Example 5. After the reaction, a change in the fluorescence intensity upon addition of CCK-8S was measured time-dependently for each well using FLEXstation (Molecular Devices Corp.). CCK-8S was used at a final concentration of 10 pM 1 µM. Further, A23187 was used at a final concentration of 20µM as a positive control of the assay.

An increase in intracellular calcium (Ca²⁺) concentration was observed upon addition of CCK-8S in both the 7tmHR stably expressing cell line and the hk01941 stably expressing cell line. In contrast, no change in intracellular calcium concentration was observed upon addition of CCK-8S in a host cell which was transfected with neither 7tmHR expression vector nor hk01941 expression vector. The similar results were obtained in a plurality of clones of both stably expressing cell lines. Changes in intracellular calcium (Ca²⁺) concentration upon addition of the physiological concentration of CCK-8, that is 1 nM, are shown in Fig. 5-A and Fig. 6-A for typical clones of the 7tmHR stably expressing cell line and the hk01941 stably expressing cell line. Further, a response to 20 µM of A23187 used as a positive control was observed similarly in both the expressing cell lines and the host cell (Fig. 5-B and Fig. 6-B).

Since a change in intracellular calcium (Ca²⁺) concentration was observed upon addition of CCK-8S in the 7tmHR stably expressing cell line and the hk01941 stably expressing cell line, but not in the host cell, the inventors consider that the gene products of 7tmHR gene and hk01941 gene both mediate a CCK-8S-induced cell response. That is, the inventors consider that the gene products of 7tmHR gene and hk01941 gene were both expressed on the surface of cell membrane, and activated an intracellular signal transduction pathway by an action of extracellular CCK-8S, and thereby caused an increase in intracellular calcium (Ca²⁺) concentration as a cell response.

### EXAMPLE 9

### (Functional analysis of stably expressing cell line of variant 3 gene)

The function of variant 3 gene was analyzed using stably expressing cell line of variant 3 gene by investigating a CCK-8S-induced change in intracellular calcium concentration (Ca²⁺). The change in intracellular calcium concentration (Ca²⁺) was measured using the same method described in Example 5. The stably expressing cell line of variant 3 gene was prepared using the variant 3 gene expression vector constructed in Example 7 by the method similar to the method described in Example 7.

The cell line was seeded in 96-well plates with a black wall surface and transparent bottom at 3 × 10⁴ cells/100 µL medium/well, and cultured overnight at 37°C in the presence of 5% CO₂. On the next day, 50 µL of the medium was removed from each well, followed by addition of 50 µL of loading buffer to each well to allow for a reaction at room temperature for 1 h so as to allow cells to incorporate fluorescent dye contained in the loading buffer. The loading buffer was prepared by the same method as described in Example 5. After the reaction, a change in the fluorescence intensity upon addition of CCK-8S was measured time-dependently for each well for 40 sec using FLEXstation (Molecular Devices Corp.). CCK-8S was used at a final concentration of 10 pM to 1 µM. Further, A23187 was used at a final concentration of 20µM as a positive control of the assay

An increase in intracellular calcium (Ca²⁺) concentration was observed upon addition of CCK-8S in the variant 3 gene stably expressing cell line. In contrast, no change in intracellular calcium concentration was observed upon addition of CCK-8S in a host cell which was not transfected with variant 3 expression vector. Changes in intracellular calcium (Ca²⁺) concentration upon addition of the physiological concentration of CCK-8, that is 1 nM, are shown in Fig. 7-A for a typical clone of the variant 3 gene stably expressing cell line. Further, a response to 20 µM of A23187 used as a positive control was observed similarly in both the expressing cell lines and the host cell (Fig. 7-B).

Since a change in intracellular calcium (Ca²⁺) concentration was observed upon addition of CCK-8S in the variant 3 gene stably expressing cell line, but not in the host cell, the inventors consider that the gene product of variant 3 gene mediates a CCK-8S-induced cell response. That is, the inventors consider that the gene product of variant 3 gene was expressed on the surface of cell membrane, and activated an intracellular signal transduction pathway by an action of extracellular CCK-8S, and thereby caused an increase in intracellular calcium (Ca²⁺) concentration as a cell response.

### EXAMPLE 10

### (Tissue expression ofph01207 gene)

Tissue expression of ph01207 gene was analyzed by search in LifeSpan DrugTarget Database^{™} (LifeSpan Bioscience) and by search in BioExpress Database (Gene Logic).

The analysis by searching in LifeSpan DrugTarget Database^{™} (LifeSpan Bioscience) revealed that the tissue immunostaining data obtained with three kinds of rabbit anti-human BAI2 polyclonal antibodies (LS-A981, A982, A984; Life Span) indicated strong general staining of neuron and astrocyte in amygdaloid body, hippocampus, medulla, hypothalamus, locus niger, and brain cortex, as well as a part of cells in anterior pituitary, and Herring body of posterior pituitary. Fig. 8-A and Fig. 8-B show tissue immunostaining data by LS-A981of protoplasmic astrocyte of amygdaloid body, and of neuron and glia of amygdaloid body, respectively. Fig. 8-C and Fig. 8-D show tissue immunostaining data by LS-A981 of neuron in CA2 region and in CA1 region of hippocampus, respectively.

Expression analysis using Genetip analysis data of BioExpress Database (Gene Logi) was carried out for ph01207 (hBAI2), CCK-A receptor (CCK-AR), CCK-B receptor (CCK-BR) and CCK. Results ofthe analysis are shown in Table 3.

A strong expression of ph01207 (hBAI2) gene was found in brain tissue, particularly in brain cortex such as temporal pole of cerebrum and motor cortex or the like, hippocampus and amygdaloid body (Table 3). Almost no expression of this gene was found in digestive organs such as pancreas, small intestine, stomach, and gall bladder.

In contrast, almost no expression of CCK-A receptor (referred to as CCK-AR in the table) was found in the brain tissue, but expression thereof in digestive organs such as pancreas, stomach, and gall bladder was observed (Table 3). Further, the expression of CCK-B receptor (referred to as CCK-BR in the table) was remarkable in pancreas and small intestine, and was also found in brain tissue such as brain cortex, hippocampus and amygdaloid body (Table 3). The amount of expression of ph01207 (hBAI2) gene in brain tissue was much higher compared with the amount of expression of CCK-B receptor.

Analysis of tissue expression of CCK revealed a similar distribution as that observed with ph01207. Thus, it was demonstrated that ph01207 and CCK were strongly expressed at the same site (Table 3).

**Table 3**

| | | CCK-AR | CCK-BR | ph01207 | | CCK |
|---|---|---|---|---|---|---|
| Brain | Brain cortex | 50> | 100~200 | 300~700 | | 500~1500 |
| | Hippocampus | 50> | 100 | 500 | | 500 |
| | Amygdaloid body | 50> | 100 | 500 | | 700 |
| Digestive organ | Pancreas | 100 | 200 | 50> | | 50> |
| | Small intestine | 50> | 50> | 50> | | 100~500 |
| | Stomach | 100 | 100~400 | 50> | | 50> |
| | Gall bladder | 100 | 50> | 50> | | 50> |

### EXAMPLE 11

Information about functions of BAI2 knockout mouse was obtained from the database relating to knockout mouse functions (Deltagen).

BAI2 knockout mouse was prepared by gene disruption by homologous recombination. Specifically, gene disruption by homologous recombination was carried out by preparing a targeting vector for targeting a domain (876D-917L region) in the nucleotide sequence of mouse BAI2 gene (encoding 1560 amino acid residues) which encoded from aspartic acid (D) at position 876 to leucine (L) at position 917 of mouse BAI12, and introducing the targeting vector into ES cell derived from 129/OlaHsd mouse. The targeting vector included a LacZ-Neo gene cassette that contained genome sequence 0.8 kb upstream from intron located upstream of a 876D-917L region of mouse BAI2 gene in 5' side (5' arm) and genome sequence 1.2 kb downstream from intron located downstream from a 876D-917L region of the gene in 3' side (3' arm). A LacZ-Neo fragment was inserted to target site of mouse BAI2 gene by homologous recombination using this targeting vector. ES cell in which homologous recombination took place was selected using selection medium containing G418, and used for preparation of a chimeric mouse by an ordinary method using C57BL/6 mouse. By mating a thus obtained chimeric mouse with C57BL/6 mouse, first filial generation (F1) heterozygous mutant mouse was generated. Further, by mating F1 heterozygous mutant mice with each other, second filial generation (F2) homozygous mutant mouse was generated.

Genotype determination for F1 heterozygous mutant mice and F2 homozygous mutant mice was carried out by PCR and northern analysis.

Determination whether or not LacZ-Neo fragment was inserted in target site of mouse BAI2 gene was carried out for F1 heterozygous mutant mouse by LacZ expression analysis. As a result, F1 heterozygous mutant mice showed strong expression of LacZ in their tissues, particularly in hippocampus and amygdaloid body (Fig. 9-A and Fig. 9-B). From these results, it was confirmed that in F1 heterozygous mutant mice, LacZ-Neo fragment was inserted in the target site of mouse BAI2 gene, indicating that the gene was destroyed. These results also suggested that in F1 heterozygous mutant mice, mouse BAI2 gene was strongly expressed in brain tissue, particularly in hippocampus and amygdaloid body.

The functions of BAI2 knockout mice were investigated in behavioral examination, physiological examination, pathological examination and anatomical examination.

The BAI2 knockout mice showed a significant difference in results of the tail suspension test in the behavioral examination in comparison to a wild type mouse (Fig. 10). In contrast, the BAI2 knockout mice did not show any significant difference in many examination items such as physiological examination, pathological examination and anatomical examination in comparison to a wild type mouse.

The tail suspension test is a technique that is ordinarily used as the test method to investigate a phenotype of depression, which comprises fixing a tail of a mouse to hang the mouse upside down, and measuring immorbility time before the mouse starts movement to escape from this state. The tail suspension test is used frequently as a test system for studying a possible relationship with depression, for example, for evaluating an anti-depressant drug (Steru L. et al., "Psychopharmacology" (Berl), 1985, Vol. 85, No. 3. p.367-370; Crowley J.J. et al., "Pharmacological Biochemical Behavior", 2004, Vol. 78, No.2 p.269-274; Nielsen D.M. et al, "European Journal of Pharmacology", 2004, Vol. 499, Nos. 1-2, p.135-146).

It is known that in the tail suspension test, the longer the immobility time lasts, the more strongly a depression state is indicated, while the shorter the immobility time is, the more strongly a contra depression state is indicated. For example, it is known that immobility time is shortened by administration of anti-depressant drug.

The tail suspension test was carried out using ten BAI2 knockout mice. As a control, the tail suspension test was carried out similarly using 16 wild type mice.

The BAI2 knockout mice showed significantly reduced immobility time in the tail suspension test compared with wild type mice (Fig. 10). Results are shown by average ± standard deviation of immobility time of each mouse in BAI2 knockout mouse group and wild type mouse group. A significant difference was obtained in statistical processing using T-test.

It can be postulated from the results of the tail suspension test that BAI2 knockout mice were in a contra depression state. Since BAI2 knockout mice exhibited a contra depression-like phenotype, the inventors consider that BAI2 gene is involved in depression.

BAI2 gene and splicing variants thereof are not expressed in BAI2 knockout mice, because of the destruction of BAI2 gene. Therefore, the inventors consider that not only BAI2 gene but also splicing variants thereof are involved in depression.

### EXAMPLE 12

Identification of a compound that inhibits a response of ph01207 gene product to a ligand was carried out with a system for measuring a change in intracellular calcium concentration using ph01207 expressing cell line.

ph01207 expressing cell line was constructed as follows. First, recombination was carried out between ph01207 cDNA clone identified in Example 1 and pcDNA3.1 (Invitrogen) by Asp718I (Boehriger) and NotI (TAKARA) to construct a ph01207 expression vector that did not contain an epitope-tag. ph01207 expression vector was transfected using Lipofectamine 2000 (Invitrogen) to CHO-K1 cell line that is a host cell, followed by carrying out selection of stably expressing cell line with a medium containing G418. Detection of the expression of introduced genes in cells was carried out by FCM analysis using anti-ph01207 antibody

As a result, a plurality of cell lines that stably expressed ph01207 was obtained. Identification of a compound was carried out using ph01207 #3F8-17 cell line that was one of clones among them.

As a ligand, CCK-8S (Peptide Institute) was used. 0.52 mg of CCK-8S (SEQ ID NO: 14) was dissolved in 4.5 mL of 1% NaHCO₃ (Wako) to prepare a solution of 0.1 mM concentration. As a test compound, SoftFocus GPCR Target-Directed Library that is a compound library (BioFocus) was used. A 2 mg/mL DMSO solution of each compound was diluted 25-fold with PBS to adjust the concentration to 80 µg/mL for use.

Specifically, identification of a compound was carried out as follows. ph01207 #3F8-17 cell line was seeded in 96-well plates with a black wall surface and transparent bottom) at 3 × 10⁴ cells /100 µL medium/well, and cultured at 37°C in the presence of 5% CO₂. DMEM/F12 (supplied by Gibco) containing 10% fetal calf serum (supplied by Moregate) was used as the medium. On the next day, 20 µL of 6 × loading buffer was added to each well to allow for a reaction at 37°C for 1 h so as to allow cells to incorporate fluorescent dye contained in the loading buffer. The 6 × loading buffer was prepared by dissolving component A of the FLIPR Calcium 3 Assay Kit (Molecular Devices Corp.) with component B followed by adding probenecid (Sigma) to have a final concentration of 15 mM. After the reaction, CCK-8S (Peptide Institute) and a compound were added to measure a change in the fluorescence intensity time-dependently for each well for 80 sec. As a control, the buffer was used instead of a compound for similar measurements. The compound was added at a final concentration of 10 µg/mL, or the buffer was added, at 15 sec after the start of the measurement, and then CCK-8S was added at a final concentration of 10 nM at 35 sec after addition of the compound or the buffer. A time-dependent measurement of the change in the fluorescence intensity was carried out using FLEXstation (Molecular Devices Corp.).

Some of the compounds tested inhibited the response of ph01207 #3F8-17 cell line to CCK-8S. As typical examples, responses of ph01207 #3F8-17 cell line to CCK-8S when adding with three kinds of compounds that are compound A, compound B and compound C are shown in Fig. 11-A, Fig. 11-B and Fig. 11-C, respectively. Compound A, compound B and compound C are those compounds represented by aforementioned structural formulae (I), (II) and (III).

No change was observed in fluorescence intensity when compound A or the buffer was added at 15 sec after the start of the measurement. When CCK-8S that is a ligand was added at 35 sec after addition of the compound A or the buffer, fluorescence intensity was increased in the wells to which the buffer was added, indicating an induction of a response of ph01207 #3F8-17 cell line to the ligand, while such a response was inhibited in the wells to which the compound A was added (Fig. 11-A). This result suggests that the compound A works as an antagonist to an interaction between CCK-8S and ph01207.

Both compound B and compound C inhibited a response of ph01207 #3F8-17 cell line to the ligand similarly to the compound A (Fig. 11-B and Fig. 11-C). This result suggests that both compound B and compound C work as antagonists to an interaction between CCK-8S and ph01207.

From above mentioned results, the inventors considered that an antagonist that inhibits a response of ph01207 to a ligand thereof such as CCK-8S can be identified by using a system for measuring a change in intracellular calcium concentration using ph01207 expression cell line.

### INDUSTRIAL APPLICABILITY

According to the present invention there can be provided a protein working as a functional membrane protein receptor that has a seven-span transmembrane domain which is considered to be a GPCR, and a DNA encoding the protein. The present protein is expressed on the cell membrane when expressed in a cell, and activates intracellular signal transduction by ligand stimulation to induce a cell response.

The present invention allows for elucidation of signal transduction pathways and cell functions in which the present protein is involved, and for regulation thereof The present invention also allows for prevention and/or treatment of diseases attributable to an abnormality in the present protein and/or the DNA, for example, depression.

Thus, the present invention is useful as contributing in a broad range of fields from basic science to pharmaceutical development.

### SEQUENCE TABLE FREE TEXT

SEQ ID NO: 1: DNA encoding a novel functional membrane protein receptor
SEQ ID NO: 2: Protein encoded by the DNA represented by the nucleotide sequence as set forth in SEQ ID NO: 1
SEQ ID NO: 2: (297) : (350) TSP-I domain
SEQ ID NO: 2: (352): (405) TSP-I domain
SEQ ID NO: 2: (408) : (461) TSP-I domain
SEQ ID NO: 2: (870) : (890) transmembrane domain
SEQ ID NO: 2: (899) : (919) transmembrane domain
SEQ ID NO: 2: (928) : (948) transmembrane domain
SEQ ID NO: 2: (970) : (990) transmembrane domain
SEQ ID NO: 2: (1012): (1032) transmembrane domain
SEQ ID NO: 2: (1087): (1107) transmembrane domain
SEQ ID NO: 2: (1114) : (1134) transmembrane domain
SEQ ID NO: 3: Partial amino acid sequence present in each c-terminal region of a peptide represented by the amino acid sequence as set forth in SEQ ID NO: 2, hBAI1 and hBAI2
SEQ ID NO: 4: A designed oligonucleotide for use as a primer
SEQ ID NO: 5: Designed oligonucleotide for use as a primer
SEQ ID NO: 6: Designed oligonucleotide for use as a primer
SEQ ID NO: 7: Designed oligonucleotide for use as a primer
SEQ ID NO: 8: Designed oligonucleotide for use as a primer
SEQ ID NO: 9: Designed oligonucleotide for use as a primer
SEQ ID NO: 10: Designed oligonucleotide for use as a primer
SEQ ID NO: 11: Designed oligonucleotide for use as a primer
SEQ ID NO: 12: Synthesized oligonucleotide
SEQ ID NO: 13: Synthesized oligonucleotide
SEQ ID NO: 14: CCK-8S
SEQ ID NO: 14: (2) (2) sulfated
SEQ ID NO: 15: Splicing variant of DNA as set forth in SEQ ID NO: 1
SEQ ID NO: 16: Protein encoded by DNA represented by nucleotide sequence as set forth in SEQ ID NO: 15
SEQ ID NO: 16: (297): (350) TSP-I domain
SEQ ID NO: 16: (353): (406) TSP-I domain
SEQ ID NO: 16: (815): (835) transmembrane domain
SEQ ID NO: 16: (844): (864) transmembrane domain
SEQ ID NO: 16: (873): (893) transmembrane domain
SEQ ID NO: 16: (915): (935) transmembrane domain
SEQ ID NO: 16: (957): (977) transmembrane domain
SEQ ID NO: 16: (1032): (1052) transmembrane domain
SEQ ID NO: 16: (1059): (1079) transmembrane domain
SEQ ID NO: 17: Splicing variant of DNA as set forth in SEQ ID NO: 1
SEQ ID NO: 18: Protein encoded by DNA represented by nucleotide sequence as set forth in SEQ ID NO: 17
SEQ ID NO: 18: (297): (350) TSP-I domain
SEQ ID NO: 18: (352): (405) TSP-I domain
SEQ ID NO: 18: (408): (461) TSP-I domain
SEQ ID NO: 18: (870): (890) transmembrane domain
SEQ ID NO: 18: (899): (919) transmembrane domain
SEQ ID NO: 18: (928): (948) transmembrane domain
SEQ ID NO: 18: (970): (990) transmembrane domain
SEQ ID NO: 18: (1012): (1032) transmembrane domain
SEQ ID NO: 18: (1087): (1107) transmembrane domain
SEQ ID NO: 18: (1114): (1134) transmembrane domain
SEQ ID NO: 19: Splicing variant of DNA as set forth in SEQ ID NO: 1
SEQ ID NO: 20: Protein encoded by DNA represented by nucleotide sequence as set forth in SEQ ID NO: 19
SEQ ID NO: 20: (297): (350) TSP-I domain
SEQ ID NO: 20: (352): (405) TSP-I domain
SEQ ID NO: 20: (407): (460) TSP-I domain
SEQ ID NO: 20: (463): (516) TSP-I domain
SEQ ID NO: 20: (925): (945) transmembrane domain
SEQ ID NO: 20: (954): (974) transmembrane domain
SEQ ID NO: 20: (983): (1003) transmembrane domain
SEQ ID NO: 20: (1025): (1045) transmembrane domain
SEQ ID NO: 20: (1067): (1087) transmembrane domain
SEQ ID NO: 20: (1142): (1162) transmembrane domain
SEQ ID NO: 20: (1169): (1189) transmembrane domain
SEQ ID NO: 21: DNA encoding hBAI2, that is a splicing variant of DNA as set forth in SEQ ID NO: 1
SEQ ID NO: 22: Protein encoded by DNA represented by nucleotide sequence as set forth in SEQ ID NO: 21
SEQ ID NO: 22: (297): (350) TSP-I domain
SEQ ID NO: 22: (352): (405) TSP-I domain
SEQ ID NO: 22: (407): (460) TSP-I domain
SEQ ID NO: 22: (463): (516) TSP-I domain
SEQ ID NO: 22: (925): (945) transmembrane domain
SEQ ID NO: 22: (954): (974) transmembrane domain
SEQ ID NO: 22: (983): (1003) transmembrane domain
SEQ ID NO: 22: (1025): (1045) transmembrane domain
SEQ ID NO: 22: (1067): (1087) transmembrane domain
SEQ ID NO: 22: (1142): (1162) transmembrane domain
SEQ ID NO: 22: (1169): (1189) transmembrane domain
SEQ ID NO: 23: Designed oligonucleotide for use as a primer
SEQ ID NO: 24: Designed oligonucleotide for use as a primer

## Claims

1. A method for identifying a compound having an anti-depressant action that is an antagonist of a protein encoded by a DNA represented by a nucleotide sequence as set forth in SEQ ID NO: 1 in the sequence listing or of a homolog protein thereof, comprising using at least one member selected from the following: a DNA represented by a nucleotide sequence as set forth in SEQ ID NO: 1 in the sequence listing or a homolog DNA thereof, a protein encoded by the DNA and a cell containing the DNA.

2. A method for identifying a compound having an anti-depressant action that is an antagonist of any one protein selected from the group consisting of a protein encoded by a DNA represented by a nucleotide sequence as set forth in SEQ ID NO: 1 in the sequence listing and splicing variants of the protein, comprising using at least one member selected from the following: a DNA represented by a nucleotide sequence as set forth in SEQ ID NO: 1 in the sequence listing, a DNA represented by any one of nucleotide sequences of splicing variants of the DNA, a protein encoded by the DNA, and a cell containing the DNA.

3. A method for identifying a compound having an anti-depressant action that is an antagonist of any one protein selected from the group consisting of a protein encoded by a DNA represented by a nucleotide sequence as set forth in SEQ ID NO: 1 in the sequence listing and splicing variants of the protein, comprising contacting a cell containing a DNA represented by any one of nucleotide sequences as set forth in SEQ ID NO: 1, 15, 17, 19 and 21 in the sequence listing with a test compound, measuring a function of a protein encoded by the DNA that is expressed on a cell membrane of the cell, comparing with a case where the cell is not made to contact with a test compound, and determining that a test compound which reduces or eliminates the function of the protein is to be a compound having anti-depressant action.

4. The identifying method according to claim 3, wherein the function of a protein encoded by the DNA that is expressed on a cell membrane of a cell is a function causing an increase in intracellular calcium concentration in response to addition of a ligand of the protein.

5. The identifying method according to claim 3, wherein the function of a protein encoded by the DNA that is expressed on a cell membrane of a cell is a function causing a change in membrane potential in response to addition of a ligand of the protein.

6. The identifying method according to claim 4 or claim 5, wherein the ligand used therein is a peptide selected from the group consisting of:
(i) cholecystokinin octapeptide sulfated form (SEQ ID NO: 14, hereunder referred to as CCK-8S),
(ii) a peptide having mutations such as deletion, substitution, or addition of one or a few amino acids in amino acid sequence of CCK-8S and having an equivalent function to CCK-8S; and
(iii) a peptide containing the amino acid sequence of the peptide described in (i) or (ii) and having an equivalent function to CCK-8S.

7. A DNA represented by the nucleotide sequence as set forth in SEQ ID NO: 15 in the sequence listing or a complementary strand thereof.

8. A DNA represented by the nucleotide sequence as set forth in SEQ ID NO: 17 in the sequence listing or a complementary strand thereof.

9. A recombinant vector comprising the DNA according to claim 7 or claim 8 or a complementary strand thereof.

10. A transformant into which the recombinant vector according to claim 9 is introduced.

11. A protein encoded by the DNA according to claim 7.

12. A protein represented by the amino acid sequence as set forth in SEQ ID NO: 16 in the sequence listing.

13. A protein encoded by the DNA according to claim 8.

14. A protein represented by the amino acid sequence as set forth in SEQ ID NO: 18 in the sequence listing.

15. A method for producing the protein according to any one of claims 11 to 14, comprising culturing a transformant into which an expression recombinant vector comprising the DNA according to claim 7 or claim 8 is introduced.

16. A reagent kit comprising at least any one of the following: a DNA represented by the nucleotide sequence as set forth in SEQ ID NO: 1 in the sequence listing and splicing variants of the DNA, a recombinant vector comprising any one DNA selected from the DNA and the splicing variants of the DNA, a transformant into which the recombinant vector is introduced, a protein encoded by the DNA, and an antibody that recognizes the protein.

17. The reagent kit according to claim 16, wherein the DNA represented by the nucleotide sequence as set forth in SEQ ID NO: 1 in the sequence listing and splicing variants of the DNA is a DNA represented by any one of nucleotide sequences as set forth in SEQ ID NO: 1,15 and 17 in the sequence listing, and the protein encoded by the DNA is a protein represented by any one of amino acid sequences as set forth in SEQ ID NO: 2, 16 and 18 in the sequence listing.

18. A method for improving depression state, comprising inhibiting the function and/or expression of any one protein selected from the group consisting of a protein encoded by a DNA represented by the nucleotide sequence as set forth in SEQ ID NO: 1 in the sequence listing and splicing variants of the protein.

19. A method for preventing and/or treating depression, comprising using the method for improving depression state according to claim 18.

20. An assay method for use in diagnosing depression, comprising employing a DNA selected from the group consisting of a DNA represented by a nucleotide sequence as set forth in SEQ ID NO: 1 in the sequence listing and splicing variants of the DNA, and/or, the protein encoded by the DNA, as a marker, and performing quantitative or qualitative analysis thereof.

21. A method for diagnosing depression, comprising employing a DNA selected from the group consisting of a DNA represented by a nucleotide sequence as set forth in SEQ ID NO: 1 in the sequence listing and a splicing variant of the DNA, and/or, the protein encoded by the DNA, as a marker, and performing quantitative or qualitative analysis thereof.
